# EUROPEAN PATENT APPLICATION

(11) **EP 4 782 450 A1**
(43) Date of publication of application: **29.07.2026**
(21) Application number: 24868702.2
(22) Date of filing: 20.09.2024
(51) Int. Cl.: C07D 471/04, A61K 31/519, A61K 31/4709, A61K 31/517, A61P 35/00, C07D 487/04, C07D 403/14, C07D 403/12

(54) **NOVEL SKELETAL DERIVATIVE EFFECTIVE FOR HER2 MUTATION, AND PHARMACEUTICAL COMPOSITION COMPRISING SAME**

(30) Priority: 22.09.2023 KR 20230126972; 30.01.2024 KR 20240014218
(71) Applicant: Hanmi Pharm. Co., Ltd., Hwaseong-si, Gyeonggi-do 18536 (KR)
(72) Inventor: NAM, Ho Yeon, Hwaseong-si, Gyeonggi-do 18536 (KR); AHN, Young Gil, Hwaseong-si Gyeonggi-do Gyeonggi-do 18536 (KR); YOO, Hyung Seok, Hwaseong-si, Gyeonggi-do 18536 (KR); LEE, Gun Woo, Hwaseong-si, Gyeonggi-do 18536 (KR); JANG, Sun Young, Hwaseong-si, Gyeonggi-do 18536 (KR); JEON, Ji Young, Hwaseong-si, Gyeonggi-do 18536 (KR)
(74) Representative: Habermann, Hruschka & Schnabel
(86) International application number: PCT/KR2024/014156
(87) International publication number: WO 2025/063725

(57) **Abstract**

Disclosed herein are compounds of Chemical Formula 1 or tautomers, stereoisomers or pharmaceutically acceptable salts thereof, wherein R¹ to R³, A¹ to A⁴, L¹, E, Z and *n* are as defined herein. The compounds disclosed herein are capable of inhibiting the activity of HER2 kinase.

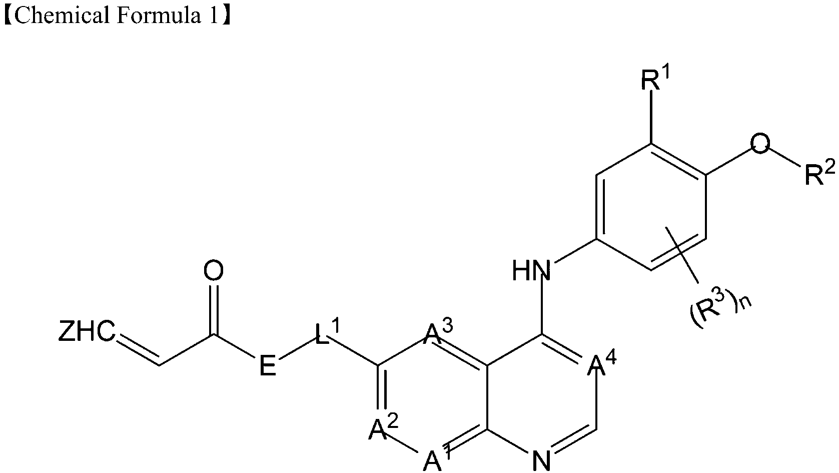

## Description

### FIELD OF THE INVENTION

The present disclosure pertains to novel compounds effective for inhibiting the kinase activity of tumors harboring HER2 gene alterations. The present disclosure also pertains to anti-tumor pharmaceutical compositions comprising the novel compounds.

### BACKGROUND OF THE INVENTION

The epidermal growth factor receptor (EGFR) is a protein consisting of a receptor domain and a tyrosine kinase domain that plays a role in transducing extracellular signals through the cell membrane towards the inside of the cell. The EGFR/ERBB protein family includes this EGFR and other tyrosine kinases structurally homologous to it, more specifically, the four members of EGFR (ERBB1), HER2 (Neu, ERBB2), HER3 (ERBB3) and HER4 (ERBB4). The EGFR family kinases play an essential role in an organism's development, involving themselves in diverse signaling processes such as cell growth and angiogenesis by forming heteromultimers among the family members. EGFR family proteins are reported to be overexpressed or undergo frequent mutations in most solid tumor cell types, which is associated with a poor prognosis. Such HER2 gene alterations or such mutations as insertions in exon 20 of the tyrosine kinase domain of the EGFR and HER help the EGFR or HER2 escape the normal controls against activation. Their constituent activation caused by such mutation is now known to be the major etiology for various cancers including non-small cell lung cancer. Exon 20 insertion is the third most commonly found EGFR mutation class in non-small cell lung cancers. A representative HER2 exon 20 insertion is A775_G776insYVMA, which accounts for around 85% of all HER2 mutations.

Due to our poor understanding of cancers that harbors these mutations and are unresponsive to EGFR inhibitors, effective targeted chemotherapy of these cancers is yet to be achieved. There still remains a need to develop a targeted chemotherapeutic substance possessing a strong antitumor activity against EGFR gene alterations with few side effects.

### RELEVANT PRIOR ART

Yu et al., Clinical Cancer Research, 19(8), 2013, pp 2240 - 2247

### TECHNICAL PROBLEM

The technical goal of the present invention lies in providing novel compounds capable of suppressing activity of HER2 kinase with a gene alteration. Furthermore, the technical goal of the present invention lies in providing selective inhibitor compounds that more strongly inhibits HER2 with a gene alteration than wild-type EGFR.

### SUMMARY OF THE INVENTION

Provided herein is a compound defined by Chemical Formula 1 or a tautomer, a stereoisomer or a pharmaceutically acceptable salt thereof.

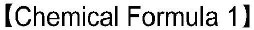

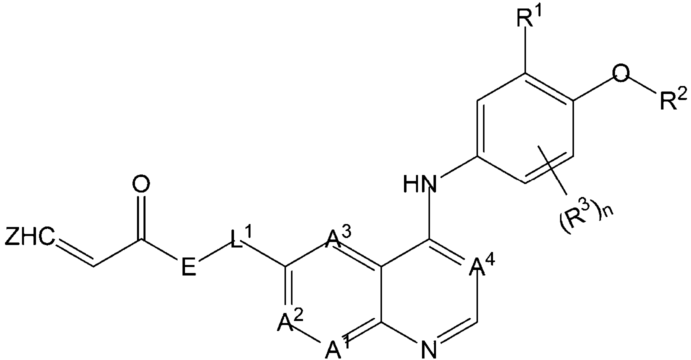

In Chemical Formula 1, A¹ and A³ are, independently of each other, N or CH, and A² and A⁴ are, independently of each other, N or CR*^{a}*, with said R*^{a}* being H, CN, C₁₋₄alkyl or C₁₋₄alkoxy. R¹ is C₁₋₄alkyl, C₁₋₄alkoxy, CF₃ or halogen. R² is (5+6)-membered fused bicyclic heteroaryl or (6+6)-membered fused bicyclic heteroaryl, and the (5+6)-membered fused bicyclic heteroaryl or (6+6)-membered fused bicyclic heteroaryl can be optionally substituted at one or more of its hydrogen atoms with a functional group independently selected from the group consisting of halogen, C₁₋₄alkyl and C₁₋₄alkoxy. In a specific embodiment, R² is one selected from the following group of fused bicyclic heteroaryl substituents: 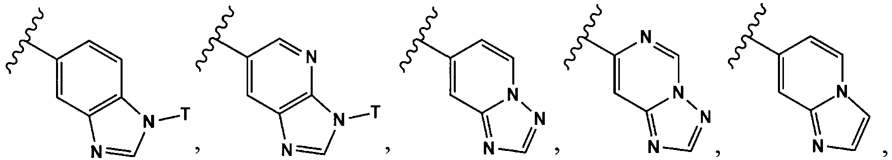 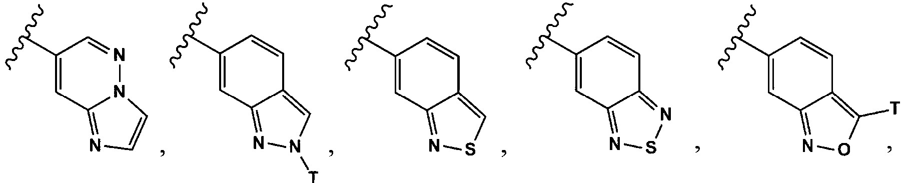 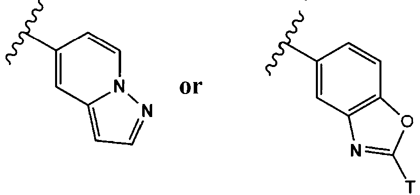 where T is H or C₁₋₄alkyl. Each R³ is independently halogen, C₁₋₄alkoxy or C₁₋₄alkyl, and n is an integer from 0 to 3. L¹ is -CH=CH-M, -CH=CHQ¹M, -NR^{b}Q¹M, -OQ¹M, - S(=O)₂Q¹M, 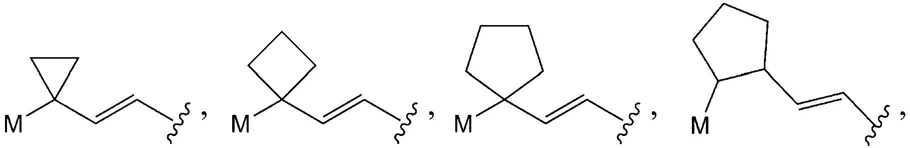 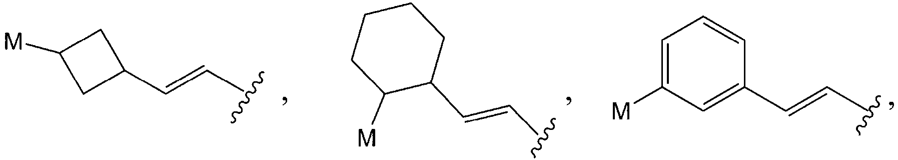 -N(Q²)C(=O)Q¹M, -C(=O)N(Q²)Q¹M, -C≡C-M or -C≡C-Q¹M, where Q¹ is C₁₋₃alkylene, Q² is H or C₁₋₃alkyl, R^{b} is H or C₁₋₃alkyl, and M is a single bond connecting to the functional 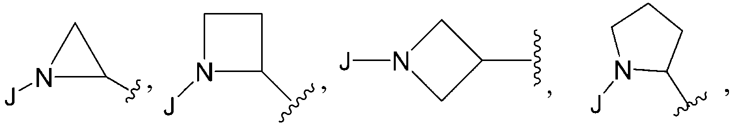 group E. E is 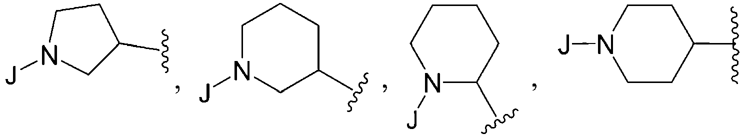 or -NR^{c}-, with J being a single bond connecting to the carbonyl group of -C(=O)CH=CHZ in Chemical Formula 1. R^{c} herein is H, C₁₋₄alkyl or C₃₋₆cycloalkyl, and Z is H, C₁₋₄alkyl, X, -C₁₋₃alkylene-X or -C₁₋₃alkylene-NR^{d}NR^{e}, where X is substituted or unsubstituted 5-6-membered heterocycloalkyl, with R^{d} and R^{e} being independently H or C₁₋₃alkyl, and said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

In an embodiment herein are provided compounds having a structure as defined in Chemical Formula 2 or a tautomer, a stereoisomer or a pharmaceutically acceptable salt thereof. 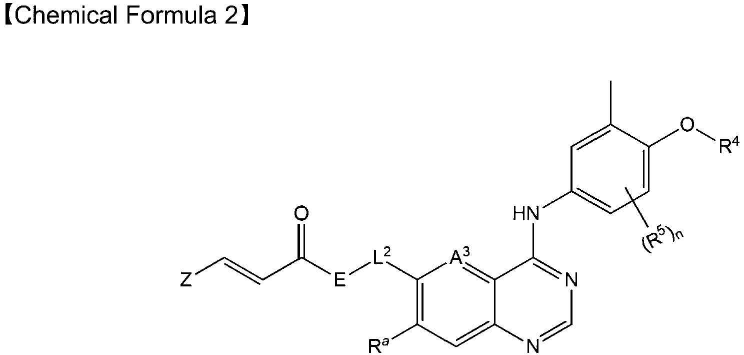 wherein R⁴ is 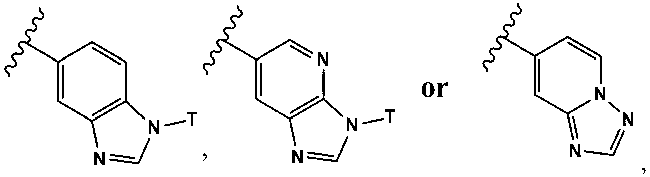 each R⁵ is independently halogen or C₁₋₄alkoxy, n is an integer from 0 to 3, and R*^{a}* is H, C₁₋₄alkyl or C₁₋₄alkoxy. L² is 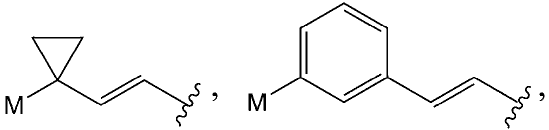 -CH=CH-M, - CH=CHQ¹M, -NR^{b}Q¹M or -OQ¹M. A³, E, Z, T, Q¹ and R^{b} are defined the same as in the foregoing Chemical Formula 1, with M indicating that the corresponding carbon is connected to the carbonyl group of C(=O)CH=CHZ via a single bond.

In a specific embodiment herein are provided compounds having a structure as defined in Chemical Formula 3 or a tautomer, a stereoisomer or a pharmaceutically acceptable salt thereof: 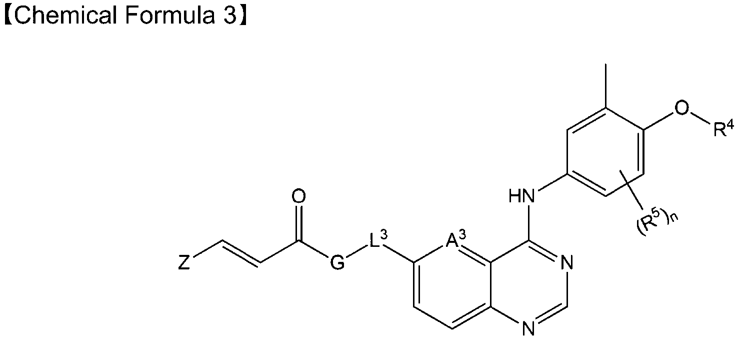 where A³, R⁴, R⁵ and Z are defined the same as in the foregoing Chemical Formula 2, L³ is -CH=CH-M, -CH=CHQ¹M, -NR^{b}Q¹M or -OQ¹M, and G is -NR^{c}-, 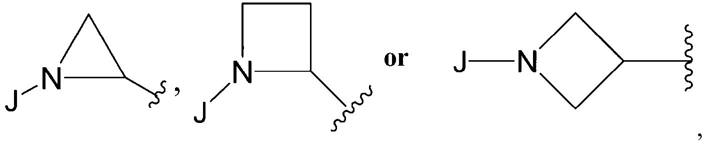 with said R^{c} being H, C₁₋₄alkyl or C₃₋₆cycloalkyl and said J being a single bond connecting to the carbonyl group of -C(=O)CH=CHZ.

In another specific embodiment herein are provided compounds having a structure as defined in Chemical Formula 4 or a tautomer, a stereoisomer or a pharmaceutically acceptable salt thereof: 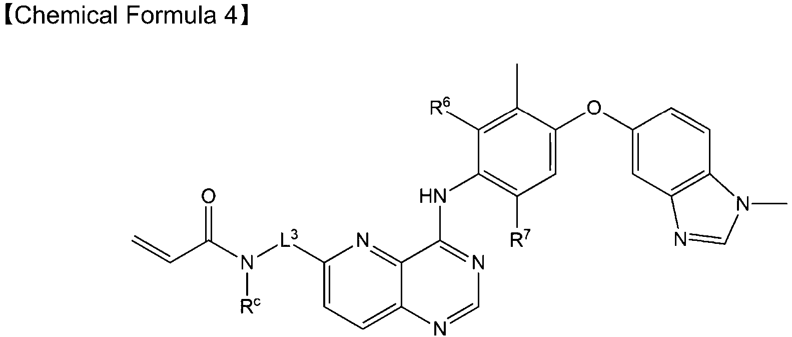 where L³ is -CH=CH-M, -CH=CHQ¹M, -NR^{b}Q¹M or -OQ¹M, with M being a single bond connecting to the functional group CH₂=CHC(=O)NR^{c}, and R^{c} being H, C₁₋₄alkyl or C₃₋₆cycloalkyl. R⁶ and R⁷ are, independently of each other, H or F, with Q¹ and R^{b} being defined the same as in the foregoing Chemical Formula 1.

In another aspect herein is provided a pharmaceutical composition for treating cancer comprising a compound described above or a tautomer, a stereoisomer or a pharmaceutically acceptable salt thereof in a therapeutically effective amount and pharmaceutically acceptable excipients.

In still another aspect herein is provided a method of treating cancer. This method comprises administering to a subject a therapeutically effective amount of a compound of the present invention, for example, a compound as defined by Chemical Formula 1 or a pharmaceutical composition comprising a compound of the present invention.

### ADVANTAGEOUS EFFECTS

The compounds of the present invention are capable of inhibiting the kinase activity of HER2, in particular, those HER2 harboring a gene alteration. In a specific embodiment, the compounds of the present invention are capable of inhibiting the kinase activity of HER2 harboring a gene alteration in exon 20. In a more specific embodiment, the compounds of the present invention are capable of inhibiting the kinase activity of HER2 harboring exon 20 insertions. In a most specific embodiment, the compounds of the present invention are capable of more strongly inhibiting the kinase activity of HER2 harboring exon 20 insertions than that of wild-type EGFR.

### DETAILED DESCRIPTION OF THE INVENTION

Reference will now be made in detail to certain embodiments, examples of which are illustrated herein. As an initial matter, it should be duly noted that the meanings of the terms or words used in this disclosure and/or the claims are not to be restricted to their ordinary or dictionary meanings, but should be construed in agreement with the technical idea of the present invention under the principle that an inventor can act as his own lexicographer.

While working examples will be described herein, it will be understood that they are intended to provide better understanding of the present invention. As these working examples are not meant to constitute and represent the technical idea of the present invention in its entirety, one skilled in the art would recognize that many equivalents or modifications to the working examples are possible and they also fall within the scope of the present invention.

Unless defined otherwise, all technical and scientific terms used herein have the same meaning as is commonly understood by one of skill in the art to which the claimed subject matter belongs. The present disclosure describes exemplar methods and/or materials but similar or equivalent methods and/or materials are also categorically encompassed by the present invention. Any numerical value used in this disclosure is to be understood to include before it, the term "about" even in the absence of the term. Any numerical range used in this disclosure includes both the starting value and the ending value respectively as the minimum and maximum defining the boundaries of the numerical range.

### HER2 and its mutation at exon 20

HER2, also known as ERBB2, is an acronym for the protein human epidermal growth factor receptor 2. HER2 is a member of the EGFR/ErbB protein family and has a tyrosine kinase activity. The wild-type messenger RNA sequence for HER2 is given a GenBank accession number of NM_004448.2. HER2 gene alterations can be broadly classified into categories of gene amplification, protein overexpression and gene mutation. HER2 gene amplification refers to a state where the HER2 gene has more copies of itself than normal, causing the overexpression of the HER2 protein. HER2 protein overexpression is a phenomenon caused by such factors as HER2 gene amplification in which abnormally many copies of HER2 protein are present on the cell surface that may lead abnormal growth and metastasis of tumor cells. HER2 gene mutation refers to alteration occurring on the DNA sequence of the HER2 gene itself, which may modify the functions of the HER2 protein. HER2 gene mutations are found in breast cancers, lung cancers, bladder cancers and ovary cancers. Among the mutations of HER2 is one called exon 20 insertion in which nucleotides are added to the wild-type sequence at exon 20 of HER2.

As used herein, the term "gene alteration" or simply "alteration" encompasses both gene mutations in which the corresponding gene has deviation in its base sequence from its wild-type gene as well as deviation from its wild-type regulatory controls such as overexpression and gene amplification for the corresponding gene.

As used herein, the term "insertion mutation", "insertion" or "insertion mutant" refers to addition of one or more nucleotide pairs to the DNA sequence or the resultant sequence thereby produced. For instance, HER2 exon 20 insertions refer to any mutation of at least one incidence of nucleotide addition whose length range from 3 to 18 nucleotides at a region in HER2 DNA corresponding to residue numbers 770 to 785 in the amino acid sequence.

HER2 exon 20 mutations and HER2 exon 20 mutants may include one or more mutations of point mutations, additions of 3 - 18 nucleotides and/or deletions between residues 770 and 785 in the amino acid sequence of HER2. Such one or more HER2 exon 20 mutations may take place at residues Ala775, Gly776, Ser779 and/or Pro780. Such one or more HER2 exon 20 mutations may be A775insV G776C, A775insYVMA, G776V, G776C V777insV, G776C V777insC, G776del insVV, G776del insVC and/or P780insGSP.

As used herein, the term "selective inhibition" or "selectively inhibit(s)" refers to the compounds of the present invention causing a greater drop in signaling activity for a kinase, the biological target for the compounds of the present invention, via either a direct or indirect interaction with this target than they do for a non-target kinase. For instance, a compound that selectively inhibits an exon 20 mutant HER2 over the wild-type EGFR has activity towards the exon 20 mutant isoforms greater by at least about two-fold (or at least about three-fold, about five-fold, about ten-fold, or about fifty-fold) than its activity towards the wild-type EGFR isoforms.

### Definitions

As used herein, the prefix "C_{x-y}" or "Cₓ-C_{y}", placed before a certain functional group, refers to the number of carbon atoms present in the main skeleton of that functional group. For instance, a C₁₋₄alkyl refers to an alkyl group of one to four carbon atoms, a C₁₋₃alkyl to an alkyl group of one to three carbon atoms. For example, a C₁-C₄alkoxy may include methoxy, ethoxy, *n-*propoxy, *i-*propoxy, *n-*butoxy, *sec-*butoxy and *t-*butoxy. For example, a C₃-C₆cycloalkyl may include cyclopropyl, cyclobutyl, cyclopentyl and cyclohexyl. Furthermore, for a cyclic functional group or a cyclic compound (including both carbocycles and heterocycles) that has a designation placed before it defining the number of atoms constituting the ring concerned, such cyclic functional group or compound herein is understood to have the defined number of atoms, *i*.e., carbon atoms and/or heteroatoms. For example, a 6-membered heteroaryl refers to a heteroaryl ring that contains one or more heteroatoms in addition to carbon atoms so that it has six atoms constituting the ring.

Unless otherwise specified herein, when a functional group is said to be linked to another part of a compound via a bond, the linkage can be present at any suitable atom of the functional group. For instance, a propyl group can refer to prop-1-yl as well as prop-2-yl.

As used herein, the term "halogen" refers to an atom selected from the group consisting of fluorine, chlorine, bromine and iodine.

As used herein, the term "C₁₋₄alkyl" refers to a monovalent, saturated hydrocarbon radical having the chemical formula of CₙH₂ₙ₊₁, when n is a natural number from 1 to 4. A C₁₋₄alkyl group encompasses every saturated hydrocarbon, straight chain or branched, that has from 1 to 4 carbon atoms, *e.g*., *n*-propyl, *i*-propyl, *sec*-butyl and *tert*-butyl, etc.

As used herein, the term "C₁₋₃alkylene" refers to a divalent, saturated hydrocarbon radical having the chemical formula of CₙH₂ₙ, when n is a natural number from 1 to 3. In the present disclosure, a functional group designated as a C₁₋₃alkylene can be a straight chain or a branched functional group and its two bonds of valence mentioned above may reside at the same carbon atom or they can be located at different carbon atoms. Examples of C₁₋₃alkylene groups include methylene, ethylenes (-CH₂CH₂- and -CH(CH₃)-) and propylenes (-CH₂CH₂CH₂-, -CH(CH₂CH₃)-, -CH₂CH(CH₃)- and -C(CH₃)₂-).

As used herein, the term "cycloalkyl" refers to a monovalent, saturated hydrocarbon radical forming a ring and having the chemical formula of CₙH₂ₙ₋₁. Examples of C₃₋₆cycloalkyl groups include cyclopropyl, cyclobutyl, cyclopentyl and cyclohexyl.

As used herein, the term "bicyclic" compound refers to a carbocycle or a heterocycle with two rings formed within it wherein the two constituent rings share one or more ring atoms between them. An atom in a bicyclic compound shared between both rings is termed a bridgehead atom. There are three classes of bicyclic compounds depending on the number of bridgehead atoms and the presence or absence of direct linkage between bridgehead atoms. A fused bicyclic compound has two bridgehead atoms directly linked to each other by a bond. Examples of fused bicyclic compounds include decalin, naphthalene, anthracene, phenanthrene, indole, benzofuran, purine and quinoline. A bicyclic compound with only a single bridgehead carbon is called a spirobicyclic compound. A bridged bicyclic compound has two bridgehead atoms that are not directly linked to each other by a bond but have one or more intervening ring atoms between them. Examples of bridged bicyclic compounds include norbornane, 7-oxabicyclo[2.2.1]heptane and adamantane.

In the present disclosure, a fused bicyclic compound can be referred to by designating the number of constituent ring atoms for each ring. For instance, thienopyridine, benzofuran and indole are (5+6)-membered fused bicyclic compounds. Similarly, purine, naphthalene, chromane, tetrahydroquinoline, quinoline, quinoxaline and pteridine are (6+6)-membered fused bicyclic compounds.

As used herein, the term "aryl" refers to an aromatic, monovalent C₆₋₁₄ hydrocarbon functional group with one to three aromatic rings in it. For instance, to an aryl group belong C₆, C₁₀, C₁₃ and C₁₄ radicals of aromatic hydrocarbon rings such as phenyl, naphthyl, anthracenyl and fluorenyl.

As used herein, the term "heteroaryl" refers to a monovalent, unsaturated ring having as ring atoms carbon and one or more heteroatoms selected from nitrogen, oxygen and sulfur which has an aromatic character. The ring system in a heteroaryl group can be monocyclic or fused bicyclic or tricyclic. In the present disclosure, the term heterocyclic excludes those rings that have direct linkage between oxygen and sulfur atoms such as -O-O-, -O-S- and -S-S- among the atoms constituting the ring. Limited examples of heteroaryl groups include pyrrolyl, imidazolyl, pyrazolyl, 2-pyridyl, 3-pyridyl, 4-pyridyl, pyrimidyl, triazolyl, quinazolinyl, 2-furyl, 3-furyl, benzofuryl, 2-thienyl, oxazolyl, isothiazolyl and thiadiazolyl. In the present disclosure, heteroaryl groups can be referred to by designating the heteroatom. For instance, heteroaryl groups with nitrogen as the heteroatom include pyrrolyl, imidazolyl, pyrazolyl and 2- pyridyl, but excludes furyl, oxazolyl and thienyl.

Fused bicyclic heteroaryl groups include, as (6+5)-membered fused bicyclic heteroaryls, benzimidazolyl, benzofuranyl, benzothiophenyl, isoindolyl, indazolyl, imidazopyridinyl, imidazopyrimidinyl, imidazopyridazinyl, pyrazolopyridinyl, pyrrolopyrimidinyl, pyrrolopyridinyl, pyrrolopyrazinyl, triazolopyridinyl, triazolopyrimidinyl, furyl, benzoxazolyl, benzothiazolyl, benzisothiazolyl, benzothiadiazolyl, anthranilyl, benzisooxazolyl, benzopyrazolyl and benzothiadiazolyl; as (6+6)-membered fused bicyclic heteroaryls, benzopyridinyl, benzopyrimidinyl, quinolinyl, isoquinolinyl, phthalzinyl, quinazolinyl, quinoxalinyl, naphthyridinyl, quinolizinyl and cinnolinyl.

As used herein, the term "heterocycloalkyl" refers to a monovalent radical that results from replacing at least one carbon atom from a cycloalkyl ring with a heteroatom selected from oxygen, nitrogen and sulfur, with each instance of replacement being independent from one another. Examples of heterocycloalkyl groups include pyrrolidinyl, imidazolidinyl, piperidinyl, piperazinyl, morpholinyl and thiazolidinyl.

As used herein, the term "substituted" placed before a certain functional group, refers, unless otherwise specified, to the structure resulting from that functional group as a consequence of replacing one or more hydrogen atoms with a designated functional group that is not hydrogen, provided that such substitution yields a chemically stable functional group with normal valences. Typically, functional groups for substitution are selected from halogen, CN, OH, C₁₋₆alkyl, C₃₋₆cycloalkyl, C₃₋₁₄heteroaryl, C₁₋₆alkoxy and CF₃. In the present disclosure, the mark " 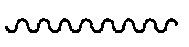 " attached to a certain atom in a functional group that is part of a compound indicates the functional group is directly linked to the remainder of the compound by a chemical bond to that atom.

In the present disclosure, the dashed line "---" placed in the line for a chemical bond indicates that the particular line for a chemical bond is an optional element that may be present or absent. For instance, a mark such as 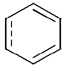 indicates that this structural formula may represent both benzene and 1,3-cyclohexdiene.

As used herein, the term "pharmaceutically acceptable salt(s)" of a compound refers to those salts which are suitable for use in contact with the tissues of humans or animals without undue toxicity, irritation, allergic response and the like, and do not harmfully affect the favorable biological activity of the compound. Pharmaceutically acceptable salts are well known in the art. For example, Berge et al. describes pharmaceutically acceptable salts in detail in J. Pharmaceutical Sciences (1977) 66: pp. 1-19. Non-limiting examples of pharmaceutically acceptable salts include, acid addition salts formed from hydrochloric acid, hydrobromic acid, phosphoric acid, sulfuric acid, perchloric acid, acetic acid, oxalic acid, maleic acid, tartaric acid, citric acid, succinic acid, malonic acid, adipic acid, alginic acid, ascorbic acid, aspartic acid, benzoic acid, benzenesulfonic acid and bisulfate as well as base addition salts such as alkali metal salts, alkaline earth metal salts, ammonium salts and quaternary ammonium [N⁺(C₁₋₄alkyl)₄] salts. Representative alkali or alkaline earth metal salts include sodium, lithium, potassium, calcium and magnesium.

Those skilled in the art will appreciate that certain of the compounds of the present invention may exist as tautomers. As a structural chemical formula can only depict one of the possible tautomeric forms, it should be noted all tautomeric forms of the compounds are contemplated to be within the scope of the present invention even when a single structural formula is used to represent the compound for the sake of convenience. Depending on the compound, one tautomeric form out of many can dominate, or the compound may exist at room temperature as a mixture of plural tautomeric forms, from which a particular tautomeric form can be isolated. Examples of tautomeric forms include pyridone form vs hydroxypyridine form as well as keto- form vs enol-form.

### Compounds

In one aspect, the present invention provides compounds as defined by Chemical Formula 1 or a tautomer, a stereoisomer or a pharmaceutically acceptable salt thereof.

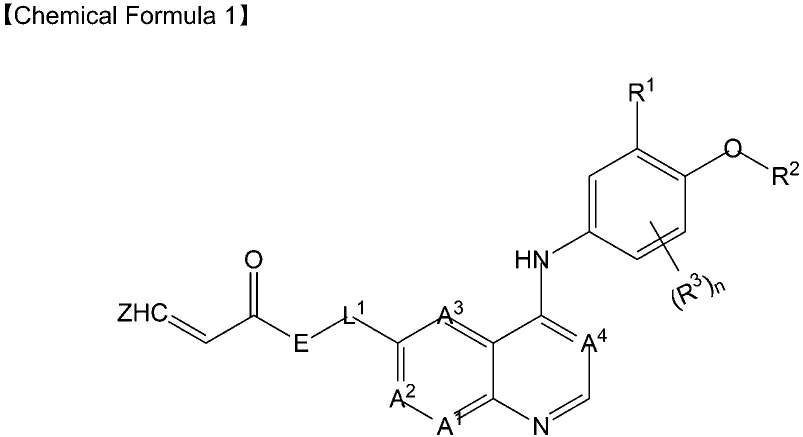

In Chemical Formula 1, A¹ and A³ are, independently of each other, N or CH. In Chemical Formula 1, A² and A⁴ are, independently of each other, N or CR*^{a}*, with said R*^{a}* being H, CN, C₁₋₄alkyl or C₁₋₄alkoxy.

In Chemical Formula 1, R¹ is C₁₋₄alkyl, C₁₋₄alkoxy, CF₃ or halogen.;
R² in Chemical Formula 1 is a (5+6)-membered fused bicyclic heteroaryl or (6+6)-membered fused bicyclic heteroaryl, and the (5+6)-membered fused bicyclic heteroaryl or (6+6)-membered fused bicyclic heteroaryl can be optionally substituted at one or more hydrogen atoms with a functional group independently selected from the group consisting of halogen, C₁₋₄alkyl and C₁₋₄alkoxy.

Each R³ in the compounds of Chemical Formula 1 is independently halogen, C₁₋₄alkoxy or C₁₋₄alkyl, and n is an integer from 0 to 3.

In the compounds of Chemical Formula 1, L¹ is a divalent substituent selected from -CH=CH-M, -CH=CHQ¹M, -NR^{b}Q¹M, -OQ¹M, -S(=O)₂Q¹M, 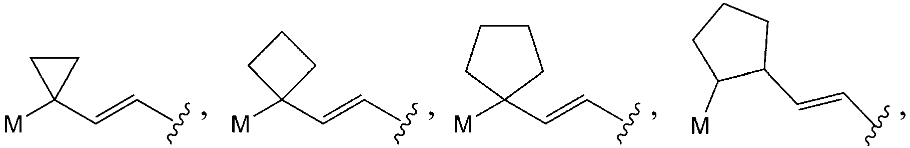 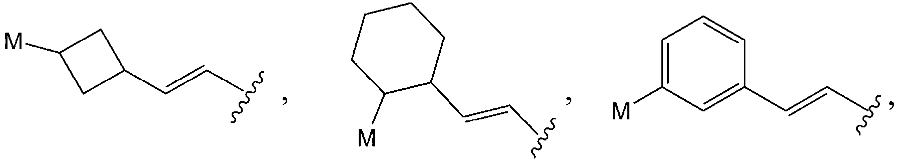 -N(Q²)C(=O)Q¹M, -C(=O)N(Q²)Q¹M, -C≡C-M and -C≡C-Q¹M, where Q¹ is C₁₋₃alkylene, Q² is H or C₁₋₃alkyl, R^{b} is H or C₁₋₃alkyl, and M is a single bond connecting to the functional group E. In other words, it represents that in the case of divalent substituent L¹ being - CH=CHQ¹M, the carbon of the vinyl group in -CH=CHQ¹M that is bonded to Q¹ has linkage to the functional group E of Chemical Formula 1, and the "-" mark represents a direct linkage in a direction opposite the aforementioned functional group E, *i.e.*, it means that the other carbon in the vinyl group is directly linked to the fused bicyclic ring of Chemical Formula 1. Similarly, it indicates that when L¹ is 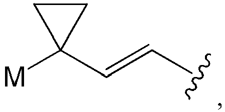 the carbon in this particular L¹ marked with M is directly linked by a single bond to the functional group E, and the vinyl group carbon marked with " 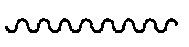 " is directly linked within the compound of Chemical Formula 1, in a direction opposite to the functional group E, *i*.*e*., to the fused bicyclic ring of Chemical Formula 1.

E in Chemical Formula 1 is selected from the following divalent functional groups: 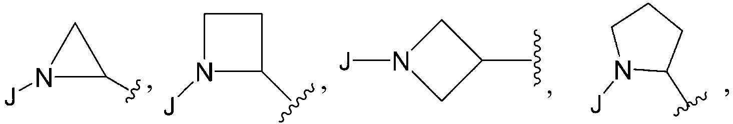 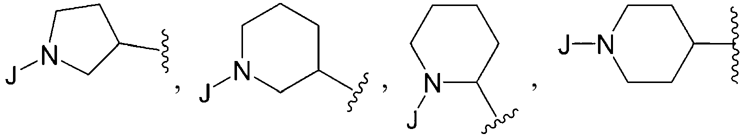 and -NR^{c}-; where R^{c} is H, C₁₋₄alkyl or C₃₋₆cycloalkyl. J and " 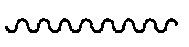 " in the divalent functional group E represent, similar to as described above for the divalent functional group L¹, that the carbon marked with J is directly linked by a single bond to the functional group of -C(=O)CH=CHZ in Chemical Formula 1 while the ring carbon marked with " 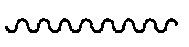 " is directly linked within the compound of Chemical Formula 1, in a direction opposite to the functional group of -C(=O)CH=CHZ, *i*.*e*., to L¹_{.}

In the compound of Chemical Formula 1, Z is H, C₁₋₄alkyl, X, -C₁₋₃alkylene-X or - C₁₋₃alkylene-NR^{d}NR^{e}, where X is substituted or unsubstituted 5-6-membered heterocycloalkyl, and R^{d} and R^{e} are independently H or C₁₋₃alkyl, with said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

In a specific embodiment of the compounds of Chemical Formula 1, at least one of A¹ to A⁴ is nitrogen. In another specific embodiment of the compounds of Chemical Formula 1, L¹ is -CH=CH-Q¹, Q¹ is CH₂ or -CH(CH₃)-, E is -NR^{c}- and Z is H. In still another specific embodiment, L¹ is -CH≡CH-Q¹, Q¹ is CH₂ and E is -NR^{c}-.

In yet another specific embodiment, R² of Chemical Formula 1 is selected from the following fused bicyclic heteroaryl groups: 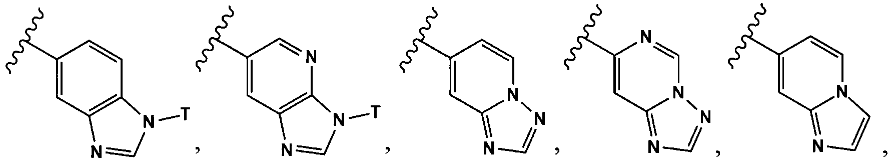 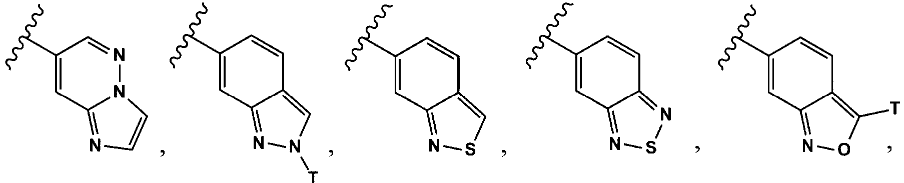 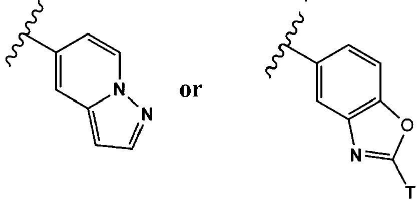 where T is H or C₁₋₄alkyl. The mark " 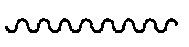 " here represents that the carbon atom marked with it is directly linked to the remainder of the compound *i.e.*, to the oxygen atom of the moiety OR² by a single bond.

In a more specific embodiment, 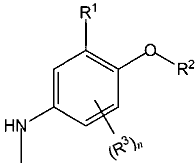 in the compound of the present invention according to Chemical Formula 1 is selected from the following functional groups: 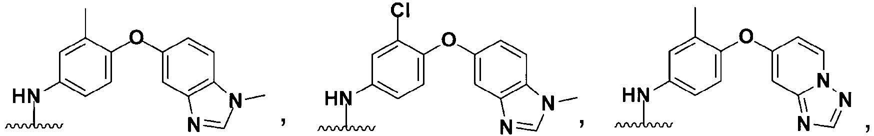 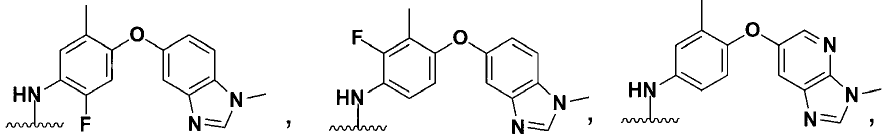 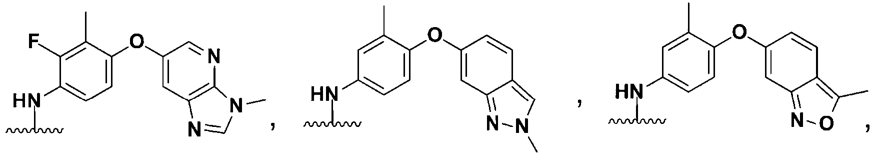 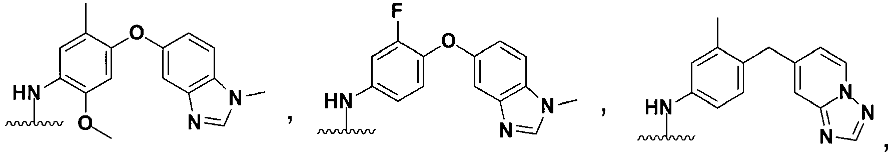 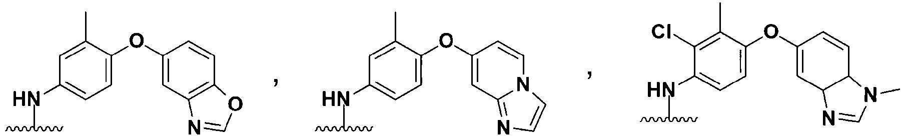 wherein the mark " 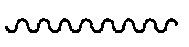 " here represents that the corresponding nitrogen atom is directly linked to the remainder of the compound *i.e.*, to the heterobicyclic ring containing A¹ to A³ by a single bond.

In a further specific embodiment, the L¹-E-C(=O)CH=CHZ moiety in the compound of the present invention according to Chemical Formula 1 is selected from the following functional groups:

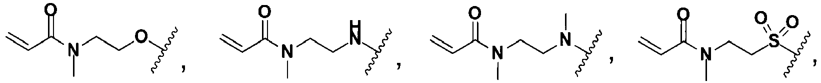

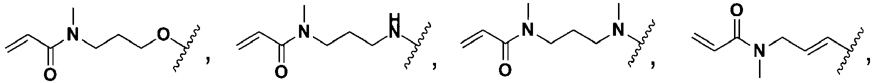

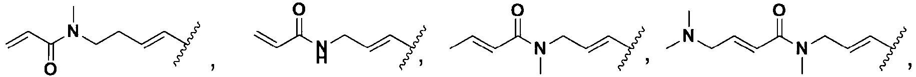

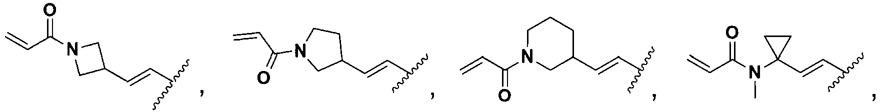

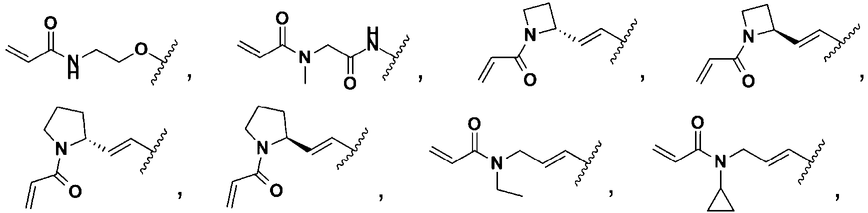

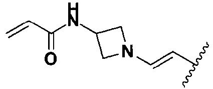

The mark " 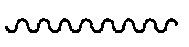 " here indicates, similar to as described above, that the atom marked with 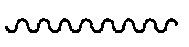 in the relevant L¹-E-C(=O)CH=CHZ moiety is directly linked to the remainder of the compound of Chemical Formula 1, *i.e.*, to the heterobicyclic ring containing A¹ to A³ by a single bond.

In an embodiment, the compounds of the present invention have a structure as defined in Chemical Formula 2.

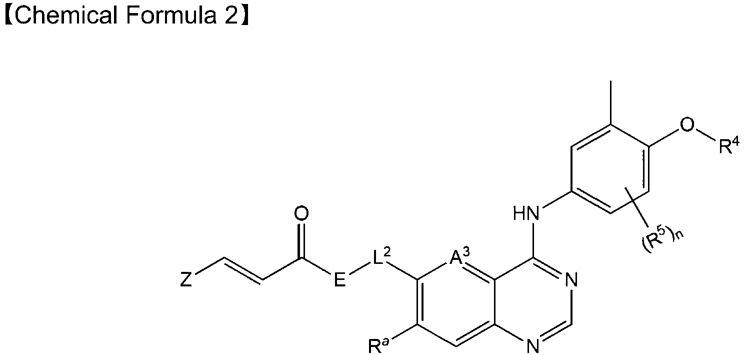

R⁴ in the compounds of Chemical Formula 2 is selected from the following heterobicyclic substituents:

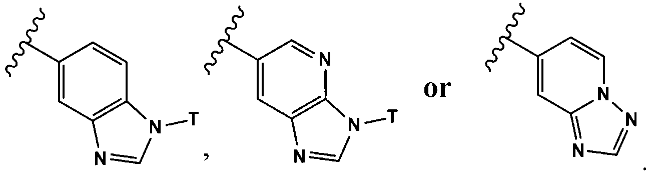

The T here is H or C₁₋₄alkyl.

Each R⁵ in the compounds of Chemical Formula 2 is independently halogen or C₁₋₄alkoxy, and n is an integer from 0 to 3.

A³ in the compounds of Chemical Formula 2 is N or CH, and R*^{a}* is H, C₁₋₄alkyl or C₁₋₄alkoxy.

L² in Chemical Formula 2 is a divalent functional group selected from 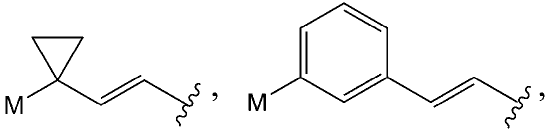 CH=CH-M, -CH=CHQ¹M, - NR^{b}Q¹M and -OQ¹M, where Q¹ is C₁₋₃alkylene, R^{b} is H or C₁₋₃alkyl. M and " 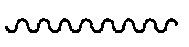 " in L² represents, similar to as described above for the divalent functional group L¹, that the carbon marked with M is directly linked by a single bond to the functional group of E in Chemical Formula 2 while the ring carbon marked with " 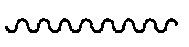 " is directly linked within the compound of Chemical Formula 2, in a direction opposite to the functional group E, *i.e.*, to the heterobicyclic ring containing A³ by a single bond.

E in Chemical Formula 2, identical to E of Chemical Formula 1, is a divalent functional group selected from 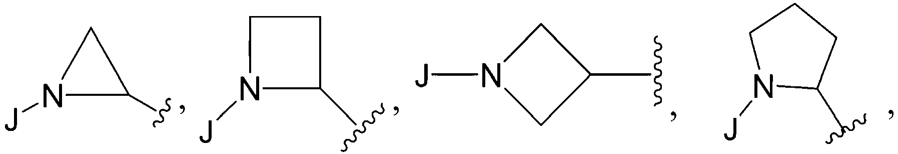 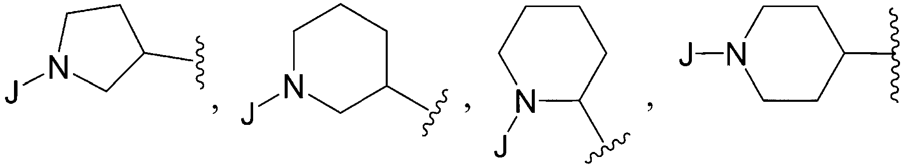 and -NR^{c}-, where R^{c} is identical to the definition in Chemical Formula 1, H, C₁₋₄alkyl or C₃₋₆cycloalkyl and J is a single bond connecting to the carbonyl group of -C(=O)CH=CHZ.

Z in Chemical Formula 2, identical to Z of Chemical Formula 1, is H, C₁₋₄alkyl, X, -C₁₋₃alkylene-X or -C₁₋₃alkylene-NR^{d}NR^{e}, where X is substituted or unsubstituted 5-6-membered heterocycloalkyl, and R^{d} and R^{e} are independently H or C₁₋₃alkyl, with said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

In a specific embodiment of the compounds of Chemical Formula 2, A³ is N.

In another specific embodiment of the compounds of Chemical Formula 2, R⁵ is fluorine (F).

In still another specific embodiment, Z is H, CH₃, 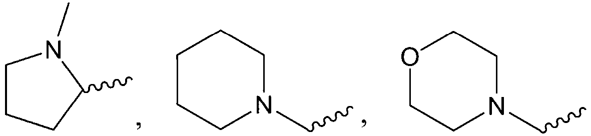 or -CH₂N(CH₃)₂.

In another embodiment, the compounds of the present invention have a structure as defined in Chemical Formula 3: 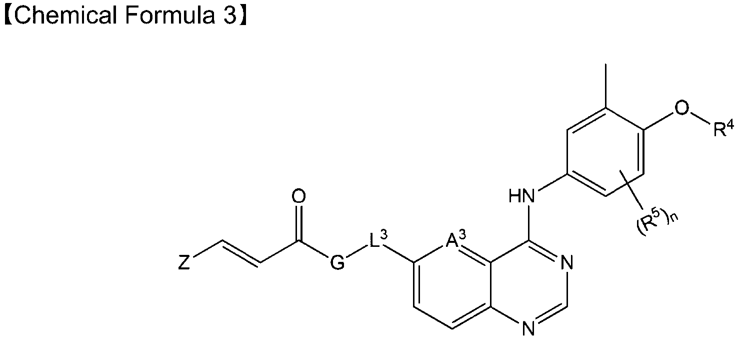 where R⁴ is 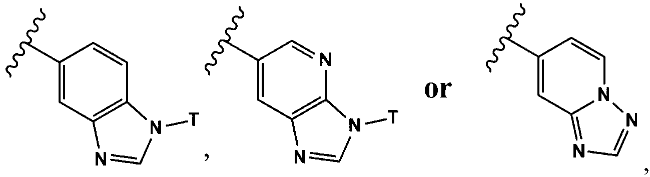 with said T being H or C₁₋₄alkyl.

Each R⁵ is independently halogen and n is an integer from 0 to 3.

A³ is N or CH.

L³ is -CH=CH-M, -CH=CHQ¹M, -NR^{b}Q¹M or -OQ¹M, where Q¹ is C₁₋₃alkylene, R^{b} is H or C₁₋₃alkyl, and M is a single bond connecting to the functional group G.

G is -NR^{c}-, 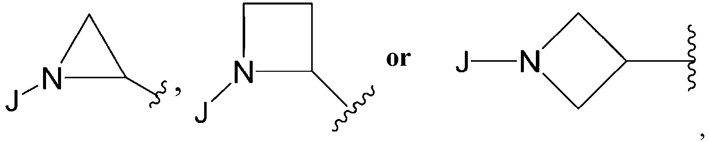 with said R^{c} being H, C₁₋₄alkyl or C₃₋₆cycloalkyl and said J being a single bond connecting to the carbonyl group of -C(=O)CH=CHZ.

Z is H, C₁₋₄alkyl, X, -C₁₋₃alkylene-X or -C₁₋₃alkylene-NR^{d}NR^{e}, where X is substituted or unsubstituted 5-6-membered heterocycloalkyl, and R^{d} and R^{e} are independently H or C₁₋₃alkyl, with said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

In a more specific embodiment, the compounds of the present invention have -NR^{c}-, 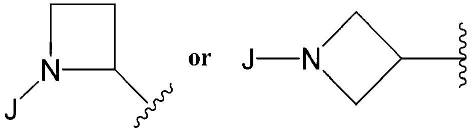 for G in Chemical Formula 3.

In yet another embodiment, the compounds of the present invention have a structure as defined in Chemical Formula 4: 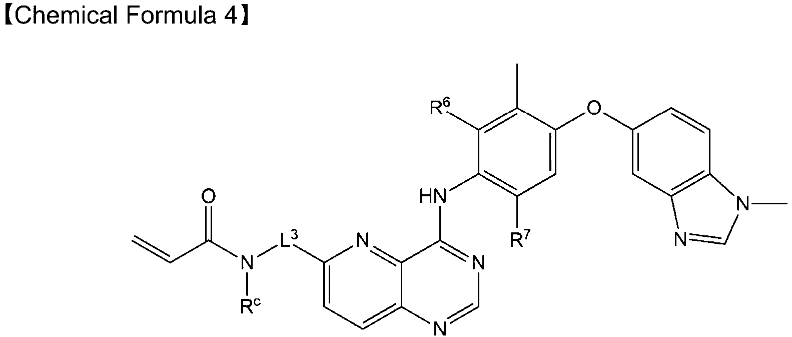 where L³ is -CH=CH-M, -CH=CHQ¹M, -NR^{b}Q¹M or -OQ¹M, with said Q¹ being C₁₋₃alkylene, R^{b} being H or C₁₋₃alkyl, and M being a single bond connecting to the functional group CH₂=CHC(=O)NR^{c}.

R^{c} is H, C₁₋₄alkyl or C₃₋₆cycloalkyl and R⁶ and R⁷ are, independently of each other, H or F.

In a more specific embodiment, the compounds of Chemical Formula 4 have methyl for R^{c}.

In yet still another embodiment, the compounds of the present invention have a structure as defined in Chemical Formula 5: 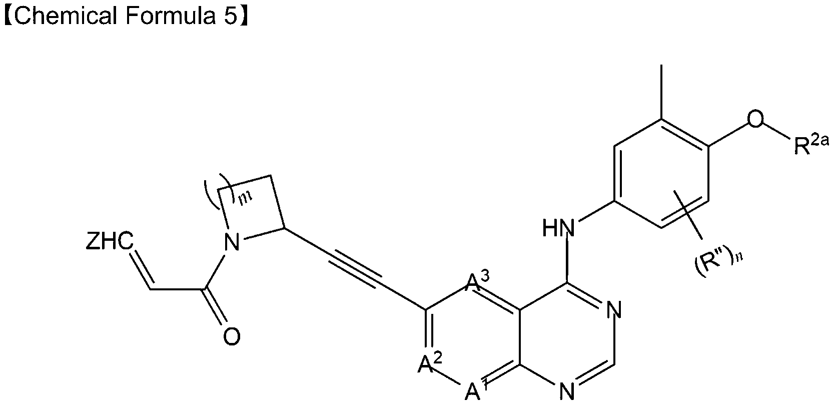 wherein A¹ and A³ are, independently of each other, N or CH, and A² is N or CR*^{a}*, with said R*^{a}* being H, C₁₋₄alkyl or C₁₋₄alkoxy.

R^{2a} in the compounds of Chemical Formula 5 is selected from the following heterobicyclic substituents: 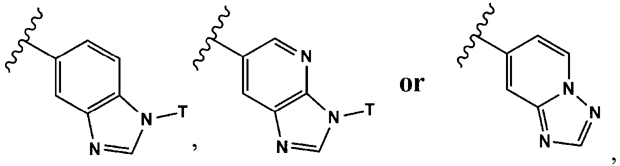 where T is H or C₁₋₄alkyl.

Each R" in the compounds of Chemical Formula 5 is independently halogen and n is an integer from 0 to 3. m in the compounds of Chemical Formula 5 is an integer from 1 to 3.

Z in Chemical Formula 5, identical to Z of Chemical Formula 1, is H, C₁₋₄alkyl, X, -C₁₋₃alkylene-X or -C₁₋₃alkylene-NR^{d}NR^{e}, where X is substituted or unsubstituted 5-6-membered heterocycloalkyl, and R^{d} and R^{e} are independently H or C₁₋₃alkyl, with said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

In a specific embodiment, the compounds of Chemical Formula 5 have 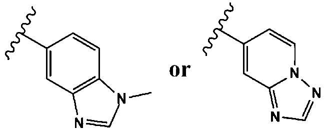 for R^{2a}.

In another specific embodiment, the compounds of Chemical Formula 5 have CH for A¹ and N for A³.

In the most specific embodiment, the compounds of the present invention are those selected from below, or a tautomer, a stereoisomer or a pharmaceutically acceptable salt thereof:
(1) *N*-(2-((4-((4-([1,2,4]triazolo[1,5-*a*]pyridin-7-yloxy)-3-methylphenyl)amino)quinazolin-6-yl)oxy)ethyl)-*N*-methylacrylamide,
(2) *N*-(2-((4-((4-([1,2,4]triazolo[1,5-*a*]pyridin-7-yloxy)-3-methylphenyl)amino)-7-methoxyquinazolin-6-yl)oxy)ethyl)-*N*-methylacrylamide,
(3) *N*-(2-((4-((4-([1,2,4]triazolo[1,5-*a*]pyridin-7-yloxy)-3-methylphenyl)amino)pyrido[3,2-*d*]pyrimidin-6-yl)oxy)ethyl)-*N*-methylacrylamide,
(4) *N*-(2-((8-((4-([1,2,4]triazolo[1,5-*a*]pyridin-7-yloxy)-3-methylphenyl)amino)pyrimido[5,4-*d*]pyrimidin-2-yl)oxy)ethyl)-*N*-methylacrylamide,,
(5) *N*-methyl-*N*-(2-((4-((3-methyl-4-((1-methyl-1*H*-benzo[*d*]imidazol-5-yl)oxy)phenyl)amino)quinazolin-6-yl)oxy)ethyl)acrylamide,
(6) *N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)oxy)ethyl)acrylamide,
(7) *N*-(2-((4-((3-methyl-4-((1-methyl-1*H*-benzo[*d*]imidazol-5-yl)oxy)phenyl)amino)pyrido[3,2-*d*]pyrimidin-6-yl)oxy)ethyl)acrylamide,
(8) *N*-methyl-*N*-(2-((8-((3-methyl-4-((1-methyl-1*H*-benzo[*d*]imidazol-5-yl)oxy)phenyl)amino)pyrimido[5,4-*d*]pyrimidin-2-yl)oxy)ethyl)acrylamide,
(9) *N*-methyl-*N*-(3-((4-((3-methyl-4-((1-methyl-1*H*-benzo[*d*]imidazol-5-yl)oxy)phenyl)amino)pyrido[3,2-*d*]pyrimidin-6-yl)oxy)propyl)acrylamide,
(10) *N*-(2-((4-((3-methyl-4-((1-methyl-1*H*-benzo[*d*]imidazol-5-yl)oxy)phenyl)amino)quinazolin-6-yl)oxy)ethyl)acrylamide,
(11) *N*-(2-((4-((4-([1,2,4]triazolo[1,5-*a*]pyridin-7-yloxy)-3-methylphenyl)amino)pyrido[3,2-*d*]pyrimidin-6-yl)amino)ethyl)-*N*-methylacrylamide,
(12) *N*-(2-((8-((4-([1,2,4]triazolo[1,5-*a*]pyridin-7-yloxy)-3-methylphenyl)amino)pyrimido[5,4-*d*]pyrimidin-2-yl)amino)ethyl)-*N*-methylacrylamide,
(13) *N*-(2-((4-((4-([1,2,4]triazolo[1,5-*a*]pyridin-7-yloxy)-3-methylphenyl)amino)pyrido[3,2-*d*]pyrimidin-6-yl)(methyl)amino)ethyl)-*N-*methylacrylamide,
(14) *N*-(2-((8-((4-([1,2,4]triazolo[1,5-*a*]pyridin-7-yloxy)-3-methylphenyl)amino)pyrimido[5,4-*d*]pyrimidin-2-yl)(methyl)amino)ethyl)-*N-*methylacrylamide,
(15) *N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)amino)ethyl)acrylamide,
(16) *N*-methyl-*N*-(2-((8-((3-methyl-4-((1-methyl-1*H*-benzo[*d*]imidazol-5-yl)oxy)phenyl)amino)pyrimido[5,4-*d*]pyrimidin-2-yl)amino)ethyl)acrylamide,
(17) *N*-methyl-*N*-(3-((4-((3-methyl-4-((1-methyl-1*H*-benzo[*d*]imidazol-5-yl)oxy)phenyl)amino)pyrido[3,2-*d*]pyrimidin-6-yl)amino)propyl)acrylamide,
(18) *N*-methyl-*N*-(2-(methyl(4-((3-methyl-4-((1-methyl-1*H*-benzo[*d*]imidazol-5-yl)oxy)phenyl)amino)pyrido[3,2-*d*]pyrimidin-6-yl)amino)ethyl)acrylamide,
(19) *N*-methyl-*N*-(2-(methyl(8-((3-methyl-4-((1-methyl-1*H*-benzo[*d*]imidazol-5-yl)oxy)phenyl)amino)pyrimido[5,4-*d*]pyrimidin-2-yl)amino)ethyl)acrylamide,
(20) *N*-methyl-*N*-(3-(methyl(4-((3-methyl-4-((1-methyl-1*H*-benzo[*d*]imidazol-5-yl)oxy)phenyl)amino)pyrido[3,2-*d*]pyrimidin-6-yl)amino)propyl)acrylamide,
(21) *N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)amino)-2-oxoethyl)acrylamide,
(22) (*E*)-*N*-methyl-*N*-(3-((4-((3-methyl-4-((1-methyl-1*H*-benzo[*d*]imidazol-5-yl)oxy)phenyl)amino)quinazolin-6-yl)aryl)acrylamide,
(23) (*E*)-*N*-methyl-*N*-(3-(4-((3-methyl-4-((1-methyl-1*H*-benzo[*d*]imidazol-5-yl)oxy)phenyl)amino)pyrido[3,2-*d*]pyrimidin-6-yl)aryl)acrylamide,
(24) (*E*)-*N*-methyl-*N*-(3-(8-((3-methyl-4-((1-methyl-1*H*-benzo[*d*]imidazol-5-yl)oxy)phenyl)amino)pyrimido[5,4-*d*]pyrimidin-2-yl)allyl)acrylamide,
(25) (*E*)-*N*-(3-(4-((3-chloro-4-((1-methyl-1*H*-benzo[*d*]imidazol-5-yl)oxy)phenyl)amino)pyrido[3,2-*d*]pyrimidin-6-yl)allyl)-*N*-methylacrylamide,
(26) (*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-4-en-1-yl)acrylamide,
(27) (*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)piperidin-1-yl)prop-2-en-1-one,
(28) (*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)azetidin-1-yl)prop-2-en-1-one,
(29) (*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)pyrrolidin-1-yl)prop-2-en-1-one,
(30) (*E*)-*N*-methyl-*N*-(1-(2-(4-((3-methyl-4-((1-methyl-1*H*-benzo[*d*]imidazol-5-yl)oxy)phenyl)amino)pyrido[3,2-*d*]pyrimidin-6-yl)vinyl)cyclopropyl)acrylamide,
(31) (*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)but-2-enamide,
(32) (*E*)-4-(dimethylamino)-*N*-methyl-*N*-((*E*)-3-(4-((3-methyl-4-((1-methyl-1*H-*benzo[*d*]imidazol-5-yl)oxy)phenyl)amino)pyrido[3,2-*d*]pyrimidin-6-yl)allyl)but-2-enamide,
(33) (*E*)-*N*-(3-(4-((3-methyl-4-((1-methyl-1*H*-benzo[*d*]imidazol-5-yl)oxy)phenyl)amino)pyrido[3,2-*d*]pyridin-6-yl)allyl)acrylamide,
(34) (*E*)-*N*-(3-(4-((3-methyl-4-((3-methyl-3*H*-imidazo[4,5-*b*]pyridin-6-yl)oxy)phenyl)amino)pyrido[3,2-*d*]pyrimidin-6-yl)allyl)acrylamide,
(35) (*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)acrylamide,
(36) (*E*)-*N*-(3-(4-((2-chloro-3-methyl-4-((1-methyl-1*H*-benzo[*d*]imidazol-5-yl)oxy)phenyl)amino)pyrido[3,2-*d*]pyrimidin-6-yl)allyl)acrylamide,
(37) (*E*)-*N*-(3-(4-((3-methyl-4-(pyrazolo[1,5-*a*]pyridin-5-yloxy)phenyl)amino)pyrido[3,2-*d*]pyrimidin-6-yl)allyl)acrylamide,
(38) (*E*)-*N*-(3-(4-((4-([1,2,4]triazolo[1,5-*a*]pyridin-7-yloxy)-3-methylphenyl)amino)pyrido[3,2-*d*]pyrimidin-6-yl)allyl)acrylamide,
(39) (*E*)-*N*-(3-(4-((4-(imidazo[1,2-*a*]pyridin-7-yloxy)-3-methylphenyl)amino)pyrido[3,2-*d*]pyrimidin-6-yl)allyl)acrylamide,
(40) (*E*)-4-(dimethylamino)-*N*-((*E*)-3-(4-((3-methyl-4-((1-methyl-1H-benzo[*d*]imidazol-5-yl)oxy)phenyl)amino)pyrido[3,2-*d*]pyrimidin-6-yl)allyl)but-2-enamide,
(41) (*E*)-4-(dimethylamino)-*N*-((*E*)-3-(4-((2-fluoro-3-methyl-4-((1-methyl-1*H-*benzo[*d*]imidazol-5-yl)oxy)phenyl)amino)pyrido[3,2-*d*]pyrimidin-6-yl)allyl)but-2-enamide,
(42) (*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(43) (*E*)-*N*-methyl-*N*-(3-(4-((3-methyl-4-((3-methyl-3*H*-imidazo[4,5-*b*]pyridin-6-yl)oxy)phenyl)amino)pyrido[3,2-*d*]pyrimidin-6-yl)allyl)acrylamide,
(44) (*E*)-*N*-methyl-*N*-(3-(4-((3-methyl-4-((3-methyl-3H-imidazo[4,5-*b*]pyridin-6-yl)oxy)phenyl)amino)pyrido[3,2-*d*]pyrimidin-6-yl)allyl)acrylamide,
(45) (*E*)-*N*-(3-(4-((2-fluoro-5-methyl-4-((1-methyl-1*H*-benzo[*d*]imidazol-5-yl)oxy)phenyl)amino)pyrido[3,2-*d*]pyrimidine -6-yl)allyl)-*N*-methylacrylamide,
(46) (*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(47) (*E*)-*N*-(3-(4-((2-fluoro-3-methyl-4-((3-methyl-3*H*-imidazolo[4,5-*b*]pyridin-6-yl)oxy)phenyl)amino)pyrido[3,2-*d*]pyrimidin-6-yl)allyl)-*N*-methylacrylamide,
(48) (*E*)-1-(3-(2-(4-((3-methyl-4-((2-methyl-2*H*-indazol-6-yl)oxy)phenyl)amino)pyrido[3,2-*d*]pyrimidin-6-yl)vinyl)azetidin-1-yl)prop-2-en-1-one,
(49) (*E*)-*N*-ethyl-*N*-(3-(4-((3-methyl-4-((1-methyl-1*H*-benzo[*d*]imidazol-5-yl)oxy)phenyl)amino)pyrido[3,2-*d*]pyrimidin-6-yl)allyl)acrylamide,
(50) (*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(51) (*S*,*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(52) (*R*,*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(53) (*E*)-*N*-(*tert*-butyl)-*N*-(3-(4-((3-methyl-4-((1-methyl-1*H*-benzo[*d*]imidazol-5-yl)oxy)phenyl)amino)pyrido[3,2-*d*]pyrimidin-6-yl)allyl)acrylamide,
(54) (*E*)-*N*-(3-(4-((2-methoxy-5-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(55) (*E*)-*N*-(3-(4-((3-fluoro-4-((1-methyl-1*H*-benzo[*d*]imidazol-5-yl)oxy)phenyl)amino)pyrido[3,2-*d*]pyrimidin-6-yl)allyl)-*N*-methylacrylamide,
(56) (*E*)-*N*-(3-(4-((4-([1,2,4]triazolo[1,5-*a*]pyridin-7-ylmethyl)-3-methylphenyl)amino)pyrido[3,2-*d*]pyrimidin-6-yl)allyl)-*N*-methylacrylamide,
(57) (*E*)-*N*-(3-(4-((4-(benzo[*d*]oxazol-5-yloxy)-3-methylphenyl)amino)pyrido[3,2-*d*]pyrimidin-6-yl)allyl)-*N*-methylacrylamide,
(58) (*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-3-((*R*)-1-methylpyrrolidin-2-yl)acrylamide,
(59) (*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-(piperidin-1-yl)but-2-enamide,
(60) (*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-morpholinobut-2-enamide,
(61) (*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-(piperidin-1-yl)but-2-enamide,
(62) (*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-morpholinobut-2-enamide,
(63) (*E*)-*N*-methyl-*N*-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)phenyl)acrylamide,
(64) (*E*)-*N*-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)phenyl)acrylamide,
(65) *N*-(3-(4-((4-([1,2,4]triazolo[1,5-*a*]pyridin-7-yloxy)-3-methylphenyl)amino)quinazolin-6-yl)prop-2 -yn-1-yl)-*N*-methylacrylamide,
(66) *N*-(3-(4-((4-([1,2,4]triazolo[1,5-*a*]pyridin-7-yloxy)-3-methylphenyl)amino)pyrido[2,3-*d*]pyrimidin-6-yl)prop-2-yn-1-yl)-*N*-methylacrylamide,
(67) *N*-(3-(4-((4-([1,2,4]triazolo[1,5-*a*]pyridin-7-yloxy)-3-methylphenyl)amino)pyrido[3,4-*d*]pyrimidin-6-yl)prop-2-yn-1-yl)-*N*-methylacrylamide,
(68) *N*-(3-(4-((4-([1,2,4]triazolo[1,5-*a*]pyridin-7-yloxy)-3-methylphenyl)amino)pyrido[3,2-*d*]pyrimidin-6-yl)prop-2-yn-1-yl)-*N*-methylacrylamide,
(69) *N*-(3-(8-((4-([1,2,4]triazolo[1,5-*a*]pyridin-7-yloxy)-3-methylphenyl)amino)pyrimido[5,4-*d*]pyrimidin-2-yl)prop-2-yn-1-yl)-*N*-methylacrylamide,
(70) *N*-(3-(4-((4-([1,2,4]triazolo[1,5-*a*]pyridin-7-yloxy)-3-methylphenyl)amino)-3-cyanoquinolin-6-yl)prop-2-yn-1-yl)-*N*-methylacrylamide,
(71) *N*-methyl-*N*-(3-(4-((3-methyl-4-((1-methyl-1*H*-benzo[*d*]imidazol-5-yl)oxy)phenyl)amino)pyrido[3,2-*d*]pyrimidin-6-yl)prop-2-yn-1-yl)acrylamide,
(72) *N*-methyl-*N*-(3-(8-((3-methyl-4-((1-methyl-1*H*-benzo[*d*]imidazol-5-yl)oxy)phenyl)amino)pyrido[5,4-*d*]pyrimidin-2-yl)prop-2-yn-1-yl)acrylamide,
(73) *N*-(3-(4-((4-([1,2,4]triazolo[1,5-*a*]pyridin-7-yloxy)-3-methylphenyl)amino)-7-methoxyquinazolin-6-yl) prop-2-yn-1-yl)-*N*-methylacrylamide,
(74) (*R*)-1-(2-((4-((4-([1,2,4]triazolo[1,5-*a*]pyridin-7-yloxy)-3-methylphenyl)amino)quinazolin-6-yl)ethynyl)pyrrolidin-1-yl)prop-2-en-1-one,
(75) (*S*)-1-(2-((4-((4-([1,2,4]triazolo[1,5-*a*]pyridin-7-yloxy)-3-methylphenyl)amino)quinazolin-6-yl)ethynyl)pyrrolidin-1-yl)prop-2-en-1-one,
(76) (*R*)-1-(2-((4-((4-([1,2,4]triazolo[1,5-*a*]pyridin-7-yloxy)-3-methylphenyl)amino)quinazolin-6-yl)ethynyl)piperidin-1-yl)prop-2-en-1-one,
(77) (*S*)-1-(2-((4-((4-([1,2,4]triazolo[1,5-*a*]pyridin-7-yloxy)-3-methylphenyl)amino)quinazolin-6-yl)ethynyl)piperidin-1-yl)prop-2-en-1-one,
(78) (*R*)-1-(2-((4-((4-([1,2,4]triazolo[1,5-*a*]pyridin-7-yloxy)-3-methylphenyl)amino)quinazolin-6-yl)ethynyl)azetidin-1-yl)prop-2-en-1-one,
(79) (*S*)-1-(2-((4-((4-([1,2,4]triazolo[1,5-*a*]pyridin-7-yloxy)-3-methylphenyl)amino)quinazolin-6-yl)ethynyl)azetidin-1-yl)prop-2-en-1-one,
(80) (*R*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)pyrrolidin-1-yl)prop-2-en-1-one,
(81) (*S*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)pyrrolidin-1-yl)prop-2-en-1-one,
(82) (*R*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)piperidin-1-yl)prop-2-en-1-one,
(83) (*R*)-1-(2-((8-((4-([1,2,4]triazolo[1,5-*a*]pyridin-7-yloxy)-3-methylphenyl)amino)pyrimido[5,4-*d*]pyrimidin-2-yl)ethynyl)pyrrolidin-1-yl)prop-2-en-1-one,
(84) (*S*)-1-(2-((8-((4-([1,2,4]triazolo[1,5-*a*]pyridin-7-yloxy)-3-methylphenyl)amino)pyrimido[5,4-*d*]pyrimidin-2-yl)ethynyl)pyrrolidin-1-yl)prop-2-en-1-one,
(85) (*R*)-1-(2-((4-((3-methyl-4-((1-methyl-1*H*-benzo[*d*]imidazol-5-yl)oxy)phenyl)amino)quinazolin-6-yl)ethynyl)pyrrolidin-1-yl)prop-2-en-1-one,
(86) 1-((2*R*)-2-((4-((3-methyl-4-((1-methyl-3*a*,7*a*-dihydro-1*H*-benzo[*d*]imidazol-5-yl)oxy)phenyl)amino)pyrido[3,2-*d*]pyrimidin-6-yl)ethynyl)pyrrolidin-1-yl)prop-2-en-1-one,
(87) (*S*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)pyrrolidin-1-yl)prop-2-en-1-one,
(88) (*R*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)piperidin-1-yl)prop-2-en-1-one,
(89) (*S*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)piperidin-1-yl)prop-2-en-1-one,
(90) 1-((2*R*)-2-((8-((3-methyl-4-((1-methyl-3*a*,7*a*-dihydro-1*H*-benzo[*d*]imidazol-5-yl)oxy)phenyl)amino )pyrimido[5,4-*d*]pyrimidin-2-yl)ethynyl)pyrrolidin-1-yl)prop-2-en-1-one,
(91) (*R*)-1-(2-((8-((3-methyl-4-((1-methyl-1*H*-benzo[*d*]imidazol-5-yl)oxy)phenyl)amino)pyrimido[5,4-*d*]pyrimidin-2-yl)ethynyl)piperidin-1-yl)prop-2-en-1-one and
(92) (*S*)-1-(2-((8-((3-methyl-4-((1-methyl-1*H*-benzo[*d*]imidazol-5-yl)oxy)phenyl)amino)pyrimido[5,4-*d*]pyrimidin-2-yl)ethynyl)piperidin-1-yl)prop-2-en-1-one.

The compounds of the present invention are capable of inhibiting the kinase activity of HER2. In one embodiment, the compounds of the present invention are capable of inhibiting the kinase activity of HER2 harboring a gene alteration in exon 20. In a specific embodiment, the compounds of the present invention are capable of inhibiting the kinase activity of HER2 harboring a mutation in exon 20. In a more specific embodiment, the compounds of the present invention are capable of inhibiting the kinase activity of HER2 harboring an insertion mutation in exon 20.

### Pharmaceutical Compositions

In another aspect, the present invention provides a pharmaceutical composition comprising at least one compound described above and pharmaceutically acceptable excipients. The pharmaceutical composition of the present invention may be used for inhibiting the activity of HER2 inside the body, for example for treating cancer. In particular, the pharmaceutical composition of the present invention can be used for treating cancer caused by a gene alteration or a mutation in exon 20 of HER2, *e.g*. an insertion mutation.

The compound of the present invention may be present in its pharmaceutically acceptable salt form in the pharmaceutical composition of the present invention. Pharmaceutically acceptable salts include, *e.g.*, acid addition salts and base addition salts. Pharmaceutically acceptable base addition salts may be in the form of metal salts and amine salts, for example, salts from alkali or alkaline earth metal bases or salts from organic amines. Pharmaceutically acceptable salts of a compound may be produced by means of pharmaceutically acceptable cations. Pharmaceutically acceptable acid addition salts include those from inorganic and organic acids.

As used herein, the term "pharmaceutically acceptable excipients" refers to inactive ingredients which are approved by a governmental regulatory authority (such as the Ministry of Food and Drug Safety of Korea and the US Food and Drug Administration (FDA)) as suitable for use in conjunction with the active pharmaceutical ingredient in the preparation of medicaments for treating diseases in humans or animals. Examples of pharmaceutically acceptable excipients include, but are not limited to, carriers, lubricants, glidants, disintegrants, sweetening agents, diluents, preservatives, colorings, flavorings, surfactants, wetting agents, dispersants, suspending agents, stabilizing agents, tonicity agents, solvents, emulsifiers and adjuvants.

The pharmaceutical composition of the present invention may, for example, be in a form suitable for oral administration such as a tablet, capsule, pill, powder, sustained release formulations, solution suspension, for parenteral injection such as a sterile solution, suspension or emulsion, for topical administration such as an ointment or cream or for rectal administration such as a suppository.

Pharmaceutical compositions containing the compounds disclosed herein can be manufactured in a conventional manner, e.g., by conventional mixing, dissolving, granulating, dragee-making, levigating, emulsifying, encapsulating, entrapping, or lyophilizing processes. Proper formulation is dependent upon the route of administration chosen.

For oral administration, suitable compositions can be formulated readily by combining a compound disclosed herein with pharmaceutically acceptable excipients such as carriers well known in the art. Such excipients and carriers enable the compounds of the present invention to be formulated as tablets, pills, dragees, capsules, liquids, gels, syrups, slurries, suspensions and the like, for oral ingestion by a patient to be treated. Pharmaceutical preparations for oral use can be obtained by adding a compound as disclosed herein with a solid excipient, optionally grinding a resulting mixture, and processing the mixture of granules, after adding suitable auxiliaries, if desired, to obtain tablets or dragee cores. Suitable excipients include, for example, fillers and cellulose preparations. If desired, disintegrating agents can be added.

Typically, the pharmaceutical composition for orally administering a therapeutically effective amount of a compound disclosed herein is in the form of a solid (e.g., tablet, capsule, pill, powder, or troche) or a liquid formulation (e.g., aqueous suspension, solution, elixir, or syrup).

When administered in tablet form, the pharmaceutical composition can additionally contain a functional solid and/or solid carrier, such as a gelatin or an adjuvant. Pharmaceutical compositions in the form of tablet, capsule or powder can contain about 1 to about 95 wt. % of the compound of the present invention, and preferably from about 15 to about 90 wt. % of the compound. As an example, a tablet composition may comprise, *e.g.*, about 80 wt. % or less of an active pharmaceutical ingredient, from about 10 wt. % to about 90 wt. % of binder, from about 0 wt. % to about 85 wt. % of diluent, from about 2 wt. % to about 10 wt. % of disintegrant and from about 0.25 wt. % to about 10 wt. % of lubricant.

When administered in liquid or suspension form, a functional liquid and/or a liquid carrier such as water, petroleum, or oils of animal or plant origin can be added. The liquid form of the pharmaceutical composition can further contain physiological saline solution, sugar alcohol solutions, dextrose or other saccharide solutions, or glycols. When administered in liquid or suspension form, the pharmaceutical composition can contain about 0.5 to about 90% by weight of a compound disclosed herein, and preferably about 1 to about 50% of a compound disclosed herein. In one embodiment contemplated, the liquid carrier is non-aqueous or substantially non-aqueous. For administration in liquid form, the composition may be supplied as a rapidly-dissolving solid formulation for dissolution or suspension immediately prior to administration.

When the pharmaceutical composition of the present invention is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, in addition to a compound disclosed herein, an isotonic vehicle. Such compositions may be prepared for administration as solutions of free base or pharmacologically acceptable salts in water suitably mixed with a surfactant, such as hydroxypropylcellulose. Dispersions also can be prepared in glycerol, liquid polyethylene glycols, and mixtures thereof and in oils. Under ordinary conditions of storage and use, these preparations can optionally contain a preservative to prevent the growth of microorganisms.

The pharmaceutical composition in the form of injectable formulations can include sterile aqueous solutions, suspensions, or dispersions and sterile powders for the extemporaneous preparation of sterile injectable solutions, suspensions, or dispersions. Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the embodiment of sterile powders for the preparation of sterile injectable solutions, the preferred methods of preparation are vacuum-drying and freeze drying techniques which yield a powder of the active ingredient plus any additional desired ingredient from a previously sterile-filtered solution thereof.

Slow release or sustained release formulations may also be prepared in order to achieve a controlled release of the active compound in contact with the body fluids in the gastrointestinal tract, and to provide a substantially constant and effective level of the active compound in the blood plasma. For example, release can be controlled by one or more of dissolution, diffusion, and ion-exchange. In addition, the slow release approach may enhance absorption via saturable or limiting pathways within the gastrointestinal tract. For example, the compound may be embedded for this purpose in a polymer matrix of a biologically degradable polymer, a water-soluble polymer or a mixture of both, and optionally suitable surfactants. Embedding can mean in this context the incorporation of micro-particles in a matrix of polymers. Controlled release formulations are also obtained through encapsulation of dispersed micro-particles or emulsified micro-droplets via known dispersion or emulsion coating technologies.

The pharmaceutical composition of the present invention can be formulated for parenteral administration by injection (*e*.*g*., by bolus injection or continuous infusion). Formulations for injection can be presented in unit dosage form (*e*.*g*., in ampules or in multidose containers), with an added preservative. The compositions can take such forms as suspensions, solutions, or emulsions in oily or aqueous vehicles, and can contain formulatory agents such as suspending, stabilizing, and/or dispersing agents.

The pharmaceutical composition of the present invention can be formulated as a unit dosage form suitable for accurately administering a single dose.

Pharmaceutical compositions comprising the compounds of the present invention can be used in accordance with the methods described below.

### Methods of Treating

In still another aspect, the present invention provides a method of treating cancer. This method comprises administering to a subject in need of treatment a therapeutically effective amount of a compound of the present invention, for example, a compound as defined by Chemical Formula 1. Alternatively, the present invention comprises administering to a subject a pharmaceutical composition comprising a therapeutically effective amount of a compound as described above and pharmaceutically acceptable excipients.

In the method for treating cancer, a compound of the present invention can be administered alone as a single agent or in combination with at least one other medicament. Such co-administration of a compound of the present invention with other medicament can be conducted simultaneously or sequentially.

As used herein, and unless otherwise specified, a "therapeutically effective amount" of a compound refers to an amount of that compound sufficient to delay or minimize one or more symptoms associated with a disease, disorder or condition or an amount of that compound sufficient to provide a therapeutic benefit to a disease, disorder or condition. The term "therapeutically effective amount" can encompass an amount that improves overall therapy, reduces or avoids symptoms or causes of disease or disorder, or enhances the therapeutic efficacy of another therapeutic agent.

The amount of compound administered can be dependent on the subject being treated, on the subject's age, health, sex, and weight, the kind of concurrent treatment (if any), severity of the affliction, the nature of the effect desired, the manner and frequency of treatment, and the judgment of the prescribing physician. The frequency of dosing also can be dependent on pharmacodynamic effects on arterial oxygen pressures. However, the most preferred dosage can be tailored to the individual subject, as is understood and determinable by one of skill in the art, without undue experimentation. This typically involves adjustment of a standard dose (*e*.*g*., reduction of the dose if the patient has a low body weight).

While individual needs vary, determination of optimal ranges of effective amounts of the compound is within the skill of the art. For administration of a compound of the present invention to a human in the curative or prophylactic treatment of the conditions and disorders identified herein, for example, typical dosages of the compounds of the present invention can be about 0.01 mg/kg/day to about 50 mg/kg/day. Such doses may be administered in a single dose or it may be divided into multiple doses.

In yet another aspect, the compounds of the present invention are provided for use as a medicament. In an embodiment, the compounds of the present invention are provided for use as a medicament for treating cancer.

In still yet another aspect, the compounds of the present invention are provided for use in the manufacture of a medicament. In an embodiment, the compounds of the present invention are provided for use in the manufacture of a medicament for the treatment of cancer.

In a further aspect yet, the present invention provides a method of inhibiting the kinase activity of HER2 in the cell. The method comprises contacting a cell whose HER2 kinase activity needs to be inhibited with an effective amount of a compound as described in the present invention, *e*.*g*., a compound as defined in Chemical Formula 1.

### Examples

The present invention will be described in further detail with reference to the following working and experimental examples. However, it is to be understood that these working and experimental examples are by no means intended to limit the scope of the invention thereto and are provided solely for the purpose of illustrating the present invention. Persons skilled in the art would readily understand that there are available many modifications and alternatives to these examples. All such modifications and alternatives are intended to be encompassed within the scope of the attached claims.

### Example 1: Synthesis of N-(2-((4-((4-([1,2,4]triazolo[1,5-a]pyridin-7-yloxy)-3-methylphenyl)amino)quinazolin-6-yl)oxy)ethyl)-N-methylacrylamide

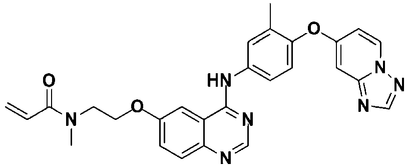

### Step (1): Preparation of 4-oxo-1H-quinazolin-6-yl acetate

3 g of 6-hydroxy-1*H*-quinazolin-4-one (18.5 mmol) and 3.4 mL of pyrinde (42.5 mmol) were diluted in 27.9 mL of acetic anhydride (296.0 mmol) and the mixture was stirred at 100°C for 2 hours. Upon completion of the reaction, the reaction mixture was slowly dropwise added to ice water and stirred. The resultant mixture was suction filtered and the solid thus obtained was rinsed with distilled water. The solid was dried in an oven at 50°C to obtain 4.4 g of the title compound (100% yield).

¹H-NMR (300 MHz, DMSO-*d*₆) δ 12.25 (s, 1H), 8.61 (s, 1H), 7.95 (d, 1H). 7.71 (s, 1H), 7.66 (d, 1H), 2.75 (s, 3H).

### Step (2): Preparation of 4-chloroquinazolin-6-yl acetate

2.2 g (10.7 mmol) of the compound prepared in step (1) was diluted in thionyl chloride (31 mL, 430.9 mmol), followed by addition of 0.3 mL *N,N*-dimethylformamide. This mixture was stirred at 90°C for 6 hours. The resultant mixture was suction filtered to obtain 2 g of the title compound (83% yield).

¹H-NMR (300 MHz, CDCl₃) δ 9.15 (s, 1H), 8.38 (d, 1H), 8.12 (s, 1H). 7.85 (d, 1H), 2.43 (s, 3H).

### Step (3): Preparation of [4-[3-methyl-4-([1,2,4]triazolo[1,5-a]pyridine-7-yloxy)anilino]quinazolin-6-yl] acetate

1 g (4.49 mmol) of the compound prepared in step (2) and 1.1 g (4.49 mmol) of 3-methyl-4-([1,2,4]triazolo[1,5-*a*]pyridine-7-yloxy)aniline were diluted in a solvent mixture of 12 mL of isopropyl alcohol and 12 mL of ethylene chloride and the solution was stirred at 80°C for 4 hours. This solution was diluted with ethyl acetate and was washed with distilled water and saturated brine. The organic layer separated was dried over anhydrous sodium sulfate and was suction filtered and vacuum distilled. The resultant residue was purified with column chromatography (dichloromethane:methanol = 15:1 (volume ratio)) to give 350 mg of the title compound (18% yield).

¹H-NMR (300 MHz, CDCl₃) δ 8.80 (s, 1H), 8.51 (d, 1H), 8.25 (s, 1H). 7.97 (d, 1H), 7.79 (s, 1H), 7.70 (s, 1H), 7.62-7.55 (m, 4 H), 7.11 (d, 1H), 6.92 (d, 1H), 6.85 (s, 1H), 2.40 (s, 3H), 2.24 (s, 3H).

### Step (4): Preparation of 4-[3-methyl-4-([1,2,4]triazolo[1,5-a]pyridin-7-yloxy)anilino]quinazolin-6-ol

350 mg (0.82 mmol) of the compound prepared in step (3) and 1.6 mL (0.82 mmol) of ammonium hydroxide were diluted in 6 mL of methanol and the mixture was stirred at 80°C for 4 hours. The resultant mixture was vacuum distilled and recrystallized in dichloromethane to obtain 305 mg of the title compound (96% yield).

¹H-NMR (300 MHz, DMSO-*d₆*) δ 10.11 (s, 1H), 9.55 (s, 1H), 8.93 (d, 1H), 8.47 (s, 1H), 8.38 (s, 1H), 7.92-7.81 (m, 3H), 7.68 (d, 1H), 7.43 (d, 1H), 7.19 (d, 1 H), 7.02 (d, 1H), 6.80 (s, 1H), 2.19 (s, 3H).

### Step (5): Preparation of 3-tert-butyl N-methyl-N-[2-[4-[3-methyl-4-([1,2,4]triazolo[1,5-a]pyridin-7-yloxy)anilino]quinazolin-6-yl]oxyethyl]carbamate

305 mg (0.79 mmol) of the compound prepared in step (4), 184 mg (0.95 mmol) of 3-*tert*-butyl *N*-(2-chloroethyl)-*N*-methyl-carbamate and 438 mg (3.17 mmol) of potassium carbonate were diluted in 2 mL of N, N-dimethylformamide and the solution was stirred at 100°C for 3 hours. Upon completion of the reaction, the reaction mixture was allowed to cool to room temperature, diluted with ethyl acetate and washed with distilled water and saturated brine. The organic layer separated was dried over anhydrous sodium sulfate and was suction filtered and vacuum distilled. The resultant residue was purified with column chromatography (dichloromethane:methanol = 10:1 (volume ratio)) to give 32 mg of the title compound (7% yield).

¹H-NMR (300 MHz, CDCl₃) δ 8.73 (s, 1H), 8.51 (d, 1H), 8.24 (s, 1H). 8.10 (d, 1H), 7.88-7.71 (m, 3H), 7.43-7.28 (m, 2H), 7.15-7.10 (m, 1H), 6.93-6.89 (m, 2 H), 4.36-4.33 (m, 2H), 3.75-3.70 (m, 2H), 3.04 (s, 3H), 2.26 (s, 3H), 1.59 (s, 9H).

### Step (6): Preparation of N-(2-((4-((4-([1,2,4]triazolo[1,5-a]pyridin-7-yloxy)-3-methylphenyl)amino)quinazolin-6-yl)oxy)ethyl)-N-methylacrylamide

32 mg (0.059 mmol) of the compound prepared in step (5) was diluted in a solution of 0.45 mL (5.9 mmol) of trifluoroacetic acid and 1 mL of dichloromethane and the solution was stirred at ambient temperature for 2 hours. Upon completion of the reaction, the reaction mixture was vacuum distilled. 6.4 µL (0.09 mmol) of acrylic acid and 27 µL (0.09 mmol) of propylphosphonic acid anhydride were diluted in 2 mL of N,N-dimethylformamide, followed by addition of 75 µL (0.42 mmol) of N,Ndiisopropylethylamine and stirring at ambient temperature for 30 minutes. This solution was transferred to the residue obtained from the vacuum distillation and the mixture was stirred at 0°C for 2 hours. Upon completion of the reaction, the reaction mixture was diluted with ethyl acetate and washed with distilled water and saturated brine. The organic layer separated was dried over anhydrous sodium sulfate and was suction filtered and vacuum distilled. The resultant residue was purified with column chromatography (dichloromethane:methanol = 10:1 (volume ratio)) to give 4 mg of the title compound (12% yield).
¹H-NMR (300 MHz, CDCl₃) δ 8.75 (s, 2H), 8.50 (d, 1H), 8.40 (s, 1H). 8.24 (s, 1H), 8.08-8.04 (m, 2H), 7.83 (d, 1H), 7.41 (d, 1H), 7.11 (d, 1H), 6.92-6.90 (m, 2 H), 6.69-6.66 (m, 1H), 6.53 (d, 1H), 5.84 (d, 1H), 4.44-4.39 (m, 2H), 3.95-3.93 (m, 2H), 3.28 (s, 3H), 2.25 (s, 3H);
MS (ESI+): m/z = 496.3 [M+H]⁺.

The compounds of Examples 2 to 10 shown in Table 1 below were prepared using the same or similar procedures as described in Example 1 above.

**[Table 1]**

| | Structural Formula | Analysis Data |
|---|---|---|
| **2** | 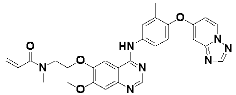 | ¹H-NMR (300 MHz, CD₃OD) δ 8.75 (d, 1H), 8.49 (brs, 1H), 8.31 (brs, 1H), 7.90-7.67 (m, 3H), 7.25-7.05 (m, 3H), 7.05-6.78 (m, 2H), 6.35-6.24 (m, 1H), 5.82-5.72 (m, 1H), 4.45-4.35 (m, 2H), 4.10-3.90 (m, 5H), 4.38 (s, 3H), 2.29 (s, 3H); |
| | | MS (ESI⁺): m/z = 526.6 [M+H]⁺. |
| **3** | 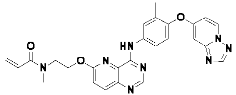 | ¹H-NMR (300 MHz, DMSO-*d*₆) δ 9.65 (d, 1H), 8.95 (d, 1H), 8.61 (d, 1H), 8.38 (s, 1H), 8.20-7.90 (m, 3H), 7.41-7.30 (m, 1H), 7.30-7.20 (m, 1H), 7.03 (dd, 1H), 6.90-6.70 (m, 2H), 6.25-6.05 (m, 1H), 5.75-5.55 (m, 1H), 4.90-4.70 (m, 2H), 4.00-3.80 (m, 2H), 3.10 (d, 3H), 2.21 (s, 3H); |
| | | MS (ESI⁺): *m*/*z* = 497.5 [M+H]⁺. |
| **4** | 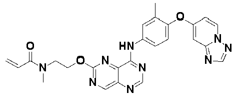 | ¹H-NMR (300 MHz, CDCl₃) δ 9.06 (s, 1H), 8.55 (s, 1H), 7.86 (m, 3H), 7.36-7.26 (m, 3H), 7.08 (d, 2H), 6.95 (d, 1H), 6.36 (d, 1H), 6.12-6.03 (m, 1H), 5.80 (d, 1H), 4.54-4.50 (m, 2H), 4.13-4.09 (m, 2H), 3.40 (s, 3H), 2.37 (s, 3H); MS (ESI⁺): m/z = 511.3 [M+H]⁺. |
| **5** | 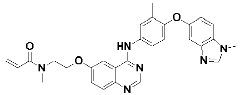 | ¹H-NMR (300 MHz, DMSO-*d*₆) δ 9.65 (s, 1H), 8.48-8.46 (m, 1H), 8.46 (s, 1H), 8.16 (s, 1H), 7.94 (d, 1H), 7.73-7.70 (m, 1H), 7.64 (d, 1H), 7.55 (d, 1H), 7.46 (d, 1H), 7.00 (d, 1H), 6.98 (d, 1H), 6.86 (d, 1H), 6.33-6.27 (m, 1H), 6.14 (d, 1H), 5.62 (d, 1H), 4.22 (t, 2H), 4.22 (s, 3H), 3.83-3.62 (m, 2H), 2.48 (s, 3H), 2.25 (s, 3H); |
| | | MS (ESI⁺): *m*/*z* = 509.6 [M+H]⁺. |
| **6** | 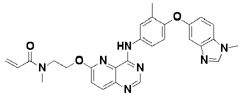 | ¹H-NMR (300 MHz, DMSO-*d*₆) δ 9.55 (d, 1H), 8.55 (d, 1H), 8.17 (s, 1H), 8.15-8.10 (m, 1H), 8.00-7.70 (m, 2H), 7.56 (d, 1H), 7.33 (dd, 1H), 7.10 (d, 1H), 7.08-7.00 (m, 1H), 6.95-6.90 (m, 1H), 6.90-6.70 (m, 1H), 6.20-6.00 (m, 1H), 5.70-5.55 (m, 1H), 5.90-5.70 (m, 2H), 3.95-3.80 (m, 5H), 3.10 (s, 3H), 2.26 (s, 3H); |
| | | MS (ESI⁺): m/z = 510.6 [M+H]⁺. |
| **7** | 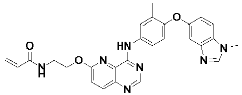 | ¹H-NMR (300 MHz, DMSO-*d*₆) δ 9.59 (s, 1H), 8.55 (s, 1H), 8.52-8.43 (m, 1H), 8.15 (s, 1H), 8.09 (d, 1H), 7.92-7.89 (m, 2H), 7.55 (d, 1H), 7.35 (d, 1H), 7.07 (d, 1H), 6.97 (d, 1H), 6.89 (d, 1H), 6.23 (d, 1H), 6.13 (d, 1H), 5.60 (d, 1H), 4.68-4.64 (m, 2H), 3.83 (s, 3H), 3.62-3.60 (m, 2H), 2.25 (s, 3H); |
| | | MS (ESI⁺): m/z = 496.5 [M+H]⁺. |
| **8** | 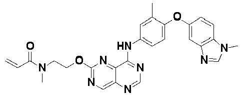 | ¹H-NMR (300 MHz, CDCl₃) δ 9.06 (s, 1H), 8.55 (s, 1H), 7.86 (s, 2H), 7.36-7.26 (m, 3H), 7.08 (d, 2H), 6.95 (d, 1H), 6.36 (d, 1H), 6.12-6.03 (m, 1H), 5.80 (d, 1H), 4.54-4.50 (m, 2H), 4.13-4.09 (m, 2H), 3.86 (s, 3H), 3.40 (s, 3H), 2.37 (s, 3H); |
| | | MS (ESI⁺): m/z = 511.3 [M+H]⁺. |
| **9** | 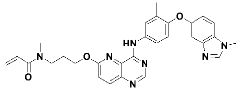 | ¹H-NMR (300 MHz, DMSO-*d*₆) δ 9.41 (s, 1H), 8.55 (s, 1H), 8.17 (s, 1H), 8.11 (dd, 1H), 7.90-7.72 (m, 2H), 7.56 (d, 1H), 7.40-7.30 (m, 1H), 7.10 (d, 1H), 7.00 (dd, 1H), 6.91 (d, 1H), 6.90-6.65 (m, 1H), 6.15-6.00 (m, 1H), 5.70-5.50 (m, 1H), 4.70-4.55 (m, 2H), 3.84 (s, 3H), 3.70-3.50 (m, 2H), 3.02 (s, 3H), 2.27 (s, 3H), 2.15-2.00 (m, 2H); |
| | | MS (ESI⁺): m/z = 524.6 [M+H]⁺. |
| **10** | 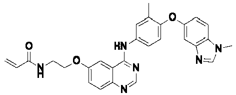 | ¹H-NMR (300 MHz, DMSO-*d*₆) δ 9.75 (s, 1H), 8.62 (s, 1H), 8.20 (s, 1H), 8.15 (d, 1H), 7.90-7.72 (m, 2H), 7.56 (d, 1H), 7.40-7.30 (m, 2H), 7.10 (d, 1H), 7.00 (dd, 1H), 6.91 (d, 1H), 6.90-6.65 (m, 1H), 6.15-6.00 (m, 1H), 5.70-5.50 (m, 1H), 4.70-4.55 (m, 2H), 3.84 (s, 3H), 3.70-3.50 (m, 2H), 2.27 (s, 3H); |
| | | MS (ESI⁺): m/z = 495.6 [M+H]⁺. |

### Example 11: Synthesis of N-(2-((4-((4-([1,2,4]triazolo[1,5-a]pyridin-7-yloxy)-3-methylphenyl)amino)pyrido[3,2-d]pyrimidin-6-yl)amino)ethyl)-N-methylacrylamide

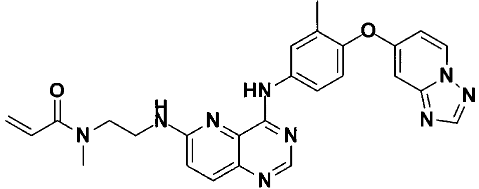

### Step (1): Preparation of 4,6-dichloropyrido[3,2-d]pyrimidine

1.2 g of 6-chloropyrido[3,2-d]pyrimidin-4-ol (6.50 mmol) was diluted in 12 mL of thionyl chloride (165.22 mmol), followed by addition of 0.1 mL N,N-dimethylformamide and stirring at 120°C for 12 hours. Upon completion of the reaction, the resultant mixture was allowed to cool to room temperature and vacuum distilled to obtain 1.4 g of the title compound (100% yield).

¹H-NMR (300 MHz, CDCl₃) δ 9.16 (s, 1H), 8.35 (d, 1H), 7.89 (d, 1H).

### Step (2): Preparation of N-(4-([1,2,4]triazolo[1,5-a]pyridin-7-yloxy)-3-methylphenyl)-6-chloropyrido[3,2-d]pyrimidin-4-amine

1.4 g (0.7 mmol) of the compound prepared in step (1) and 1.5 g (6.3 mmol) of 4-([1,2,4] triazolo[1,5-*a*]pyridin-7-yloxy)-3-methylaniline were diluted in a solvent mixture of 15 mL of isopropanol and 15 mL of 1,2-dichloroethane. To this was added 1 mL of trifluoroacetic acid and the solution was stirred at 80°C for 5 hours. Upon completion of the reaction, the resultant mixture was allowed to cool to room temperature and vacuum distilled. This resultant residue was diluted with ethyl acetate and was washed with saturated aqueous sodium bicarbonate. The organic layer separated was dried over anhydrous sodium sulfate followed by suction filtration and vacuum distillation. The resultant residue was purified with column chromatography (hexane : ethyl acetate = 2:1 (volume ratio)) to give 1.5 g of the title compound (59% yield).

¹H-NMR (300 MHz, DMSO-*d*₆) δ 10.16 (s, 1H), 8.94 (d, 1H), 8.71 (s, 1H), 8.39 (s, 1H), 8.28 (d, 1H), 8.20-8.10 (m, 1H), 8.07-7.95 (m, 2H), 7.22 (d, 1H), 7.03 (dd, 1H), 6.81 (d, 1H), 2.21 (s, 3H).

### Step (3): Preparation of 3-tert-butyl (2-((4-((4-([1,2,4]triazolo[1,5-a]pyridin-7-yloxy)-3-methylphenyl)amino)pyrido[3,2-d]pyrimidin-6-yl)amino)ethyl)(methyl)carbamate

200 mg (0.5 mmol) of the compound prepared in step (2) and 863 mg (4.95 mmol) of 3-*tert*-butyl (2-aminoethyl)(methyl)carbamate were diluted in 1-butanol and the solution was stirred at 110°C for 12 hours. Upon completion of the reaction, the resultant mixture was allowed to cool to room temperature and vacuum distilled, followed by dilution in ethyl acetate and washing with water. The organic layer separated was dried over anhydrous sodium sulfate and was suction filtered and vacuum distilled. The resultant residue was purified with column chromatography (dichloromethane:methanol = 10:1 (volume ratio)) to give 278 mg of the title compound (100% yield).

¹H-NMR (300 MHz, DMSO-*d*₆) δ 9.30 (d, 1H), 8.93 (d, 1H), 8.39 (d, 2H), 8.12-8.00 (m, 2H), 7.78 (d, 1H), 7.70-7.60 (m, 1H), 7.30-7.20 (m, 1H), 7.09 (d, 1H), 7.03 (dd, 1H), 6.80-6.70 (m, 1H), 3.80-3.50 (m, 4H), 3.00-2.70 (m, 3H), 2.20 (s, 3H), 1.39 (s, 9H).

### Step (4): Preparation of N-(4-([1,2,4]triazolo[1,5-a]pyridin-7-yloxy)-3-methylphenyl)-N⁶-(2-(methylamino)ethyl)pyrido[3,2-d]pyrimidin-4-diamine

278 mg (0.51 mmol) of the compound prepared in step (3) was diluted in 3 mL of dichloromethane and 1.5 mL (19.6 mmol) of trifluoroacetic acid was added. This mixture was stirred at ambient temperature for 2 hours. Upon completion of the reaction, the resultant mixture was vacuum distilled and 2 N aqueous sodium hydroxide was slowly added dropwise followed by dilution and washing with a solvent mixture of chloroform:2-propanol (3:1 (volume ratio)). The organic layer thus separated was dried over anhydrous sodium sulfate and was suction filtered and vacuum distilled. The resultant residue was purified with column chromatography (dichloromethane:methanol = 1:99 (volume ratio)) to give 143 mg of the title compound (63% yield)

¹H-NMR (300 MHz, DMSO-*d*₆) δ 9.20 (bs, 1H), 8.93 (d, 1H), 8.42-8.37 (m, 2H), 8.02-7.96 (m, 2H), 7.76 (d, 1H), 7.51-7.40 (m, 1H), 7.21 (d, 1H), 7.14 (d, 1H), 7.03 (dd, 1H), 6.79 (dd, 1H), 4.10 (bs, 1H), 3.64 (dd, 2H), 3.18 (s, 3H), 2.76 (t, 2H), 2.20 (s, 3H).

### Step (5): Preparation of N-(2-((4-((4-([1,2,4]triazolo[1,5-a]pyridin-7-yloxy)-3-methylphenyl)amino)pyrido[3,2-d]pyrimidin-6-yl)amino)ethyl)-N-methylacrylamide

0.03 mL (0.47 mmol) of acrylic acid and 0.17 mL (0.95 mmol) of *N,N-*diisopropylethylamine were diluted in 0.5 mL of *N,N*-dimethylformamide, followed by addition of 0.14 mL (0.47 mmol) of propylphosphonic anhydride solution and stirring at ambient temperature for 30 minutes. This solution was added dropwise at 0°C to 1 mL of an *N,N*-dimethylformamide solution containing 140 mg (0.31 mmol) of the compound prepared in step (4) and 0.08 mL (0.47 mmol) of *N,N*-diisopropylethylamine and the resultant solution was stirred for 2 hours. Upon completion of the reaction, the reaction mixture was diluted with ethyl acetate and washed with water. The organic layer separated was dried over anhydrous sodium sulfate and was suction filtered and vacuum distilled. The resultant residue was purified with column chromatography (dichloromethane:methanol = 10:1 (volume ratio)) to give 15.0 mg of the title compound (10% yield).

¹H-NMR (300 MHz, DMSO-*d₆*) δ 9.45 (d, 1H), 8.93 (d, 1H), 8.43 (d, 1H), 8.37 (s, 1H), 8.30-7.90 (m, 3H), 7.82-7.73 (m, 1H), 7.70-7.60 (m, 1H), 7.30-7.20 (m, 1H), 7.15-7.10 (m, 1H), 7.10-7.00 (m, 1H), 6.80 (s, 1H), 6.27-5.98 (m, 1H), 5.75-5.40 (m, 1H), 3.70-3.60 (m, 4H), 3.20 (s, 3H), 2.20 (s, 3H);

MS (ESI+): m/z = 517.2 [M+H]⁺.

The compounds of Examples 12 to 21 shown in Table 2 below were prepared using the same or similar procedures as described in Example 11 above.

**[Table 2]**

| | Structural Formula | Analysis Data |
|---|---|---|
| **12** | 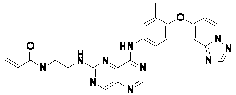 | ¹H-NMR (300 MHz, CDCl₃) δ 9.59 (s, 1H), 9.04 (s, 1H), 8.62 (s, 1H), 8.52 (d, 1H). 8.24-8.17 (m, 3H), 7.14 (d, 1H), 6.92-6.89 (m, 2H), 6.64-6.57 (m, 1H), 6.43-6.37 (m, 1H), 5.89 (s, 1H), 5.15-5.17 (m, 1H), 3.85-3.79 (m, 4H), 3.21 (s, 3H), 2.27 (s, 3H); |
| | | MS (ESI⁺): m/z = 497.4 [M+H]⁺. |
| **13** | 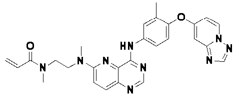 | ¹H-NMR (300 MHz, DMSO-*d*₆) δ 9.41 (d, 1H), 8.93 (d, 1H), 8.44 (s, 1H), 8.38 (s, 1H), 8.20-8.00 (m, 2H), 7.92 (d, 1H), 7.40-7.32 (m, 1H), 7.30-7.20 (m, 1H), 7.10-7.00 (m, 1H), 6.79 (d, 1H), 6.75-6.50 (m, 1H), 6.20-5.90 (m, 1H), 5.70-5.40 (m, 1H), 4.10-3.90 (m, 2H), 3.78-3.60 (m, 2H), 3.19 (s, 3H), 3.00 (s, 3H), 2.20 (s, 3H); |
| | | MS (ESI⁺): m/z = 510.6 [M+H]⁺. |
| **14** | 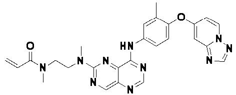 | ¹H-NMR (300 MHz, DMSO-*d*₆) δ 9.79 (s, 1H), 9.11 (s, 1H), 8.95 (d, 1H), 8.47 (s, 1H), 8.38 (s, 1H), 8.30-8.21 (m, 1H), 7.98 (s, 1H), 7.26 (d, 1H), 7.04 (d, 1H), 6.80 (s, 1H), 6.73-6.71 (m, 1H), 6.17-6.00 (m, 1H), 5.66-5.63 (m, 1H), 3.94-3.92 (m, 2H), 3.73-3.70 (m, 2H), 3.30 (s, 3H). 2.50 (s, 3H), 2.20 (s, 3H); |
| | | MS (ESI⁺): m/z = 511.3 [M+H]⁺. |
| **15** | 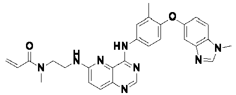 | ¹H-NMR (300 MHz, DMSO-*d*₆) δ 9.31 (d, 1H), 8.38 (d, 1H), 8.16 (s, 1H), 8.10-7.75 (m, 3H), 7.70-7.50 (m, 2H), 7.10-7.03 (m, 2H), 7.03-6.95 (m, 1H), 6.95-6.90 (m, 1H), 6.80-6.60 (m, 1H), 6.25-5.95 (m, 1H), 5.70-5.40 (m, 1H), 3.87-3.70 (m, 4H), 3.66 (s, 3H), 3.16 (s, 3H), 2.23 (s, 3H); |
| | | MS (ESI⁺): m/z = 509.6 [M+H]⁺. |
| **16** | 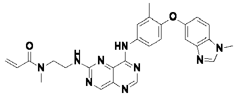 | ¹H-NMR (300 MHz, CDCl₃) δ 9.59 (s, 1H), 9.04 (s, 1H), 8.62 (s, 1H), 8.52 (d, 1H). 8.24-8.17 (m, 3H), 7.14 (d, 1H), 6.92-6.89 (m, 2H), 6.64-6.57 (m, 1H), 6.43-6.37 (m, 1H), 5.89 (s, 1H), 5.15-5.17 (m, 1H), 3.85-3.79 (m, 4H), 3.21 (s, 3H), 2.27 (s, 3H); |
| | | MS (ESI⁺): m/z = 497.4 [M+H]⁺. |
| **17** | 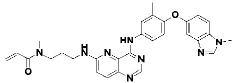 | ¹H-NMR (300 MHz, CDCl₃) δ 8.69 (s, 1H), 8.56 (s, 1H), 7.85 (s, 1H), 7.82-7.76 (m, 2H), 7.68 (d, 1H), 7.34-7.32 (m, 2H), 7.09 (d, 1H), 6.98-6.94 (m, 2H), 6.70-6.61 (m, 1H), 6.43 (d, 1H), 6.19 (t, 1H), 5.80 (d, 1H), 3.86 (s, 3H), 3.64-3.62 (m, 2H), 3.57-3.55 (m, 2H), 3.13 (s, 3H), 2.35 (s, 3H), 1.98-1.94 (m, 2H); |
| | | MS (ESI⁺): m/z = 523.2 [M+H]⁺. |
| **18** | 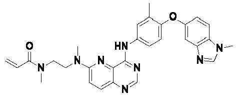 | ¹H-NMR (300 MHz, DMSO-*d*₆) δ 9.49 (s, 1H), 8.38 (s, 1H), 8.15 (s, 1H), 8.05 (s, 1H), 7.86 (d, 1H), 7.54 (d, 1H), 7.34 (d, 1H), 7.06 (d, 1H), 6.98 (d, 1H), 6.89 (d, 1H), 6.73-6.64 (m, 1H), 6.14 (d, 1H), 5.63 (d, 1H), 3.96-3.83 (m, 2H), 3.83 (s, 3H), 3.69-3.64 (m, 2H), 3.22 (s, 3H), 3.11 (s, 3H), 2.24 (s, 3H); |
| | | MS (ESI⁺): m/z = 523.6 [M+H]⁺. |
| **19** | 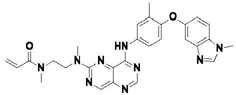 | ¹H-NMR (300 MHz, CDCl₃) δ 9.54 (s, 1H), 9.05 (s, 1H), 8.54 (s, 1H), 8.07 (s, 1H), 7.86 (s, 1H), 7.74-7.63 (m, 1H), 7.34-7.32 (m, 2H), 7.08 (d, 1H), 6.97 (d, 1H), 6.55-6.46 (m, 1H), 6.32 (d, 1H), 5.66 (d, 1H), 4.02-3.99 (m, 2H), 3.86 (s, 3H), 3.81-3.79 (m, 2H), 3.38 (s, 3H), 3.15 (s, 3H), 2.35 (s, 3H); |
| | | MS (ESI⁺): m/z = 524.2 [M+H]⁺. |
| **20** | 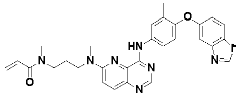 | ¹H-NMR (300 MHz, CDCl₃) δ 8.71 (s, 1H), 8.57 (s, 1H), 7.94 (d, 1H), 7.86 (s, 1H), 7.80 (s, 1H), 7.70-7.65 (m, 1H), 7.35-7.32 (m, 2H), 7.15 (d, 1H), 7.09 (d, 1H), 6.97 (d, 1H), 6.60-6.55 (m, 1H), 6.37 (d, 1H), 5.73-5.69 (m, 1H), 3.86 (s, 3H). 3.78 (t, 2H), 3.61-3.56 (m, 2H), 3.25 (s, 3H), 3.11 (s, 3H), 2.36 (s, 3H), 2.07-2.00 (m, 2H); |
| | | MS (ESI⁺): m/z = 537.4 [M+H]⁺. |
| **21** | 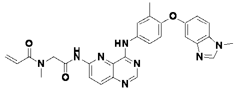 | ¹H-NMR (300 MHz, DMSO-*d*₆) δ 11.08 (s, 1H), 9.30 (s, 1H), 8.62 (s, 1H), 8.52 (d, 1H), 8.25 (d, 1H), 8.17 (s, 1H), 7.92 (s, 1H), 7.76 (d, 1H), 7.59 (d, 1H), 7.12 (s, 1H), 7.03 (d, 1H), 6.92 (d, 2H), 6.19-6.10 (m, 1H), 5.77-5.64 (m, 1H), 4.36 (s, 2H), 3.84 (s, 3H), 3.19 (s, 2H), 2.28 (s, 3H); |
| | | MS (ESI⁺): m/z =523.2 [M+H]⁺. |

### Example 22: Synthesis of (E)-N-.methyl-N-(3-((4-((3-methyl-4-((1-methyl-1H-benzo[d]imidazol-5-yl)oxy)phenyl)amino)quinazolin-6-yl)aryl)acrylamide

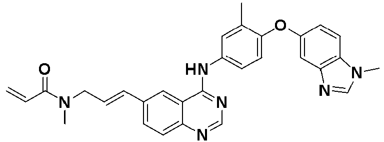

### Step (1): Preparation of 1-methyl-5-(2-methyl-4-nitro-phenoxy)benzimidazole

1.05 g (7.0 mmol) of 1-methylbenzimidazol-5-ol, 1.4 g (9.2 mmol) of 1-fluoro-2-methyl-4-nitrobezene and 2.4 g (17.7 mmol) of potassium carbonate were diluted in 30 mL of *N,N*-dimethylformamide and the solution was stirred at 80°C for 12 hours. Upon completion of the reaction, the reaction mixture was allowed to cool to room temperature, and distilled water was added dropwise and the mixture was slowly stirred. The solid obtained was washed with distilled water, ethyl acetate and hexane and followed by suction filtration to give 1.8 mg of the title compound (89% yield).

¹H-NMR (300 MHz, DMSO-*d₆*) δ 8.25 (s, 1H), 8.23 (s, 1H), 8.00 (d, 1H), 7.67 (d, 1H), 7.42 (s, 1H), 7.09 (d, 1H), 6.68 (d, 1H), 3.87 (s, 3H), 2.42 (s, 3H).

### Step (2): Preparation of 3-methyl-4-(1-methylbenzimidazol-5-yl)oxy-aniline

1.8 g (6.3 mmol) of the compound prepared in step (1) was diluted in a solvent mixture of 40 mL of ethyl acetate and 40 mL of methanol. To this was added 300 mg of palladium/carbon (15 wt%) and the mixture was allowed to react for 16 hours at ambient temperature under a hydrogen atmosphere. Upon completion of the reaction, the reaction mixture was transferred to a Celite^{®}-filled filter and washed with methanol and ethyl acetate and was subject to suction filtration. The filtrate was vacuum distilled to give 1.57 g of the title compound (97% yield).

¹H-NMR (300 MHz, DMSO-*d₆*) δ 8.09 (s, 1H), 7.45 (d, 1H), 6.87-6.85 (m, 2H), 6.64 (d, 1H), 6.50 (s, 1H), 6.41 (d, 1H), 4.87 (s, 2H), 3.79 (s, 3H), 1.99 (s, 3H).

### Step (3): Preparation of 6-iodo-N-[3-methyl-4-(1-methylbenzimidazol-5-yl)oxyphenyl]quinazolin-4-amine

1.3 g (5.5 mmol) of the compound prepared in step (2) and 1.6 g (5.5 mmol) of 4-chloro-6-iodo-quinazoline were diluted in a solvent mixture of 16 mL of isopropyl alcohol and 16 mL of ethylene chloride, followed by addition of 0.8 mL (11.0 mmol) trifluoroacetic acid and the solution was stirred at 80°C for 3 hours. Upon completion of the reaction, the reaction mixture was diluted with ethyl acetate and was washed with distilled water and saturated brine. The organic layer separated was dried over anhydrous sodium sulfate and was suction filtered and vacuum distilled. The resultant residue was purified with column chromatography (dichloromethane:methanol = 15:1 (volume ratio)) to give 1.3 g of the title compound (46% yield).

¹H-NMR (300 MHz, DMSO-*d₆*) δ 8.77 (s, 1H), 8.35 (s, 1H), 8.04 (d, 1H), 7.89 (s, 1H), 7.73 (s, 1H), 7.65 (d, 1H), 7.61 (s, 1H), 7.44 (d, 1H), 7.36 (d, 1H), 7.31 (s, 1H), 7.10 (d, 1H), 6.88 (d, 1H), 3.88 (s, 3H), 2.34 (s, 3H).

### Step (4): Preparation of 3-tert-butyl N-methyl-N-[(E)-3-[4-[3-methyl-4-(1-methylbenzimidazol-5-yl)oxy-anilino]quinazolin-6-yl]allyl]carbamate

1.3 g (2.5 mmol) of the compound prepared in step (3), 1.1 g (3.8 mmol) of 3-*tert-*butyl *N*-methyl-*N*-[(*E*)-3-(4,4,5,5-tetramethyl-1,3,2-dioxaborolan-2-yl)allyl]carbamate, 296 mg (0.2 mmol) of tetrakis(triphenylphosphine)palladium(0) and 579 mg (5.6 mmol) of sodium carbonate were diluted in a solvent mixture of 6 mL of 1,4-dioxane and 2 mL of distilled water, and the mixture was stirred at 100°C for 16 hours. Upon completion of the reaction, the reaction mixture was allowed to cool to room temperature and diluted with dichloromethane, followed by washing with distilled water and saturated brine. The organic layer thus separated was dried over anhydrous sodium sulfate and was suction filtered and vacuum distilled. The resultant residue was purified with column chromatography (dichloromethane:methanol = 15:1 (volume ratio)) to give 720 mg of the title compound (51% yield).

¹H-NMR (300 MHz, DMSO-*d₆*) δ 8.25 (s, 1H), 8.23 (s, 1H), 8.00 (d, 1H), 7.67 (d, 1H), 7.42 (s, 1H), 7.09 (d, 1H), 6.68 (d, 1H), 3.87 (s, 3H), 2.42 (s, 3H).

### Step (5): Preparation of N-methyl-N-(1-methyl-4-nitro-1H-pyrazol-3-yl)methanesulfonamide

130 mg (0.59 mmol) of the compound prepared in step (4) was diluted in 5 mL of *N,N*-dimethylformamide and 28 mg (0.71 mmol) of sodium hydride was slowly added at 0°C. The mixture was stirred at ambient temperature for 30 minutes. To this solution, 55 µL (0.89 mmol) of methyl iodide was slowly added at 0°C and the mixture was stirred at ambient temperature for 12 hours. Upon completion of the reaction, the reaction mixture was diluted with ethyl acetate and washed with distilled water and saturated brine. The organic layer separated was dried over anhydrous sodium sulfate and was suction filtered and vacuum distilled. The resultant residue was purified with column chromatography (dichloromethane:methanol = 15:1 (volume ratio)) to give 88 mg of the title compound (64% yield).

¹H-NMR (300 MHz, CDCl₃) δ 8.74 (s, 1H), 8.35 (s, 1H), 8.04 (d, 1H), 7.88 (s. 1H), 7.64 (d, 1H), 7.46 (m, 1H), 7.37-7.31 (m, 2H), 7.11-7.08 (d, 1H), 6.92-6.87 (m, 1H), 6.63 (d, 1H), 6.34-6.29 (m, 1H), 4.06 (d, 1H), 3.87 (s, 3H), 2.92 (s, 3H), 2.35 (s, 3H), 1.51 (s, 9H).

### Step (6): Preparation of (E)-N-methyl-N-(3-((4-((3-methyl-4-((1-methyl-1H-benzo[d]imidazol-5-yl)oxy)phenyl)amino)quinazolin-6-yl)aryl)acrylamide

6.4 µL (0.09 mmol) of acrylic acid and 27 µL (0.09 mmol) of propylphosphonic acid anhydride were diluted in 2 mL of *N,N*-dimethylformamide, followed by addition of 75 µL (0.42 mmol) of N, N-diisopropylethylamine and stirring at ambient temperature for 30 minutes. To this solution was added 75 mg (0.16 mmol) of the compound prepared in step (5) and the mixture was stirred at 0°C for 2 hours. Upon completion of the reaction, the reaction mixture was diluted with ethyl acetate and washed with distilled water and saturated brine. The organic layer thus separated was dried over anhydrous sodium sulfate and was suction filtered and vacuum distilled. The resultant residue was purified with column chromatography (dichloromethane:methanol = 10:1 (volume ratio)) to give 19 mg of the title compound (21% yield).
¹H-NMR (300 MHz, CDCl₃) δ 8.75 (s, 1H), 7.88-7.84 (m, 4H), 7.64 (s. 1H), 7.56-7.49 (m, 2H), 7.37-7.34 (m, 2H), 7.10 (d, 1H), 6.92 (d, 1H), 6.72-6.61 (m, 2H), 6.45-6.39 (m, 2H), 5.80-5.72 (m, 1H), 4.30-4.25 (m, 2H), 3.87 (s, 3H), 3.14 (s, 3H), 2.36 (s, 3H);
MS (ESI+): m/z = 505.3 [M+H]⁺.

The compounds of Examples 23 to 64 shown in Table 3 below were prepared using the same or similar procedures as described in Example 22 above.

**[Table 3]**

| | Structural Formula | Analysis Data |
|---|---|---|
| **23** | 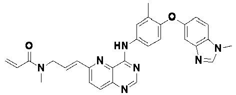 | ¹H-NMR (300 MHz, CDCl₃) δ 9.07 (s, 1H), 8.73 (s, 1H), 8.11 (t, 1H), 7.87-7.78 (m, 4H), 7.37-7.34 (m, 2H), 7.10 (d, 1H) 6.97 (d, 1H), 6.81 (s, 2H), 6.74-6.56 (m, 1H), 6.47 (d, 1H), 5.82 (t, 1H), 4.39 (d, 2H), 3.87 (s, 3H), 3.18 (s, 3H), 2.38 (s, 3H); |
| | | MS (ESI⁺): m/z = 506.3 [M+H]⁺. |
| **24** | 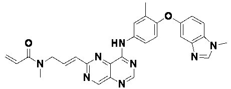 | ¹H-NMR (300 MHz, DMSO-*d*₆) δ 10.24 (d, 1H), 9.38 (d, 1H), 8.68 (s, 1H), 8.18 (s, 1H), 7.90 (d, 1H), 7.77 (d, 1H), 7.57 (d, 1H), 7.50-7.20 (m, 1H), 7.12 (d, 1H), 7.01 (dd, 1H), 7.00-6.60 (m, 3H), 6.20 (dd, 1H), 5.74 (dd, 1H), 4.44 (d, 1H), 4.33 (d, 1H), 3.85 (s, 3H), 3.10 (d, 3H), 2.27 (s, 3H); |
| | | MS (ESI⁺): m/z = 507.6 [M+H]⁺. |
| **25** | 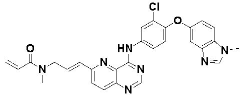 | ¹H-NMR (300 MHz, DMSO-*d*₆) δ 10.13 (s, 1H), 8.65 (s, 1H), 8.37 (d, 1H), 8.19 (d, 1H), 8.18-8.03 (m, 2H), 8.00-7.90 (m, 1H), 7.61 (d, 1H), 7.30-7.10 (m, 2H), 7.15-7.00 (m, 2H), 7.00-6.71 (m, 2H), 6.20 (dd, 1H), 5.75 (dd, 1H), 4.39 (d, 1H), 4.31 (d, 1H), 4.30 (s, 3H), 3.10 (d, 3H); |
| | | MS (ESI⁺): m/z = 527.0 [M+H]⁺. |
| **26** | 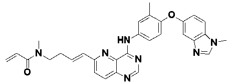 | ¹H-NMR (300 MHz, DMSO-*d*₆) δ 9.83 (d, 1H), 8.59 (s, 1H), 8.18 (s, 1H), 8.17-8.10 (m, 1H), 8.03-7.90 (m, 2H), 7.90-7.80 (m, 1H), 7.58 (d, 1H), 7.23-7.07 (m, 2H), 7.01 (dd, 1H), 6.91 (d, 1H), 6.90-6.60 (m, 2H), 6.12 (ddd, 1H), 5.65 (dt, 1H), 3.85 (s, 3H), 3.60 (dt, 2H), 3.06 (d, 3H), 2.70-2.52 (m, 2H), 2.27 (s, 3H); |
| | | MS (ESI⁺): m/z = 520.6 [M+H]⁺. |
| **27** | 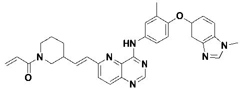 | ¹H-NMR (300 MHz, DMSO-*d*₆) δ 9.89 (d,1H), 8.60 (s, 1H), 8.19 (s, 1H), 8.14 (d, 1H), 8.01 (d, 1H), 7.93 (d, 1H), 7.83 (dd, 1H), 7.62-7.53 (m, 2H), 7.20 (d, 1H), 7.12 (d, 1H), 7.02 (dd, 1H), 6.92 (d, 1H), 6.84 (d, 1H), 6.13 (d, 1H), 5.70 (dd, 1H), 4.55-4.52 (m, 1H), 4.42-4.39 (m, 1H), 4.13-4.02 (m, 1H), 3.86 (s, 3H), 3.18-3.05 (m, 1H), 2.86-2.64 (m, 1H), 2.29 (s, 3H), 2.10-1.88 (m, 1H), 1.86-1.70 (m, 1H), 1.58-1.49 (m, 1H); |
| | | MS (ESI⁺): m/z = 546.6 [M+H]⁺. |
| **28** | 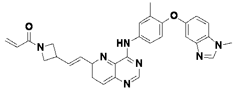 | ¹H-NMR (300 MHz, DMSO-*d*₆) δ 9.90 (s, 1H), 8.59 (s, 1H), 8.20-8.13 (m, 2H), 7.99 (d, 1H), 7,90 (d, 1H), 7.80 (dd, 1H), 7.60-7.45 (m, 2H), 7.11 (d, 1H), 7.00 (dd, 1H), 6.95-6.80 (m, 2H), 6.36 (dd, 1H), 6.13 (dd, 1H), 5.70 (dd, 1H), 4.54 (t, 1H), 4.30-4.20 (m, 2H), 4.00-3.90 (m, 1H), 3.84 (s, 3H), 3.72-3.60 (m, 1H), 2.27 (s, 3H); |
| | | MS (ESI⁺): m/z = 518.6 [M+H]⁺. |
| **29** | 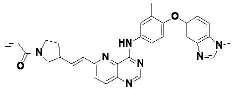 | ¹H-NMR (300 MHz, DMSO-*d*₆) δ 9.87 (s, 1H), 8.60 (s, 1H), 8.25-8.10 (m, 2H), 8.10-7.98 (m, 1H), 7.91 (s, 1H), 7.83 (d, 1H), 7.58 (d, 1H), 7.25 (dd, 1H), 7.11 (d, 1H), 7.00 (dd, 1H), 6.95-6.80 (m, 2H), 6.70-6.55 (m, 1H), 6.17 (dd, 1H), 5.70 (dd, 1H), 3.85 (s, 3H), 3.80-3.70 (m, 1H), 3.60-3.50 (m, 1H), 3.50-3.40 (m, 2H), 3.30-3.10 (m, 1H), 2.27 (s, 3H), 2.25-2.10 (m, 1H), 2.00-1.80 (m, 1H); |
| | | MS (ESI⁺): m/z = 532.6 [M+H]⁺. |
| **30** | 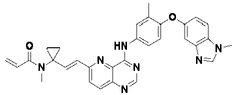 | ¹H-NMR (300 MHz, DMSO-*d*₆) δ 9.92 (d, 1H), 8.57 (d, 1H), 8.18 (d, 1H), 8.11-8.06 (m, 1H), 8.03 (d, 1H), 7.94-7.82 (m, 1H), 7.76 (d, 1H), 7.57 (d, 1H), 7.30 (d, 1H), 7.08 (dd, 1H), 7.00 (dd, 1H), 6.94-6.83 (m, 1H), 6.83-6.70 (m, 1H), 6.55 (dd, 1H), 6.20 (dd, 1H), 5.79-5.65 (m, 1H), 3.84 (s, 3H), 2.95 (s, 3H), 2.23 (s, 3H), 1.46-1.39 (m, 2H), 1.34-1.24 (m, 2H); |
| | | MS (ESI⁺): m/z = 532.6 [M+H]⁺. |
| **31** | 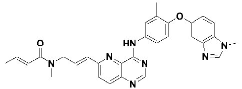 | ¹H-NMR (300 MHz, DMSO-*d*₆) δ 9.94 (d, 1H), 8.58 (s, 1H), 8.17 (s, 1H), 8.16-8.00 (m, 2H), 7.89 (s, 1H), 7.79 (dd, 1H), 7.57 (d, 1H), 7.32-7.12 (m, 1H), 7.10 (d, 1H), 7.00 (dd, 1H), 6.89 (d, 1H), 6.82-6.70 (m, 2H), 6.60-6.40 (m, 1H), 4.36 (d, 1H), 4.28 (d, 1H), 3.84 (s, 3H), 3.06 (d, 3H), 2.27 (s, 3H), 1.85 (q, 3H); |
| | | MS (ESI⁺): m/z = 520.6 [M+H]⁺. |
| **32** | 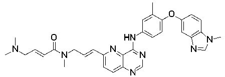 | ¹H-NMR (300 MHz, DMSO-*d*₆) δ 9.95 (s, 1H), 8.59 (s, 1H), 8.20-8.12 (m, 2H), 8.10-8.00 (m, 1H), 7.89 (d, 1H), 7.79 (dd, 1H), 7.57 (d, 1H), 7.11 (d, 1H), 7.00 (dd, 1H), 6.89 (d, 1H), 6.85-6.70 (m, 1H), 6.70-6.50 (m, 2H), 4.37 (d, 1H), 4.29 (d, 1H), 3.85 (s, 3H), 3.17-2.97 (m, 6H), 2.27 (s, 3H), 2.17 (s, 3H), 2.11 (s, 3H); |
| | | MS (ESI⁺): m/z = 563.7 [M+H]⁺. |
| **33** | 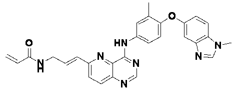 | ¹H-NMR (300 MHz, CDCl₃) δ 9.06 (s, 1H), 8.72 (s, 1H), 8.09 (d, 1H), 7.86-7.68 (m, 4H), 7.36-7.34 (m, 2H), 7.08 (d, 1H), 6.96-6.84 (m, 3 H), 6.43-6.37 (m, 1H), 6.25-6.16 (m, 1H), 5.93 (s, 1H), 5.75 (d, 1H), 4.32-4.28 (m, 2H), 3.87 (s, 3H), 2.37 (s, 3H); |
| | | MS (ESI⁺): m/z = 492.3 [M+H]⁺. |
| **34** | 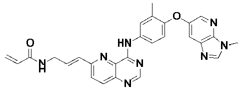 | ¹H-NMR (300 MHz, DMSO-*d*₆) δ 9.95 (s, 1H), 8.59 (s, 1H), 8.60-8.46 (m, 1H), 8.44 (s, 1H), 8.25 (d, 1H), 8.12 (d, 1H), 8.03 (d, 1H), 7.94 (d, 1H), 7.82 (dd, 1H), 7.62 (d, 1H), 7.30-7.15 (m, 1H), 6.90 (d, 1H), 6.82 (d, 1H), 6.33 (dd, 1H), 6.15 (dd, 1H), 5.65 (dd, 1H), 4.11 (t, 2H), 3.86 (s, 3H), 2.31 (s, 3H); |
| | | MS (ESI⁺): m/z =493.5 [M+H]⁺. |
| **35** | 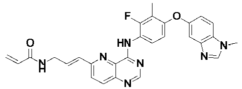 | ¹H-NMR (300 MHz, DMSO-*d*₆) δ 9.83 (s, 1H), 8.60-8.45 (m, 2H), 8.21 (s, 1H), 8.14 (d, 1H), 8.03 (d, 1H), 7.69 (t, 1H), 7.64 (d, 1H), 7.24 (d, 1H), 7.24-7.10 (m, 1H), 7.06 (dd, 1H), 6.80 (d, 1H), 6.69 (d, 1H), 6.33 (dd, 1H), 6.15 (dd, 1H), 5.65 (dd, 1H), 4.11 (t, 2H), 3.86 (s, 3H), 2.25 (s, 3H); |
| | | MS (ESI⁺): m/z =510.5 [M+H]⁺. |
| **36** | 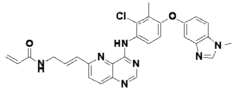 | ¹H-NMR (300 MHz, CDCl₃) δ 9.82 (s, 1H), 8.71 (s, 1H), 8.66-8.63 (d, 1H), 8.11-8.08 (d, 1H), 7.85 (s 1H), 7.76-7.73 (d,1H), 7.35-7.31 (m, 2H), 7.07-7.03 (d, 1H), 6.98-6.91 (m, 2H), 6.84-6.78 (d, 1H), 6.41-6.35 (d,1H), 5.75-5.71 (d, 1H), 6.23-6.14 (m, 1H), 5.89 (brs, 1H), 4.30-4.29 (m, 2H), 3.85 (s, 3H), 2.43 (s, 3H); |
| | | MS (ESI⁺): m/z =526.2 [M+H]⁺. |
| **37** | 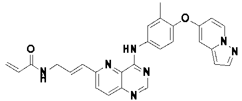 | ¹H-NMR (300 MHz, DMSO-*d*₆) δ 10.0 (s, 1H), 8.64 (d, 1H), 8.61 (s, 1H), 8.49 (t, 1H), 8.12 (d, 1H), 8.05 (d, 1H), 8.02-7.89 (m, 3H), 7.24-7.15 (m, 2H), 6.84-6.72 (m, 3H), 6.40-6.34 (m, 2H), 6.15 (d, 1H), 5.65 (d, 1H), 4.12-4.09 (m, 2H), 2.21 (s, 3H); |
| | | MS (ESI⁺): m/z =478.5 [M+H]⁺. |
| **38** | 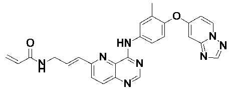 | ¹H-NMR (300 MHz, DMSO-*d*₆) δ 10.0 (s, 1H), 8.95 (d, 1H), 8.63 (s, 1H), 8.50 (t, 1H), 8.38 (s, 1H), 8.15 (d, 1H), 8.07-8.00 (m, 3H), 7.25-7.20 (m, 2H), 7.02 (dd, 1H), 6.86-6.80 (m, 2H), 6.38-6.29 (m, 1H), 6.15 (d, 1H), 5.67 (d, 1H), 4.14-4.11 (m, 2H), 2.22 (s, 3H); |
| | | MS (ESI⁺): m/z =479.2 [M+H]⁺. |
| **39** | 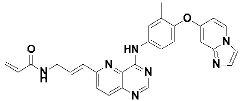 | ¹H-NMR (300 MHz, CDCl₃) δ 9.13 (s, 1H), 8.77 (s, 1H), 8.08-8.05 (m, 2H), 7.89-7.78 (m, 3H), 7.51 (d, 2H), 7.13 (d, 1H), 6.88-6.87 (m, 2H), 6.80-6.87 (m, 1H), 6.74-6.72 (m, 1H), 6.41 (d, 1H), 6.25-6.16 (m,1H), 5.90 (brs, 1H), 5.76 (d, 1H), 4.33-4.30 (m, 2H), 2.30 (s, 3H); |
| | | MS (ESI⁺): m/z =478.5 [M+H]⁺. |
| **40** | 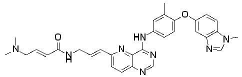 | ¹H-NMR (300 MHz, CD₃Cl₃) δ 9.06 (s, 1H), 8.73 (s, 1H), 8.10 (d, 1H), 7.87 (s, 1H), 7.81-7.69 (m, 3H), 7.35-7.34 (m, 2H), 7.09 (d, 1H), 6.97-6.84 (m, 4H), 6.06 (d, 1H), 5.77 (brs,1H), 4.31-4.28 (m, 2H), 3.87 (s, 3H), 3.11 (d, 2H), 2.38 (s, 3H), 2.29 (s, 6H); |
| | | MS (ESI⁺): m/z =549.6 [M+H]⁺. |
| **41** | 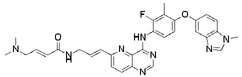 | ¹H-NMR (300 MHz, DMSO-*d*₆) δ 9.84 (t, 1H), 8.53 (s, 1H), 8.39 (s, 1H), 8.21 (s, 1H), 8.15 (d, 1H), 8.03 (d, 1H), 7.69 (t, 1H), 7.61 (d, 1H), 7.23 (d, 1H), 7.20-7.05 (m, 2H), 6.90-6.80 (m, 1H), 6.80-6.65 (m, 2H), 6.13 (d, 1H), 4.09 (t, 2H), 3.86 (s, 3H), 3.01 (d, 2H), 2.25 (d, 3H), 2.16 (s, 6H). |
| | | MS (ESI⁺): m/z =567.6 [M+H]⁺. |
| **42** | 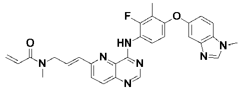 | ¹H-NMR (300 MHz, DMSO-*d*₆) δ 9.85 (d, 1H), 8.52 (s, 1H), 8.21 (s, 1H), 8.20-8.10 (m, 1H), 8.10-8.01 (m, 1H), 7.70-7.58 (m, 2H), 7.24 (d, 1H), 7.20-7.10 (m, 1H), 7.06 (dd, 1H), 6.90-6.65 (m, 3H), 6.18 (dd, 1H), 5.74 (dd, 1H), 4.38 (d, 1H), 4.29 (d, 1H), 3.86 (s, 3H), 3.06 (d, 3H), 2.25 (d, 3H); |
| | | MS (ESI⁺): m/z =524.5 [M+H]⁺. |
| **43** | 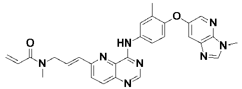 | ¹H-NMR (300 MHz, DMSO-*d*₆) δ 9.97 (s, 1H), 8.59 (s, 1H), 8.45 (s, 1H), 8.25 (d, 1H), 8.15-8.06 (m, 2H), 7.94 (s, 1H), 7.80 (dd, 1H), 7.62 (d, 1H), 7.28-7.13 (m, 1H), 6.90 (d, 1H), 6.81-6.75 (m, 2H), 6.21 (d, 1H), 5.77-5.68 (m, 1H), 4.41-4.20 (m, 2H), 3.86 (s, 3H), 2.31 (s, 3H); |
| | | MS (ESI⁺): m/z =507.6 [M+H]⁺. |
| **44** | 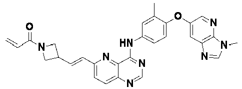 | ¹H-NMR (300 MHz, CDCl₃) δ 9.08 (s, 1H), 8.75 (s, 1H), 8.33 (s, 1H), 8.13 (d, 1H), 8.04 (s, 1H), 7.85-7.75 (m, 3H), 7.62 (s, 1H), 7.10-7.02 (m, 1H), 6.95 (d, 1H), 6.80 (d, 1H), 6.40 (d, 1H), 6.29-6.20 (m, 1H), 5.73 (d, 1H), 4.60-4.40 (m, 2H), 4.25-4.11 (m, 2H), 3.95 (s, 3H), 3.67 (brs, 1H), 2.39 (s, 3H); |
| | | MS (ESI⁺): m/z =519.6 [M+H]⁺. |
| **45** | 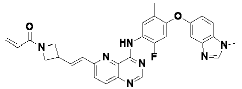 | ¹H-NMR (300 MHz, DMSO-*d*₆) δ9.82 (s, 1H), 8.53 (s, 1H), 8.22 (s, 1H), 8.16 (d, 1H), 7.99 (d, 1H), 7.76 (d, 1H), 7.63 (d, 1H), 7.49 (dd, 1H), 7.25 (d, 1H), 7.06 (dd, 1H), 6.83 (d, 1H), 6.72 (d, 1H), 6.35 (dd, 1H), 6.12 (dd, 1H), 5.71 (dd, 1H), 4.54 (t, 1H), 4.30-4.10 (m, 2H), 4.00-3.95 (m, 1H), 3.86 (s, 3H), 3.70-3.60 (m, 1H), 2.28 (s, 3H); |
| | | MS (ESI⁺): m/z =536.6 [M+H]⁺. |
| **46** | 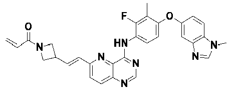 | ¹H-NMR (300 MHz, CDCl₃) δ 9.34 (s, 1H), 8.74 (s, 1H), 8.51-8.45 (m, 1H), 8.13 (d, 1H), 7.88 (s, 1H), 7.79 (d, 1H), 7.38-7.35 (m, 2H), 7.13-7.05 (m, 2H), 6.82-6.77 (m, 2H), 6.40 (d, 1H), 6.30-6.21 (m, 1H), 5.73 (d, 1H), 4.60-4.41 (m, 2H), 4.27-4.11 (m, 2H), 3.88 (s, 3H), 3.68 (brs, 1H), 2.34 (s, 3H); |
| | | MS (ESI⁺): m/z =536.6 [M+H]⁺. |
| **47** | 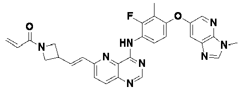 | ¹H-NMR (300 MHz, CDCl₃) δ 9.37 (s, 1H), 8.75 (s, 1H), 8.57-8.51 (t, 1H), 8.32 (s, 1H), 8.14 (d, 1H), 8.06 (s, 1H), 7.80 (d, 1H), 7.66 (s, 1H), 7.14-7.06 (m, 1H), 6.83-6.77 (m, 2H), 6.41 (d, 1H), 6.30-6.21 (m, 1H), 5.73 (d, 1H), 4.60-4.41 (m, 2H), 4.26-4.14 (m, 2H), 3.96 (s, 3H), 3.69 (brs, 1H), 2.35 (s, 3H); |
| | | MS (ESI⁺): m/z =537.5 [M+H]⁺. |
| **48** | 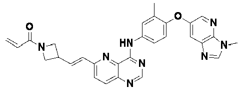 | ¹H-NMR (300 MHz, DMSO-*d*₆) δ 9.93 (s, 1H), 8.60 (s, 1H), 8.30 (s, 1H), 8.16 (d, 1H), 8.00 (d, 1H), 7.94 (d, 1H), 7.87 (dd, 1H), 7.72 (d, 1H), 7.52 (dd, 1H), 7.03 (d, 1H), 6.86 (dd, 1H), 6.84 (d, 1H), 6.73 (d, 1H), 6.36 (dd, 1H), 6.13 (dd, 1H), 5.70 (dd, 1H), 4.55 (t, 1H), 4.21 (q, 2H), 4.10 (s, 3H), 3.95 (dd, 1H), 3.70-3.60 (m, 1H), 2.24 (s, 3H); |
| | | MS (ESI⁺): m/z =518.6 [M+H]⁺. |
| **49** | 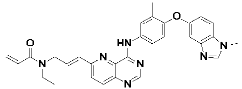 | ¹H-NMR (300 MHz, CDCl₃) δ 9.06 (s, 1H), 8.73 (s, 1H), 8.11-8.08 (d, 1H), 7.87 (s, 1H), 7.82-7.73 (m, 3H), 7.37-7.35 (d, 2H), 7.10-7.07 (d, 1H), 6.97-6.95 (d, 1H), 6.83 (s, 2H), 6.70-6.50 (m,2H), 5.81-5.78 (m, 1H), 4.37-4.31 (m, 2H), 3.87 (s, 3H), 3.63-3.51 (m, 2H), 2.38 (s, 3H), 1.57 (s, 3H); |
| | | MS (ESI⁺): m/z =520.6 [M+H]⁺. |
| **50** | 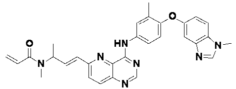 | ¹H-NMR (300 MHz, CDCl₃) δ 9.06 (s, 1H), 8.73 (s, 1H), 8.12-8.09 (d, 1H), 7.86-7.72 (m, 4H), 7.36-7.34 (m, 2H), 7.10-7.07 (d, 1H), 6.98-6.95 (d, 1H), 6.80 (s, 2H), 6.67-6.62 (m,1H), 6.47-6.41 (m, 1H), 5.77 (brs, 2H), 3.87 (s, 3H), 3.01 (s, 3H), 2.38 (s, 3H), 1.59 (s, 3H); |
| | | MS (ESI⁺): m/z =520.6 [M+H]⁺. |
| **51** | 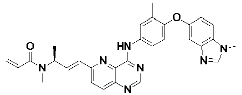 | ¹H-NMR (300 MHz, CDCl₃) δ 9.04 (s, 1H), 8.71 (s, 1H), 8.09-8.07 (d, 1H), 7.84-7.69 (m, 4H), 7.34-7.25 (m, 2H), 7.08-7.04 (d, 1H), 6.95-6.92 (d, 1H), 6.77 (s, 2H), 6.68-6.59 (m, 1H), 6.44-6.39 (m, 1H), 5.78 (brs, 2H), 3.84 (s, 3H), 2.98 (s, 3H), 2.35 (s, 3H), 1.26 (s, 3H); MS (ESI⁺): m/z =520.6 [M+H]⁺. |
| **52** | 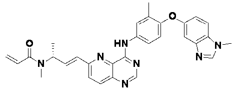 | ¹H-NMR (300 MHz, DMSO-*d*₆) δ 9.95 (s, 1H), 8.58 (s, 1H), 8.18 (s, 1H), 8.12-8.04 (m, 2H), 7.89 (s, 1H), 7.78 (d, 1H), 7.57 (d, 1H), 7.30-7.15 (m, 1H), 7.10 (d, 1H), 7.00 (dd, 1H), 6.90 (d, 1H), 6.80 (d, 2H), 6.19 (d, 1H), 5.75-5.69 (m, 1H), 5.60-5.00 (m, 1H), 3.84 (s, 3H), 2.91 (d, 3H), 2.27 (s, 3H), 1.42 (dd, 3H); |
| | | MS (ESI⁺): m/z =520.6 [M+H]⁺. |
| **53** | 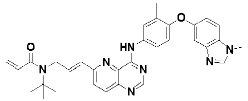 | ¹H-NMR (300 MHz, CDCl₃) δ 9.03 (s, 1H), 8.70 (s, 1H), 8.08-8.05 (d, 1H), 7.84-7.70 (m, 4H), 7.34-7.32 (d, 2H), 7.07-7.04 (d, 1H), 6.94-6.92 (d, 1H), 6.79 (s, 2H), 6.67-6.47 (m,2H), 5.78-5.75 (m, 1H), 4.34-4.28 (m, 2H), 3.84 (s, 3H), 2.35 (s, 3H), 1.42 (s, 9H); |
| | | MS (ESI⁺): m/z =548.7 [M+H]⁺. |
| **54** | 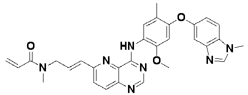 | ¹H-NMR (300 MHz, DMSO-*d*₆) δ 9.79 (d, 1H), 8.65 (d, 1H), 8.50 (d, 1H), 8.17-8.14 (m, 2H), 8.06-8.04 (m, 1H), 7.54 (d, 1H), 7.06 (d, 1H), 7.07-6.97 (m, 3H), 6.82-6.73 (m, 3H), 6.16 (d, 1H), 5.74-5.70 (m, 1H), 4.30 (d, 1H), 3.82 (s, 3H), 3.79 (d, 3H), 3.05 (d, 3H), 2.18 (s, 3H); |
| | | MS (ESI⁺): m/z =536.2 [M+H]⁺. |
| **55** | 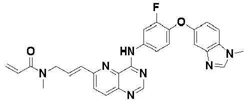 | ¹H-NMR (300 MHz, DMSO-*d*₆) δ 10.15 (s, 1H), 8.66 (s, 1H), 8.30-8.05 (m, 4H), 7.81 (d, 1H), 7.59 (d, 1H), 7.34-7.10 (m, 3H), 7.07 (dd, 1H), 6.90-6.70 (m, 2H), 6.18 (d, 1H), 5.75 (dd, 1H), 4.39 (d, 1H), 4.31 (d, 1H), 3.85 (s, 3H), 3.07 (d, 3H); |
| | | MS (ESI⁺): m/z =510.5 [M+H]⁺. |
| **56** | 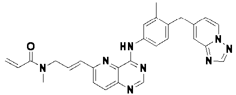 | ¹H-NMR (300 MHz, DMSO-*d*₆) δ 9.92 (s, 1H), 8.86 (d, 1H), 8.60 (s, 1H), 8.44 (s, 1H), 8.19-8.02 (m, 2H), 7.90-7.80 (m, 2H), 7.52 (s, 1H), 7.28 (d, 1H), 7.27-7.10 (m, 1H), 7.02 (dd, 1H), 7.92-7.70 (m, 2H), 6.19 (d, 1H), 5.80-5.65 (m, 1H), 4.37 (d, 1H), 4.30 (d, 1H), 4.14 (s, 2H), 3.07 (d, 3H), 2.28 (s, 3H); |
| | | MS (ESI⁺): m/z =491.5 [M+H]⁺. |
| **57** | 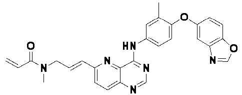 | ¹H-NMR (300 MHz, DMSO-*d*₆) δ 9.99 (s, 1H), 8.76 (s, 1H), 8.60 (s, 1H), 8.20-8.03 (m, 2H), 7.94 (s, 1H), 7.85 (d, 1H), 7.78 (d, 1H), 7.30-7.27 (m, 2H), 7.14-7.09 (m, 1H), 6.98 (d, 1H), 6.83-6.70 (m, 2H), 6.19 (dd, 1H), 5.72 (dd, 1H), 4.38 (d, 1H), 4.30 (d, 1H), 3.07 (d, 3H), 2.25 (s, 3H); |
| | | MS (ESI⁺): m/z =493.5 [M+H]⁺. |
| **58** | 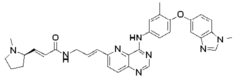 | ¹H-NMR (300 MHz, DMSO-*d*₆) δ 9.92 (s, 1H), 8.59 (s, 1H), 8.38 (t, 1H), 8.17 (s, 1H), 8.12 (d, 1H), 8.03 (d, 1H), 7.90 (d, 1H), 7.80 (dd, 1H), 7.57 (d, 1H), 7.30-7.15 (m, 1H), 7.11 (d, 1H), 7.00 (dd, 1H), 6.89 (dd, 1H), 6.83 (d, 1H), 6.51 (dd, 1H), 6.12 (d, 1H), 4.09 (t, 1H), 3.84 (s, 3H), 3.05-2.97 (m, 1H), 2.80-2.60 (m, 2H), 2.27 (s, 3H), 2.18 (s, 3H), 2.05-1.90 (m, 2H), 1.80-1.60 (m, 2H), 1.60-1.50 (m, 1H); |
| | | MS (ESI⁺): m/z =575.6 [M+H]⁺. |
| **59** | 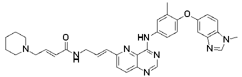 | ¹H-NMR (300 MHz, DMSO-*d*₆) δ 9.93 (s, 1H), 8.59 (s, 1H), 8.45 (s, 1H), 8.18 (s, 1H), 8.13 (d, 1H), 8.02 (d, 1H), 7.91 (d, 1H), 7.81 (dd, 1H), 7.57 (d, 1H), 7.25-7.15 (m, 1H), 7.10 (d, 1H), 7.00 (dd, 1H), 6.89 (d, 1H), 6.81 (d, 1H), 6.70-6.58 (m, 1H), 6.18 (d, 1H), 4.10 (t, 2H), 3.85 (s, 3H), 2.27 (s, 3H), 1.60-1.50 (m, 4H), 1.50-1.20 (m, 6H), 0.90-0.80 (m, 2H); |
| | | MS (ESI⁺): m/z =589.7 [M+H]⁺. |
| **60** | 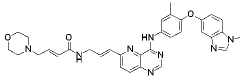 | ¹H-NMR (300 MHz, DMSO-*d*₆) δ 9.93 (s, 1H), 8.59 (s, 1H), 8.40 (t, 1H), 8.17 (s, 1H), 8.12 (d, 1H), 8.03 (d, 1H), 7.90 (d, 1H), 7.80 (dd, 1H), 7.57 (d, 1H), 7.27-7.15 (m, 1H), 7.10 (d, 1H), 7.00 (dd, 1H), 6.98 (d, 1H), 6.81 (d, 1H), 6.70-6.60 (m, 1H), 6.15 (d, 1H), 4.09 (t, 2H), 3.85 (s, 3H), 3.58 (t, 4H), 3.08 (dd, 2H), 2.38 (t, 4H), 2.27 (s, 3H); |
| | | MS (ESI⁺): m/z =591.7 [M+H]⁺. |
| **61** | 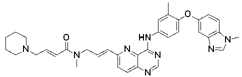 | ¹H-NMR (300 MHz, DMSO-*d*₆) δ 9.94 (s, 1H), 8.59 (s, 1H), 8.17 (s, 1H), 8.15-8.10 (m, 1H), 8.10-8.01 (m, 1H), 7.90 (d, 1H), 7.80 (d, 1H), 7.57 (d, 1H), 7.30-7.12 (m, 1H), 7.10 (d, 1H), 7.00 (dd, 1H), 6.89 (d, 1H), 6.80-6.70 (m, 1H), 6.70-6.50 (m, 2H), 4.36 (d, 1H), 4.29 (d, 1H), 3.85 (s, 3H), 3.15-3.07 (m, 2H), 3.07-3.00 (m, 3H), 2.35 (s, 2H), 2.27 (s, 5H), 1.60-1.48 (m, 2H), 1.45-1.35 (m, 3H), 1.32-1.20 (m, 1H); |
| | | MS (ESI⁺): m/z =603.7 [M+H]⁺. |
| **62** | 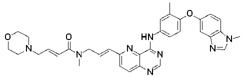 | ¹H-NMR (300 MHz, DMSO-*d*₆) δ 9.94 (s, 1H), 8.59 (s, 1H), 8.17 (s, 1H), 8.16-8.10 (m, 1H), 8.10-8.00 (m, 1H), 7.90 (d, 1H), 7.80 (d, 1H), 7.57 (d, 1H), 7.30-7.13 (m, 1H), 7.11 (d, 1H), 7.00 (dd, 1H), 6.89 (d, 1H), 6.85-6.70 (m, 1H), 6.70-6.50 (m, 2H), 4.42-4.33 (m, 1H), 4.31-4.22 (m, 1H), 3.85 (s, 3H), 3.59 (t, 2H), 3.48 (t, 2H), 3.12 (s, 2H), 3.07 (d, 1H), 3.00 (s, 2H), 2.38 (s, 2H), 2.31 (s, 2H), 2.27 (s, 3H); |
| | | MS (ESI⁺): m/z =605.7 [M+H]⁺. |
| **63** | 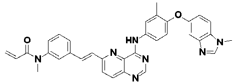 | ¹H-NMR (300 MHz, DMSO-*d*₆) δ 9.97 (s, 1H), 8.61 (s, 1H), 8.29-8.17 (m, 3H), 8.08 (d, 1H), 7.95 (d, 1H), 7.85 (dd, 1H), 7.75 (d, 1H), 7.66 (d, 2H), 7.59 (d, 1H), 7.55 (d, 1H), 7.31 (d, 1H), 7.11 (d, 1H), 7.01 (dd, 1H), 6.92 (d, 1H), 6.30-6.10 (m, 2H), 5.64-5.60 (m, 1H), 3.85 (s, 3H), 3.30 (s, 3H, hide), 2.29 (s, 3H); |
| | | MS (ESI⁺): m/z =568.6 [M+H]⁺. |
| **64** | 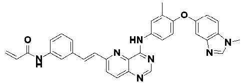 | ¹H-NMR (300 MHz, DMSO-*d*₆) δ 10.28 (s, 1H), 10.07 (s, 1H), 8.61 (s, 1H), 8.21-8.12 (m, 5H), 7.97 (d, 1H), 7.86 (dd, 1H), 7.60-7.50 (m, 2H), 7.50-7.42 (m, 3H), 7.12 (d, 1H), 7.01 (dd, 1H), 6.92 (d, 1H), 6.49 (dd, 1H), 6.30 (dd, 1H), 5.80 (dd, 1H), 3.85 (s, 3H), 2.29 (s, 3H); |
| | | MS (ESI⁺): m/z =554.6 [M+H]⁺. |

### Example 65: Synthesis of N-(3-(4-((4-([1,2,4]triazolo[1,5-a]pyridin-7-yloxy)-3-methylphenyl)amino)quinazolin-6-yl)prop-2-yn-1-yl)-N-methylacrylamide

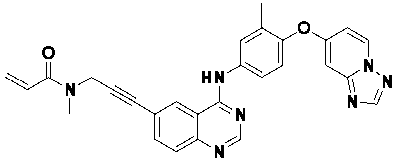

### Step (1): Preparation of 6-iodo-N-[3-methyl-4-([1,2,4]triazolo[1,5-a]pyridin-7-yloxy)phenyl]quinazolin-4-amine

300 mg (1.03 mmol) of 4-chloro-6-iodoquinazoline and 248 mg (1.03 mmol) of 3-methyl-4-([1,2,4]triazolo[1,5-*a*]pyridin-7-yloxy)aniline were diluted in a solvent mixture of 2.4 mL of isopropyl alcohol and 2.4 mL of 1,2-dichloroethane and the solution was stirred at 80°C for 4 hours. The reaction mixture was diluted with ethyl acetate and was washed with distilled water and saturated brine. The organic layer thus separated was dried over anhydrous sodium sulfate and was suction filtered and vacuum distilled. The resultant residue was purified with column chromatography (dichloromethane:methanol = 15:1 (volume ratio)) to give 277 mg of the title compound (54% yield).

¹H-NMR (300MHz, DMSO-*d₆*) δ 9.92 (s, 1H), 9.03 (s, 1H), 8.93 (d, 1H). 8.65 (s, 1H), 8.38 (s, 1H), 8.13 (d, 1H), 7.88-7.85 (m, 2 H), 7.57 (d, 1H), 7.22 (d, 1H), 7.03 (d, 1H), 6.81 (s, 1H), 2.20 (s, 3H).

### Step (2): Preparation of tert-butyl N-methyl-N-[3-[4-[3-methyl-4-([1,2,4]triazolo[1,5-a]pyridin-7-yloxy)anilino]quinazolin-6-yl]prop-2-ynyl]carbamate

150 mg (0.30 mmol) of the compound prepared in step (1), 102 mg (0.60 mmol) of *tert-*butyl *N*-methyl-*N*-prop-2-ynyl-carbamate, 11 mg (0.06 mmol) of copper(I) iodide, 21 mg (0.03 mmol) of bis(triphenylphosphine)palladium(II) dichloride and 0.17 mL (1.21 mmol) of triethylamine were diluted in 1.5 mL of *N,N-*dimethylformamide and the mixture was stirred at 70°C for 16 hours. Upon completion of the reaction, the reaction mixture was allowed to cool to room temperature and diluted with ethyl acetate, followed by washing with distilled water and saturated brine. The organic layer thus separated was dried over anhydrous sodium sulfate and was suction filtered and vacuum distilled. The resultant residue was purified with column chromatography (hexane:ethyl acetate = 1:1 (volume ratio)) to give 100 mg of the title compound (61% yield).

¹H-NMR (300MHz, CDCl₃) δ 8.78 (s, 1H), 8.52 (d, 1H), 8.25 (s, 1H). 8.10 (s, 1H), 7.88-7.69 (m, 4H), 7.12 (d, 1H), 6.92-6.87 (m, 2 H), 4.34 (s, 2H), 4.15-4.12 (m, 1H), 3.03 (s, 3H), 2.25 (s, 3H), 1.52 (s, 9H).

### Step (3): Preparation of N-(3-(4-((4-([1,2,4]triazolo[1,5-a]pyridin-7-yloxy)-3-methylphenyl)amino)quinazolin-6-yl)prop-2-yn-1-yl)-N-methylacrylamide

100 mg (0.18 mmol) of the compound prepared in step (2) was diluted in a mixture of 0.34 mL (4.5 mmol) of trifluoroacetic acid and 1.2 mL of dichloromethane and the resulting solution was stirred at ambient temperature for 2 hours. Upon completion of the reaction, the reaction mixture was vacuum distilled. 18 µL (0.27 mmol) of acrylic acid and 81 µL (0.27 mmol) of propylphosphonic acid anhydride were diluted in 2 mL of *N,N-*dimethylformamide, followed by addition of 0.22 mL (1.26 mmol) of *N,N-*diisopropylethylamine and stirring at ambient temperature for 30 minutes. This resultant solution was transferred to the residue obtained from the vacuum distillation and the mixture was stirred at 0°C for 2 hours. Upon completion of the reaction, the reaction mixture was diluted with ethyl acetate and washed with distilled water and saturated brine. The organic layer thus separated was dried over anhydrous sodium sulfate and was suction filtered and vacuum distilled. The resultant residue was purified with column chromatography (dichloromethane:methanol = 10:1 (volume ratio)) to give 12 mg of the title compound (13% yield).
¹H-NMR (300MHz, DMSO-*d*₆) δ 9.96 (s, 1H), 8.93 (d, 1H), 8.76 (s, 1H), 8.63 (s, 1H), 8.38 (s, 1H), 7.89-7.74 (m, 4H), 7.22 (d, 1H), 7.03 (d, 1H), 6.81 (d, 2H), 6.21 (d, 1H), 5.77 (d, 1H), 4.65 (s, 2H), 3.33 (s, 3H), 2.20 (s, 3H);
MS (ESI+): m/z = 490.2 [M+H]⁺.

The compounds of Examples 66 to 73 shown in Table 4 below were prepared using the same or similar procedures as described in Example 65 above.

**[Table 4]**

| | Structural Formula | Analysis Data |
|---|---|---|
| **66** | 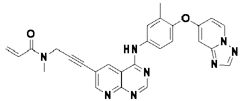 | ¹H-NMR (300MHz, DMSO-*d*₆) δ 10.2 (s, 1H), 9.21-9.13 (m, 1H), 9.07 (s, 1H), 8.99-8.91 (m, 1H), 8.76 (s, 1H), 8.39 (s, 1H), 7.88 (s, 1H), 7.86 (d, 1H), 7.24 (d, 1H), 7.06-7.01 (m, 1H), 7.00-6.78 (m, 2H), 6.28-6.18 (m, 1H), 5.80-5.76 (m, 1H), 4.75 (d, 2H), 3.16 (d, 3H), 2.21 (s, 3H); |
| | | MS (ESI⁺): m/z =491.2 [M+H]⁺. |
| **67** | 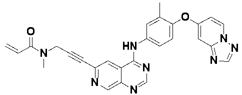 | ¹H-NMR (300MHz, DMSO-*d*₆) δ 8.99 (s, 1H), 8.93 (d, 1H), 8.61-8.58 (m, 2H), 8.38 (s, 1H), 7.80-7.76 (m, 3H), 7.18 (d, 2H), 7.02 (d, 2H), 6.21 (d, 1H), 5.77 (d, 1H), 4.67-4.56 (m, 2H), 3.33 (s, 3H), 2.19 (s, 3H); |
| | | MS (ESI+): m/z =491.2 [M+H]⁺. |
| **68** | 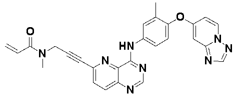 | ¹H-NMR (300MHz, DMSO-*d*₆) δ 10.2 (s, 1H), 8.97-8.92 (m, 1H), 8.68 (s, 1H), 8.39 (s, 1H), 8.20 (d, 1H), 8.05 (s, 1H), 7.99 (d, 2H), 7.21 (d, 1H), 7.03 (dd, 1H), 7.00-6.80 (m, 2H), 6.22 (dd, 1H), 5.78 (dd, 1H), 4.65 (d, 2H), 3.18 (s, 3H), 2.20 (s, 3H); |
| | | MS (ESI+): m/z =491.2 [M+H]⁺. |
| **69** | 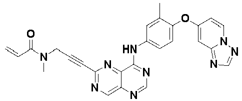 | ¹H-NMR (300MHz, DMSO-*d*₆) δ 8.97-8.92 (m, 1H), 8.68 (s, 1H), 8.39 (s, 1H), 8.20 (d, 1H), 8.05 (s, 1H), 7.99 (d, 2H), 7.21 (d, 1H), 7.03 (dd, 1H), 7.00-6.80 (m, 2H), 6.22 (dd, 1H), 5.78 (dd, 1H), 4.65 (d, 2H), 3.18 (s, 3H), 2.20 (s, 3H); |
| | | MS (ESI+): m/z =492.2 [M+H]⁺. |
| **70** | 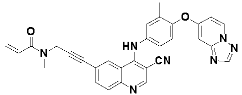 | ¹H-NMR (300MHz, DMSO-*d*₆) δ 10.1 (s, 1H), 8.96-8.93 (m, 1H), 8.70 (s, 1H), 8.37 (s, 1H), 8.58 (s, 1H), 7.93-7.80 (m, 2H), 7.40 (s, 1H), 7.38-7.23 (m, 2H), 7.13-7.05 (m, 1H), 7.00-6.80 (m, 2H), 6.20 (dd, 1H), 5.78 (dd, 1H), 4.60 (d, 2H), 3.15 (d, 3H), 2.18 (s, 3H); |
| | | MS (ESI+): m/z =514.2 [M+H]⁺. |
| **71** | 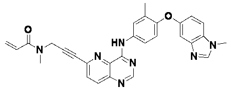 | ¹H-NMR (300MHz, DMSO-*d*₆) δ 10.0 (d, 1H), 8.59 (s, 1H), 8.13 (d, 2H), 7.94-7.83 (m, 1H), 7.73 (s, 1H), 7.55 (d, 1H), 7.08 (d, 1H), 6.98 (dd, 1H), 6.85-6.76 (m, 2H), 6.66-6.56 (m, 1H), 6.20 (d, 1H), 5.72 (d, 1H), 4.53 (s, 2H), 3.82 (s, 3H), 2.23 (s, 3H), 2.02 (s, 3H); |
| | | MS (ESI+): m/z =504.2 [M+H]⁺. |
| **72** | 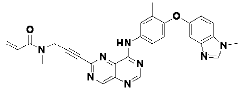 | ¹H-NMR (300MHz, CDCl₃) δ 9.35 (s, 1H), 8.90 (s, 1H), 8.83 (s, 1H), 7.88 (s, 1H), 7.82 (s, 1H), 7.73 (d, 1H), 7.38-7.30 (m, 2H), 7.11 (d, 1H), 6.95 (d, 1H), 6.67-6.61 (m, 1H), 6.46 (d, 1H), 5.83 (d, 1H), 4.60 (s, 2H), 3.88 (s, 3H), 3.31 (s, 3H), 2.39 (s, 3H); |
| | | MS (ESI+): m/z =505.2 [M+H]⁺. |
| **73** | 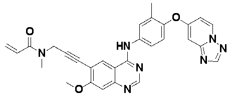 | ¹H-NMR (300MHz, CDCl₃) δ 8.48-8.38 (m, 2H), 8.22 (s, 1H), 7.81 (s, 1H), 7.04-6.87 (m, 7H), 6.69-6.63 (m, 1H), 6.58 (s, 1H), 5.83 (d, 1H), 4.62 (s, 2H), 3.94 (s, 3H), 3.75 (s, 3H), 2.18 (s, 3H); |
| | | MS (ESI+): m/z =520.2 [M+H]⁺. |

### Example 74: Synthesis of (R)-1-(2-((4-((4-([1,2,4]triazolo[1,5-a]pyridin-7-yloxy)-3-methylphenyl)amino)quinazolin-6-yl)ethynyl)pyrrolidin-1-yl)prop-2-en-1-one

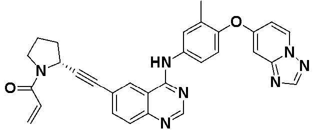

### Step (1): Preparation of N-(4-([1,2,4]triazolo[1,5-a]pyridin-7-yloxy)-3-methylphenyl)-6-iodoquinazolin-4-amine

1.5 g (5.16 mmol) of 4-chloro-6-iodoquinazoline and 1.24 g (5.16 mmol) of 4-([1,2,4]triazolo[1,5-*a*]pyridin-7-yloxy)-3-methylaniline were diluted in a solvent mixture of 8 mL of isopropyl alcohol and 8 mL of 1,2-dichloroethane and the resulting solution was stirred at 80°C for 12 hours. Upon completion of the reaction, the reaction mixture was allowed to cool to room temperature and vacuum distilled. The resultant residue was washed with isopropyl alcohol and hexane to give 2.73 g of the title compound (100% yield).

¹H-NMR (300 MHz, DMSO-*d₆*) δ 11.4 (s, 1H), 9.29 (d, 1H), 8.99 (s, 1H), 8.97 (s, 1H), 8.43 (s, 1H), 8.37 (dd, 1H), 7.79 (d, 1H), 7.73 (d, 2H), 7.30 (d, 1H), 7.06 (dd, 1H), 6.87 (d, 1H), 2.24 (s, 3H).

### Step (2): Preparation of tert-butyl (R)-2-ethynylpyrrolidin-1-carboxylate

500 mg (2.5 mmol) of *tert-*butyl (*R*)-2-formylpyrrolidin-l-carboxylate and 694 mg (5.0 mmol) of potassium carbonate were diluted in 20 mL of methanol. To this mixture was added 578 mg (3.0 mmol) of dimethyl (1-diazo-2-oxopropyl)phosphonate, followed by stiring at ambient temperature for 3 hours. Upon completion of the reaction, the reaction mixture was vacuum distilled and diluted in dichloromethane, followed by washing with saturated aqueous sodium bicarbonate. The organic layer thus separated was dried over anhydrous sodium sulfate and was suction filtered and vacuum distilled to give 476 mg of the title compound (97% yield).

¹H-NMR (300 MHz, CDCl₃) δ 4.75-4.40 (m, 1H), 3.48 (bs, 1H), 3.35 (bs, 1H), 2.23 (s, 1H), 2.20-2.00 (m, 3H), 2.00-1.85 (m, 1H), 1.50 (s, 9H).

### Step (3): Preparation of tert-butyl (R)-2-((4-((4-([1,2,4]triazolo[1,5-a]pyridin-7-yloxy)-3-methylphenyl)amino)quinazolin-6-yl)ethynyl)pyrrolidin-1-carboxylate

300 mg (0.6 mmol) of the compound prepared in step (1), 5.8 mg (0.03 mmol) of copper(I) iodide and 30 mg (0.04 mmol) of bis(triphenylphosphine)palladium(II) dichloride were diluted in 2 mL of *N*,*N*-dimethylformamide and the mixture was stirred at ambient temperature. 178 mg (0.91 mmol) of the compound prepared in step (2) and 0.25 mL (1.82 mmol) of triethylamine were dissolved in 1 mL of *N*,*N*-dimethylformamide and the resulting solution was added dropwise to the reactants followed by stirring for 18 hours at 65°C. Upon completion of the reaction, the reaction mixture was allowed to cool to room temperature and diluted with ethyl acetate, followed by washing with distilled water and brine. The organic layer thus separated was dried over anhydrous sodium sulfate and was suction filtered and vacuum distilled. The resultant residue was purified with column chromatography (dichloromethane:methanol = 15:1 (volume ratio)) to give 310 mg of the title compound (91% yield).

¹H-NMR (300 MHz, DMSO-*d₆*) δ 9.94 (d, 1H), 8.94 (d, 1H), 8.71 (d, 2H), 8.39 (s, 1H), 8.07-7.95 (m, 2H), 7.70-7.55 (m, 1H), 7.25 (d, 2H), 7.03 (dd, 1H), 6.82 (s, 1H), 4.09 (d, 4H), 2.21 (s, 3H), 2.10-1.85 (m, 3H), 1.44 (s, 9H).

### Step (4): Preparation of (R)-N-(4-([1,2,4]triazolo[1,5-a]pyridin-7-yloxy)-3-methylphenyl)-6-(pyrrolidin-2-ylethynyl)quinazolin-4-amine 2,2,2-trifluoroacetic acid

305 mg (0.54 mmol) of the compound prepared in step (3) was diluted in 2 mL of dichloromethane. To this mixture, 1 mL (13.1 mmol) of trifluoroacetic acid was added, followed by stirring at ambient temperature for 2 hours. Upon completion of the reaction, the reaction mixture was vacuum distilled to yield 400 mg of product mixture, which was used without purification in the following step.

### Step (5): Preparation of (R)-1-(2-((4-((4-([1,2,4]triazolo[1,5-a]pyridin-7-yloxy)-3-methylphenyl)amino)quinazolin-6-yl)ethynyl)pyrrolidin-1-yl)prop-2-en-1-one

0.05 mL (0.75 mmol) of acrylic acid and 0.26 mL (1.5 mmol) of *N,N-*diisopropylethylamine were diluted in 1 mL of *N*,*N*-dimethylformamide, followed by addition of 0.22 mL (0.75 mmol) of propylphosphonic acid anhydride and stirring at ambient temperature for 30 minutes. This solution was added dropwise at 0 °C to a 1 mL *N,N-*dimethylformamide solution containing 288 mg (0.5 mmol) of the compound prepared in step (4) and 0.39 mL (2.25 mmol) of *N,N-*diisopropylethylamine and the resulting mixture was stirred for 3 hours. Upon completion of the reaction, the reaction mixture was diluted with ethyl acetate and washed with water. The organic layer thus separated was dried over anhydrous sodium sulfate and was suction filtered and vacuum distilled. The resultant residue was purified with column chromatography (dichloromethane:methanol = 10:1 (volume ratio)) to give 18.0 mg of the title compound (7% yield).
¹H-NMR (300 MHz, DMSO-*d*₆) δ 10.0 (s, 1H), 8.95 (d, 1H), 8.70 (s, 1H), 8.63 (s, 1H), 8.39 (s, 1H), 7.93-7.70 (m, 4H), 7.22 (d, 1H), 7.03 (dd, 1H), 6.90-6.60 (m, 2H), 6.30-6.18 (m, 1H), 5.80-5.70 (m, 1H), 5.28-4.98 (m, 1H), 3.77-3.50 (m, 2H), 2.40-2.00 (m, 7H);
MS (ESI+): m/z = 516.2 [M+H]⁺.

The compounds of Examples 75 to 92 shown in Table 5 below were prepared using the same or similar procedures as described in Example 74 above.

**[Table 5]**

| | Structural Formula | Analysis Data |
|---|---|---|
| **75** | 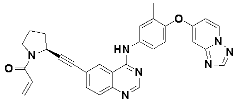 | ¹H-NMR (300 MHz, DMSO-*d*₆) δ 9.98 (s, 1H), 8.93 (d, 1H), 8.66-8.55 (m, 2H), 8.38 (s, 1H), 7.84-7.71 (m, 4H), 7.20 (d, 1H), 7.04-7.01 (m, 1H), 6.87-6.78 (m, 1H), 6.26-6.19 (m, 1H), 5.80-5.72 (m, 2H), 5.21-4.99 (m, 1H), 4.42-4.37 (m, 2H), 3.77-3.57 (m, 2H), 2.27 (s, 3H) 2.19-2.08 (m, 2H); |
| | | MS (ESI+): m/z = 516.2 [M+H]⁺. |
| **76** | 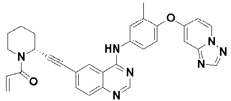 | ¹H-NMR (300 MHz, CDCl₃) δ 8.80 (s, 1H), 8.52 (d, 1H), 8.25 (s, 1H). 8.07 (s, 1H), 7.89-7.69 (m, 4H), 7.14 (d, 1H), 6.93-6.88 (m, 2H), 6.68-6.59 (m, 1H), 6.38-6.33 (m, 1H), 6.00 (s, 1H), 5.77 (d, 1 H), 3.92-3.50 (m, 1H), 2.27 (s, 3H), 2.03-1.99 (m, 2H), 1.85-1.80 (m, 4H), 1.62-1.51 (m, 2H); |
| | | MS (ESI+): m/z = 530.2 [M+H]⁺. |
| **77** | 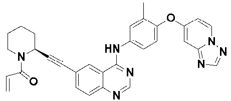 | ¹H-NMR (300 MHz, CDCl₃) δ 8.81 (s, 1H), 8.52 (d, 1H), 8.25 (s, 1H). 8.15-8.08 (m, 2H), 7.87-7.71 (m, 4H), 7.12 (d, 1H), 6.93-6.86 (m, 2H), 6.67-6.58 (m, 1H), 6.37-5.97 (m, 1H), 5.76 (d, 1 H), 3.95-3.50 (m, 1H), 2.25 (s, 3H), 2.00-1.96 (m, 2H), 1.82-1.72 (m, 4H), 1.61-1.53 (m, 2H); |
| | | MS (ESI+): m/z = 530.2 [M+H]⁺. |
| **78** | 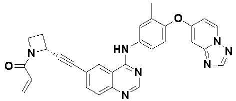 | ¹H-NMR (300 MHz, CDCl₃) δ 8.80 (s, 1H), 8.52 (d, 1H), 8.25 (s, 1H). 8.15 (s, 1H), 7.88-7.72 (m, 5H), 7.13 (d, 1H), 6.93-6.90 (m, 2H), 6.49-6.43 (m, 1H), 5.77 (d, 1H), 5.51 (d, 1H), 4.38-4.12 (m, 1H), 2.83-2.52 (m, 2H), 2.27 (s, 3H), 1.30-1.24 (m, 2H); |
| | | MS (ESI+): m/z = 502.2 [M+H]⁺. |
| **79** | 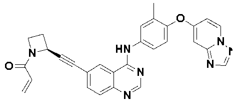 | ¹H NMR (300 MHz, DMSO-*d*₆) δ 9.99 (s, 1H), 8.92 (d, 1H), 8.80 (s, 1H), 8.63 (d, 1H), 8.37 (s, 1H), 7.93-7.67 (m, 5H), 7.43 (s, 1H), 7.21 (d, 1H), 7.02 (dd, 1H), 6.80 (d, 1H), 5.49 (d, 1H), 3.43-3.41 (m, 1H), 2.19 (s, 3H), 1.29-1.13 (m, 4H); |
| | | MS (ESI+): m/z = 502.2 [M+H]⁺. |
| **80** | 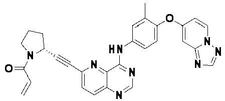 | ¹H-NMR (300 MHz, DMSO-*d*₆) δ 10.2 (s, 1H), 8.95 (d, 1H), 8.67 (d, 1H), 8.39 (s, 1H), 8.23-8.14 (m, 1H), 8.09-8.01 (m, 1H), 8.01-7.90 (m, 2H), 7.22 (d, 1H), 7.10-7.00 (m, 1H), 6.90-6.60 (m 2H), 6.30-6.19 (m, 1H), 5.60 (d, 1H), 3.86-3.40 (m, 1H), 2.35 (s, 3H), 2.30-1.95 (m, 6H); |
| | | MS (ESI+): m/z = 517.2 [M+H]⁺. |
| **81** | 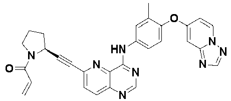 | ¹H-NMR (300 MHz, CDCl₃) δ 9.54 (s, 1H), 9.05 (s, 1H), 8.54 (s, 1H), 8.07 (s, 1H), 7.86 (s, 1H), 7.74-7.63 (m, 1H), 7.34-7.32 (m, 2H), 7.08 (d, 1H), 6.97 (d, 1H), 6.55-6.46 (m, 2H), 6.32 (d, 1H), 5.66 (d, 1H), 4.02-3.99 (m, 1H), 3.86 (s, 3H), 3.81-3.79 (m, 2H), 3.38-3.15 (m, 4H), 2.35 (s, 3H); |
| | | MS (ESI+): m/z = 517.2 [M+H]⁺. |
| **82** | 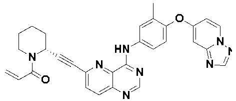 | ¹H NMR (300 MHz, DMSO-*d₆*) δ 10.2 (s, 1H), 9.00-8.87 (m, 1H), 8.67 (s, 1H), 8.38 (s, 1H), 8.20 (d, 1H), 8.12-7.93 (m, 3H), 7.21 (d, 1H), 7.02 (d, 1H), 6.80-6.79 (m, 2H), 6.18 (d, 1H), 5.78 (d, 1H), 3.42-3.39 (m, 1H), 2.20 (s, 3H), 1.96-1.94 (m, 2H), 1.78-1.74 (m, 2H), 1.26-1.24 (m, 2H), 0.88-0.86 (m, 2H); |
| | | MS (ESI+): m/z = 531.2 [M+H]⁺. |
| **83** | 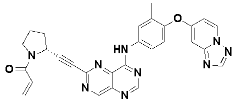 | ¹H-NMR (300 MHz, CDCl₃) δ 9.37 (d, 1H), 9.01 (d, 1H), 8.87 (d, 1H), 8.52 (d, 1H), 8.25 (s, 1H), 7.98-7.92 (m, 2H), 7.19-7.16 (m, 1H), 6.93-6.87 (m, 2H), 6.52-6.49 (m, 2H), 5.81-5.77 (m, 1H), 5.22-4.96 (m, 1H), 3.86-3.62 (m, 2H), 2.40-2.38 (m, 2H), 2.30 (s, 3H), 2.29-2.01 (m, 2H); |
| | | MS (ESI+): m/z = 518.2 [M+H]⁺. |
| **84** | 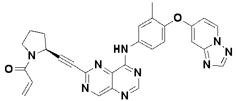 | ¹H-NMR (300 MHz, CDCl₃) δ 9.36 (s, 1H), 9.00 (d, 1H), 8.85 (s, 1H), 8.54 (s, 1H), 8.25 (s, 1H), 7.98-7.92 (m, 3H), 7.19-7.16 (m, 1H), 6.93-6.86 (m, 2H), 6.53-6.49 (m, 1H), 5.78 (m, 1H), 5.23-4.95 (m, 1H), 3.86-3.62 (m, 2H), 2.43-2.40 (m, 2H), 2.38 (s, 3H), 2.36-2.25 (m, 2H), 1.28-1.27 (m, 1H); |
| | | MS (ESI+): m/z = 518.2 [M+H]⁺. |
| **85** | 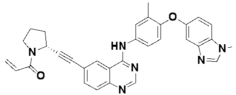 | ¹H NMR (300 MHz, DMSO-*d₆*) δ 9.86 (s, 1H), 8.65 (s, 1H), 8.56 (s, 1H), 8.16 (s, 1H), 7.85-7.66 (m, 3H), 7.56 (d, 1H), 7.10 (d, 1H), 6.99 (d, 1H), 6.85 (d, 1H), 6.57 (s, 1H), 6.32-6.14 (d, 1H), 5.76-5.75 (m, 2H), 3.83 (s, 3H), 3.48-3.47 (m, 1H), 3.23-2.88 (m, 4H), 2.24 (s, 3H), 2.20-2.08 (m, 2H); |
| | | MS (ESI+): m/z = 529.2 [M+H]⁺. |
| **86** | 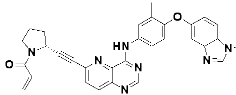 | ¹H NMR (300 MHz, DMSO-*d₆*) δ 10.1 (s, 1H), 8.62 (s, 1H), 8.23-8.13 (m, 2H), 7.99-7.85 (m, 2H), 7.77 (d, 1H), 7.57 (d, 1H), 7.10 (d, 1H), 7.00 (d, 1H), 6.99-6.85 (m, 2H), 6.20 (d, 1H), 5.83-5.69 (m, 1H), 4.09-4.07 (m, 1H), 3.84 (s, 3H), 3.27-3.08 (m, 4H), 3.08-3.04 (m, 2H), 2.25 (s, 3H); |
| | | MS (ESI+): m/z = 532.2 [M+H]⁺. |
| **87** | 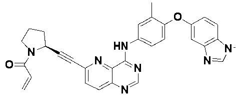 | ¹H-NMR (300 MHz, CDCl₃) δ 9.05 (s, 1H), 8.75 (s, 1H), 8.12-8.05 (m, 1H) 7.87 (s, 1H), 7.82-7.70 (m, 3H), 7.36-7.34 (m, 2H). 7.10 (d, 1H), 6.96 (d, 1H), 6.83-6.48 (m, 2H), 5.81 (d, 1H). 5.20-5.18 (m, 1H), 3.87 (s, 3H), 3.64-3.61 (m, 2H), 2.38 (s, 3H), 2.32-2.10 (m, 4H); |
| | | MS (ESI+): m/z = 532.2 [M+H]⁺. |
| **88** | 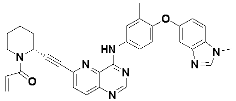 | ¹H NMR (300 MHz, DMSO-*d₆*) δ 10.1 (s, 1H), 8.62 (s, 1H), 8.21-8.14 (m, 2H), 8.01 (d, 1H), 7.91 (d, 1H), 7.77 (d, 1H), 7.57 (d, 1H), 7.11 (d, 1H), 7.00 (d, 1H), 6.86 (d, 2H), 6.18 (d, 1H), 5.80-5.68 (m, 1H), 4.07-4.03 (m, 1H), 3.84 (s, 3H), 2.25 (s, 3H), 1.77 (m, 4H), 1.32-1.01 (m, 4H); |
| | | MS (ESI+): m/z = 544.2 [M+H]⁺. |
| **89** | 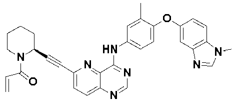 | ¹H NMR (300 MHz, DMSO-*d₆*) δ 10.1 (s, 1H), 8.62 (s, 1H), 8.21-8.12 (m, 2H), 8.01 (d, 1H), 7.91 (d, 1H), 7.81-7.72 (m, 1H), 7.57 (d, 1H), 7.11 (d, 1H), 7.00 (d, 1H), 6.86 (d, 1H), 6.18 (d, 1H), 5.86 (s, 1H), 5.80 (d, 1H), 4.08-4.06 (m, 1H), 3.84 (s, 3H), 2.25 (s, 3H), 1.99-1.94 (m, 3H), 1.78-1.74 (m, 3H), 1.50-1.45 (m, 2H); |
| | | MS (ESI+): m/z = 544.2 [M+H]⁺. |
| **90** | 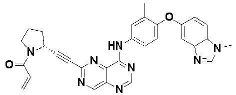 | ¹H-NMR (300 MHz, CDCl₃) δ 9.35 (s, 1H), 8.93 (s, 1H), 8.82 (s, 1H), 7.87 (s, 1H), 7.82 (s, 1H), 7.73 (t, 1H), 7.38-7.35 (m, 1H), 7.10 (d, 1H), 6.96 (d. 1H), 6.83-6.74 (m, 1H), 6.52-6.46 (m, 2H), 5.80 (d, 1H), 5.22-4.94 (m, 1H), 3.88 (s, 3H), 3.84-3.64 (m, 2H), 2.39 (s, 3H), 2.34-2.10 (m, 4H); |
| | | MS (ESI+): m/z = 533.2 [M+H]⁺. |
| **91** | 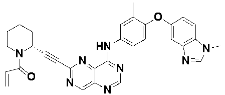 | ¹H-NMR (300 MHz, CDCl₃) δ 9.35 (s, 1H), 8.91 (s, 1H), 8.82 (s, 1H), 7.84 (d, 2H), 7.73 (d, 1H), 7.38-7.35 (m, 2H), 7.10 (d, 1H), 6.94 (d. 1H), 6.64-6.59 (m, 1H), 6.36 (d, 1H), 5.77 (d, 1H), 3.87 (s, 3H), 3.71-3.64 (m, 1H), 2.39 (s, 3H), 2.13-2.04 (m, 2H), 1.87-1.83 (m, 2H), 1.59-1.55 (m, 4H); |
| | | MS (ESI+): m/z = 545.2 [M+H]⁺. |
| **92** | 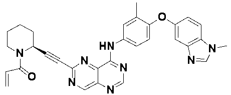 | ¹H-NMR (300 MHz, CDCl₃) δ 9.36 (s, 1H), 8.91 (s, 1H), 8.82 (s, 1H), 7.84 (d, 2H), 7.73 (d, 1H), 7.38-7.35 (m, 2H), 7.09 (d, 1H), 6.94 (d. 1H), 6.83-6.59 (m, 1H), 6.36 (d, 1H), 5.77 (d, 1H), 3.88 (s, 3H), 3.71-3.64 (m, 1H), 2.39 (s, 3H), 2.09-2.05 (m, 2H), 1.87-1.83 (m, 2H), 1.59-1.51 (m, 4H); |
| | | MS (ESI+): m/z = 545.2 [M+H]⁺. |

### Experimental Example: Growth inhibition of Ba/F3 HER2 A775insYVMA and wild-type Ba/F3 cells

In this Experimental Example, the compounds obtained in Examples 1 to 92 were evaluated for their ability to inhibit growth of cells harboring either the HER2 A775insYVMA mutation or wild-type EGFR. For this purpose, Ba/F3 cells (ATCC, USA) were genetically engineered to overexpress either HER2 A775_G776insYVMA or wild-type EGFR, and the growth of these engineered Ba/F3 cells were monitored in the presence of the compounds described in the Examples to evaluate their effects on growth inhibition.

Specifically, culture flasks of the engineered Ba/F3 cells suspended in RPMI 1640 medium supplemented with 10% fetal bovine serum (FBS) and 1% penicillin/streptomycin (Gibco BRL, USA) were placed in a cell incubator and incubated at 37°C and 5% CO₂ level to allow them to reach a stable logarithmic growth phase. The cells were then harvested by centrifugation to remove the old media and placed into a 96-well plate by dividing them into 100 µL aliquots per well at a dilution of 1×10⁵ cells/mL in fresh media. Each of the compounds prepared in the preceding Examples were dissolved in 99.5% (cell culture grade) dimethylsulfoxide (DMSO) to prepare a stock solution of 10 mM concentration, which were serially diluted in the culture medium to final concentrations of 1000, 200, 40, 8, 1.6 and 0.32 nM for HER2 A775insYVMA Ba/F3 cells and concentrations of 10000, 5000, 1000, 200, 40 and 8 nM for wild-type EGFR overexpressing Ba/F3 cells.

To each aliquot of cells in the 96-well plate, a 50 µL test solution comprising one of the compounds was added to result in each well a 150 µL volume of cells with a compound in a final concentration ranging from 10 µM to 0.32 nM. The Ba/F3 cells treated with the test solution were incubated for 72 hours at 37°C and 5% CO₂ level and underwent a 30-minute acclimation by placing the 96-well plate carrying the cells in ambient temperature. Thereafter, to each well was added 50 µL CellTiter-Glo^{®} Luminescent Cell Assay Reagent (CTG, Promega, USA). After allowing 10 minutes to stabilize the signal, luminescence intensity was measured using a microplate reader. The half-maximal growth inhibition concentration (GI₅₀) was calculated from the luminescence readout of the test compounds with the difference between the initial and final cell densities for the well untreated with a compound set at 100% growth. Nonlinear regression analysis based on log[inhibitor] vs. normalized response mode of the Graph Prism software was used for calculating GI₅₀ for each compound, which are summarized in Table 6. The calculated GI₅₀ values are classified in Table 6 into four categories: A for GI₅₀ values less than 100 nM, B for GI₅₀ values more than 100 nM but less than 1,000 nM, C for GI₅₀ values more than 1,000 nM but less than 5,000 nM, and D for GI₅₀ values of more than 5,000 nM.

**[Table 6]**

| Ex. No. | Ba/F3 HER2^{YVMA} GI₅₀ class | Ba/F3 EGFR^{WT} GI₅₀ class | Ex. No. | Ba/F3 HER2^{YVMA} Gl₅₀ class | Ba/F3 EGFR^{WT} GI₅₀ class |
|---|---|---|---|---|---|
| **1** | A | B | **47** | A | C |
| **2** | A | B | **48** | B | D |
| **3** | A | B | **49** | A | D |
| **4** | C | C | **50** | A | C |
| **5** | A | D | **51** | A | C |
| **6** | A | D | **52** | A | C |
| **7** | B | D | **53** | A | C |
| **8** | B | C | **54** | A | D |
| **9** | B | D | **55** | A | D |
| **10** | A | C | **56** | A | B |
| **11** | B | C | **57** | A | B |
| **12** | C | C | **58** | B | C |
| **13** | B | C | **59** | A | C |
| **14** | C | C | **60** | A | D |
| **15** | A | C | **61** | A | C |
| **16** | B | D | **62** | B | D |
| **17** | A | D | **63** | B | C |
| **18** | B | D | **64** | B | D |
| **19** | B | D | **65** | A | B |
| **20** | B | D | **66** | C | C |
| **21** | B | C | **67** | B | B |
| **22** | A | C | **68** | A | B |
| **23** | A | D | **69** | B | B |
| **24** | A | C | **70** | B | C |
| **25** | A | C | **71** | B | C |
| **26** | A | C | **72** | B | A |
| **27** | B | C | **73** | C | C |
| **28** | A | C | **74** | A | A |
| **29** | A | C | **75** | A | B |
| **30** | B | B | **76** | A | B |
| **31** | B | C | **77** | A | A |
| **32** | B | D | **78** | A | B |
| 33 | A | C | **79** | C | C |
| 34 | A | D | **80** | A | B |
| 35 | A | D | **81** | A | B |
| 36 | A | D | **82** | A | B |
| 37 | B | A | **83** | A | A |
| 38 | A | B | **84** | A | B |
| 39 | A | B | **85** | A | B |
| 40 | A | D | **86** | A | C |
| 41 | A | C | **87** | A | C |
| 42 | A | C | **88** | A | C |
| 43 | A | C | **89** | A | C |
| 44 | A | D | **90** | A | A |
| 45 | A | D | **91** | B | B |
| 46 | A | C | **92** | B | B |

While the foregoing disclosure has described the present invention with reference to a limited number of working examples, it is to be understood that the technical idea and scope of the present invention is not limited to these examples disclosed herein. Embodiments as defined in the appended claims as well as various modifications, changes, and variations to these embodiments that are obvious to one skilled in the art to which the invention pertains are encompassed within the scope of this invention.

## Claims

1. A compound of Chemical Formula 1 or a tautomer, a stereoisomer or a pharmaceutically acceptable salt thereof: 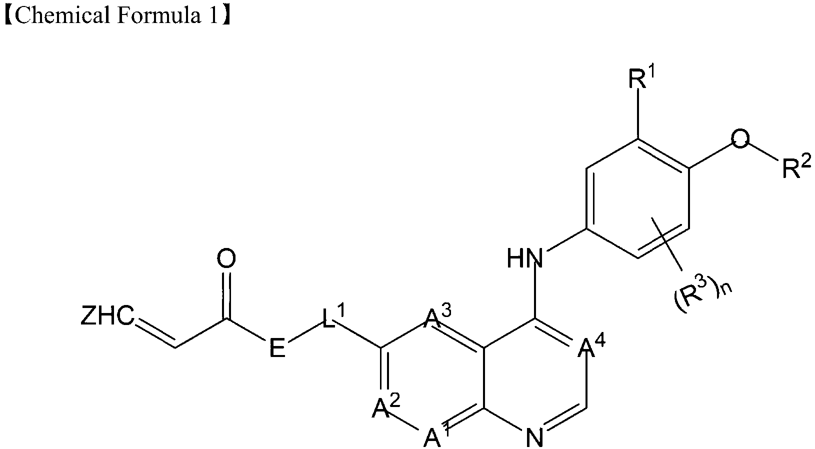
wherein A¹ and A³ are, independently of each other, N or CH, and A² and A⁴ are, independently of each other, N or CR*^{a}*, said R*^{a}* being H, CN, C₁₋₄alkyl or C₁₋₄alkoxy;
R¹ is C₁₋₄alkyl, C₁₋₄alkoxy, CF₃ or halogen;
R² is (5+6)-membered fused bicyclic heteroaryl or (6+6)-membered fused bicyclic heteroaryl, said (5+6)-membered fused bicyclic heteroaryl or (6+6)-membered fused bicyclic heteroaryl being optionally substituted at one or more hydrogen atoms with a functional group independently selected from the group consisting of halogen, C₁₋₄alkyl and C₁₋₄alkoxy;
each R³ is independently halogen or C₁₋₄alkoxy, and n is an integer from 0 to 3;
L¹ is -CH=CH-M, -CH=CHQ¹M, -NR^{b}Q¹M, -OQ¹M, -S(=O)₂Q¹M, 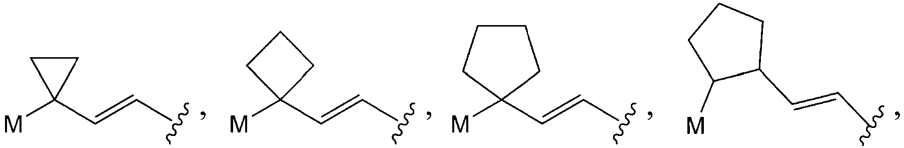 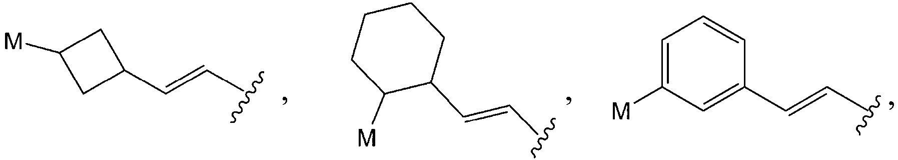
-N(Q²)C(=O)Q¹M, -C(=O)N(Q²)Q¹M, -C≡C-M or -C≡C-Q¹M, said Q¹ being C₁₋₃alkylene, Q² being H or C₁₋₃alkyl, R^{b} being H or C₁₋₃alkyl, and M being a single bond connecting to the functional group E; 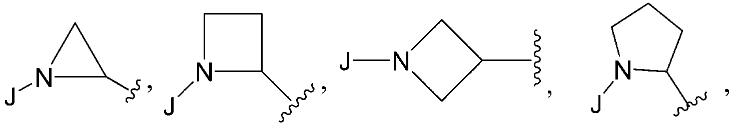
E is 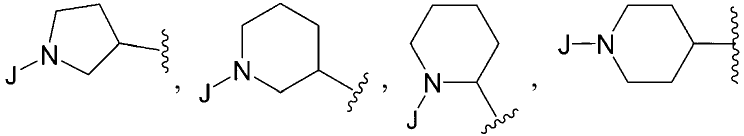 or -NR^{c}-, said R^{c} being H, C₁₋₄alkyl or C₃₋₆cycloalkyl and J being a single bond connecting to the carbonyl group of -C(=O)CH=CHZ in Chemical Formula 1; and
Z is H, C₁₋₄alkyl, X, -C₁₋₃alkylene-X or -C₁₋₃alkylene-NR^{d}NR^{e}, X is substituted or unsubstituted 5-6-membered heterocycloalkyl, and R^{d} and R^{e} are, independently of each other, H or C₁₋₃alkyl, said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

2. The compound according to claim 1, wherein at least one of A¹ to A⁴ is N.

3. The compound according to claim 2, wherein L¹ is -CH=CH-Q¹M, Q¹ is CH₂ or - CH(CH₃)-, E is -NR^{c}- and Z is H.

4. The compound according to claim 2, wherein L¹ is -CH≡CH-Q¹M, Q¹ is CH₂ and E is -NR^{c}-.

5. The compound according to claim 1, wherein R² is 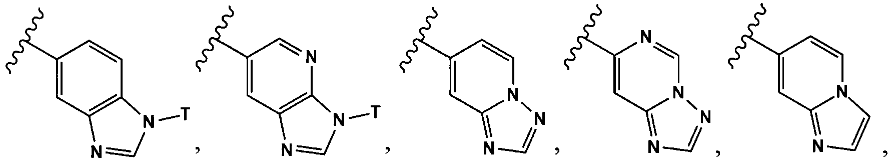 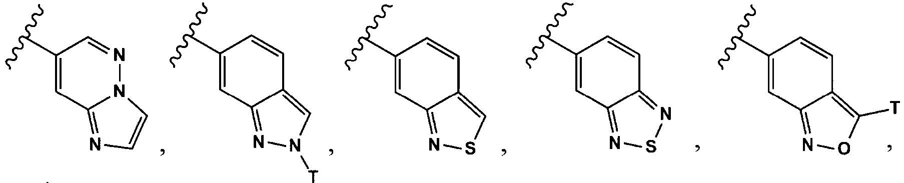 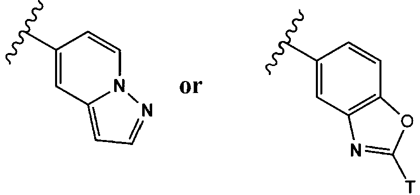 , said T being H or C₁₋₄alkyl.

6. The compound according to claim 1, wherein the compound has a structure as defined in Chemical Formula 2: 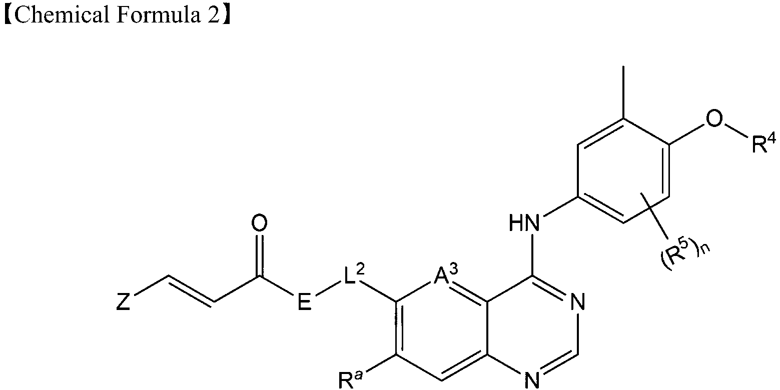
wherein R⁴ is 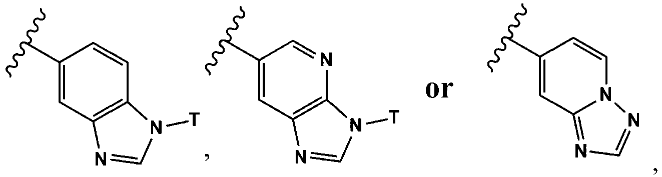 said T being H or C₁₋₄alkyl;
each R⁵ is independently halogen or C₁₋₄alkoxy and n is an integer from 0 to 3;
A³ is N or CH and R*^{a}* is H, C₁₋₄alkyl or C₁₋₄alkoxy;
L² is 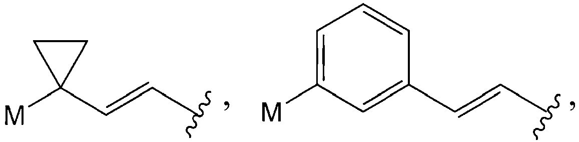 -CH=CH-M, - CH=CHQ¹M, -NR^{b}Q¹M or -OQ¹M, said Q¹ being C₁₋₃alkylene, R^{b} being H or C₁₋₃alkyl, and M being a single bond connecting to the functional group E; 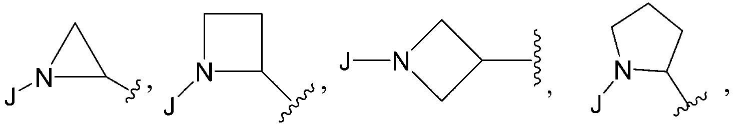
E is 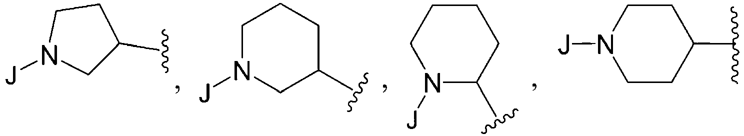 or -NR^{c}-, said R^{c} being H, C₁₋₄alkyl or C₃₋₆cycloalkyl and J being a single bond connecting to the carbonyl group of -C(=O)CH=CHZ; and
Z is H, C₁₋₄alkyl, X, -C₁₋₃alkylene-X or -C₁₋₃alkylene-NR^{d}NR^{e}, X is substituted or unsubstituted 5-6-membered heterocycloalkyl, and R^{d} and R^{e} are, independently of each other, H or C₁₋₃alkyl, said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

7. The compound according to claim 6, wherein A³ is N.

8. The compound according to claim 6, wherein R⁵ is F.

9. The compound according to claim 6, wherein Z is H, CH₃, 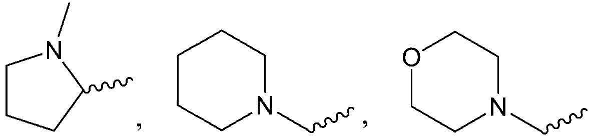 or -CH₂N(CH₃)₂.

10. The compound according to claim 6, wherein the compound has a structure as defined in Chemical Formula 3: 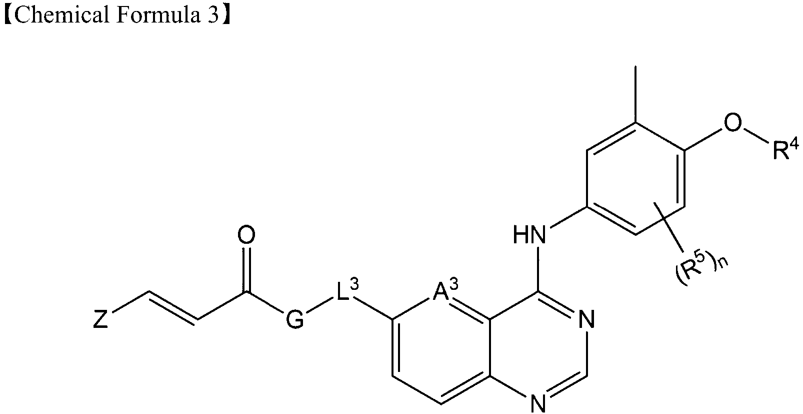
wherein R⁴ is 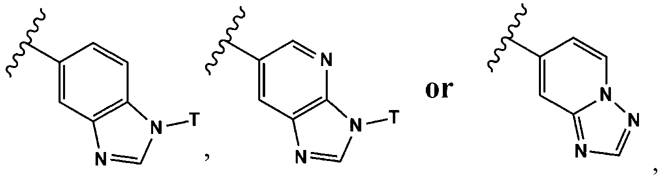 said T being H or C₁₋₄alkyl;
each R⁵ is independently halogen and n is an integer from 0 to 3;
A³ is N or CH;
L³ is -CH=CH-M, -CH=CHQ¹M, -NR^{b}Q¹M or -OQ¹M, said Q¹ being C₁₋₃alkylene, R^{b} being H or C₁₋₃alkyl, and M being a single bond connecting to the functional group G;
G is -NR^{c}-, 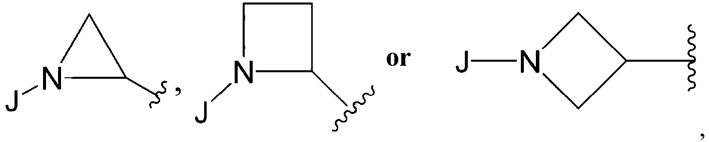 said R^{c} being H, C₁₋₄alkyl or C₃₋₆cycloalkyl and J being a single bond connecting to the carbonyl group of -C(=O)CH=CHZ; and
Z is H, C₁₋₄alkyl, X, -C₁₋₃alkylene-X or -C₁₋₃alkylene-NR^{d}NR^{e}, X is substituted or unsubstituted 5-6-membered heterocycloalkyl, and R^{d} and R^{e} are, independently of each other, H or C₁₋₃alkyl, said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

11. The compound according to claim 10, wherein the compound has a structure as defined in Chemical Formula 4: 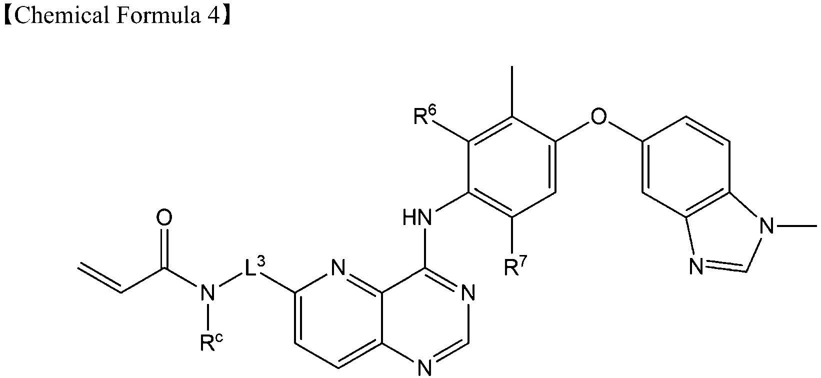
wherein L³ is -CH=CH-M, -CH=CHQ¹M, -NR^{b}Q¹M or -OQ¹M, said Q¹ being C₁₋₃alkylene, R^{b} being H or C₁₋₃alkyl, and M being a single bond connecting to the functional group CH₂=CHC(=O)NR^{c};
R^{c} is H, C₁₋₄alkyl or C₃₋₆cycloalkyl; and
R⁶ and R⁷ are, independently of each other, H or F.

12. The compound according to claim 1, wherein the compound has a structure as defined in Chemical Formula 5: 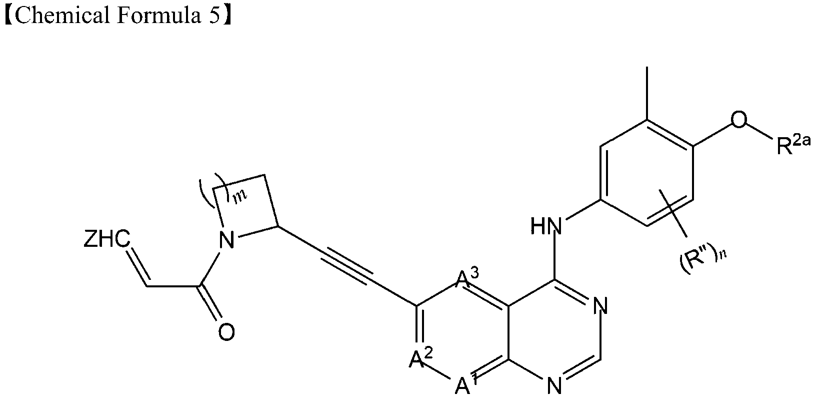
wherein A¹ and A³ are, independently of each other, N or CH, and A² is N or CR*^{a}*, said R*^{a}* being H, C₁₋₄alkyl or C₁₋₄alkoxy;
R^{2a} is 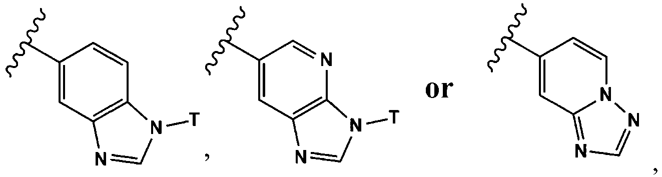 said T being H or C₁₋₄alkyl;
each R" is independently halogen and n is an integer from 0 to 3;
m is an integer from 1 to 3; and
Z is H, C₁₋₄alkyl, X, -C₁₋₃alkylene-X or -C₁₋₃alkylene-NR^{d}NR^{e}, X is substituted or unsubstituted 5-6-membered heterocycloalkyl, and R^{d} and R^{e} are, independently of each other, H or C₁₋₃alkyl, said substituted 5-6-membered heterocycloalkyl being a ring in which one or more hydrogen atoms in the corresponding unsubstituted 5-6-membered heterocycloalkyl are substituted with a functional group independently selected from the group consisting of C₁₋₄alkyl, halogen, CN, NO₂ or CF₃.

13. The compound according to claim 12, wherein R^{2a} is

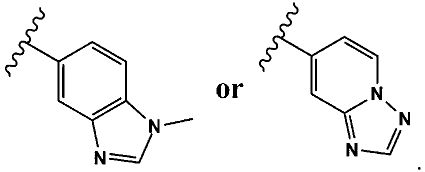

14. The compound according to claim 13, wherein A¹ is CH, and A³ is N.

15. A compound or a tautomer, a stereoisomer or a pharmaceutically acceptable salt thereof selected from the group consisting of:
*N*-(2-((4-((4-([1,2,4]triazolo[1,5-*a*]pyridin-7-yloxy)-3-methylphenyl)amino)quinazolin-6-yl)oxy)ethyl)-*N*-methylacrylamide,
*N*-(2-((4-((4-([1,2,4]triazolo[1,5-*a*]pyridin-7-yloxy)-3-methylphenyl)amino)-7-methoxyquinazolin-6-yl)oxy)ethyl)-*N*-methylacrylamide,
*N*-(2-((4-((4-([1,2,4]triazolo[1,5-*a*]pyridin-7-yloxy)-3-methylphenyl)amino)pyrido[3,2-*d*]pyrimidin-6-yl)oxy)ethyl)-*N*-methylacrylamide,
*N*-(2-((8-((4-([1,2,4]triazolo[1,5-*a*]pyridin-7-yloxy)-3-methylphenyl)amino)pyrimido[5,4-*d*]pyrimidin-2-yl)oxy)ethyl)-*N*-methylacrylamide,,
*N*-methyl-*N*-(2-((4-((3-methyl-4-((1-methyl-1*H*-benzo[*d*]imidazol-5-yl)oxy)phenyl)amino)quinazolin-6-yl)oxy)ethyl)acrylamide,
*N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)oxy)ethyl)acrylamide,
*N*-(2-((4-((3-methyl-4-((1-methyl-1*H*-benzo[*d*]imidazol-5-yl)oxy)phenyl)amino)pyrido[3,2-*d*]pyrimidin-6-yl)oxy)ethyl)acrylamide,
*N*-methyl-*N*-(2-((8-((3-methyl-4-((1-methyl-1*H*-benzo[*d*]imidazol-5-yl)oxy)phenyl)amino)pyrimido[5,4-*d*]pyrimidin-2-yl)oxy)ethyl)acrylamide,
*N*-methyl-*N*-(3-((4-((3-methyl-4-((1-methyl-1*H*-benzo[*d*]imidazol-5-yl)oxy)phenyl)amino)pyrido[3,2-*d*]pyrimidin-6-yl)oxy)propyl)acrylamide,
*N*-(2-((4-((3-methyl-4-((1-methyl-1*H*-benzo[*d*]imidazol-5-yl)oxy)phenyl)amino)quinazolin-6-yl)oxy)ethyl)acrylamide,
*N*-(2-((4-((4-([1,2,4]triazolo[1,5-*a*]pyridin-7-yloxy)-3-methylphenyl)amino)pyrido[3,2-*d*]pyrimidin-6-yl)amino)ethyl)-*N*-methylacrylamide,
*N*-(2-((8-((4-([1,2,4]triazolo[1,5-*a*]pyridin-7-yloxy)-3-methylphenyl)amino)pyrimido[5,4-*d*]pyrimidin-2-yl)amino)ethyl)-*N*-methylacrylamide,
*N*-(2-((4-((4-([1,2,4]triazolo[1,5-*a*]pyridin-7-yloxy)-3-methylphenyl)amino)pyrido[3,2-*d*]pyrimidin-6-yl)(methyl)amino)ethyl)-*N-*methylacrylamide,
*N*-(2-((8-((4-([1,2,4]triazolo[1,5-*a*]pyridin-7-yloxy)-3-methylphenyl)amino)pyrimido[5,4-*d*]pyrimidin-2-yl)(methyl)amino)ethyl)-*N-*methylacrylamide,
*N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)amino)ethyl)acrylamide,
*N*-methyl-*N*-(2-((8-((3-methyl-4-((1-methyl-1*H*-benzo[*d*]imidazol-5-yl)oxy)phenyl)amino)pyrimido[5,4-*d*]pyrimidin-2-yl)amino)ethyl)acrylamide,
*N*-methyl-*N*-(3-((4-((3-methyl-4-((1-methyl-1*H*-benzo[*d*]imidazol-5-yl)oxy)phenyl)amino)pyrido[3,2-*d*]pyrimidin-6-yl)amino)propyl)acrylamide,
*N*-methyl-*N*-(2-(methyl(4-((3-methyl-4-((1-methyl-1*H*-benzo[*d*]imidazol-5-yl)oxy)phenyl)amino)pyrido[3,2-*d*]pyrimidin-6-yl)amino)ethyl)acrylamide,
*N*-methyl-*N*-(2-(methyl(8-((3-methyl-4-((1-methyl-1*H*-benzo[*d*]imidazol-5-yl)oxy)phenyl)amino)pyrimido[5,4-*d*]pyrimidin-2-yl)amino)ethyl)acrylamide,
*N*-methyl-*N*-(3-(methyl(4-((3-methyl-4-((1-methyl-1*H*-benzo[*d*]imidazol-5-yl)oxy)phenyl)amino)pyrido[3,2-*d*]pyrimidin-6-yl)amino)propyl)acrylamide,
*N*-methyl-*N*-(2-((4-((3-methyl-4-((1-methyl-1*H*-benzo[*d*]imidazol-5-yl)oxy)phenyl)amino)pyrido[3,2-*d*]pyrimidin-6-yl)amino)-2-oxoethyl)acrylamide,
(*E*)-*N*-methyl-*N*-(3-((4-((3-methyl-4-((1-methyl-1*H*-benzo[*d*]imidazol-5-yl)oxy)phenyl)amino)quinazolin-6-yl)aryl)acrylamide,
(*E*)-*N*-methyl-*N*-(3-(4-((3-methyl-4-((1-methyl-1*H*-benzo[*d*]imidazol-5-yl)oxy)phenyl)amino)pyrido[3,2-*d*]pyrimidin-6-yl)aryl)acrylamide,
(*E*)-*N*-methyl-*N*-(3-(8-((3-methyl-4-((1-methyl-1*H*-benzo[*d*]imidazol-5-yl)oxy)phenyl)amino)pyrimido[5,4-*d*]pyrimidin-2-yl)allyl)acrylamide,
(*E*)-*N*-(3-(4-((3-chloro-4-((1-methyl-1*H*-benzo[*d*]imidazol-5-yl)oxy)phenyl)amino)pyrido[3,2-*d*]pyrimidin-6-yl)allyl)-*N*-methylacrylamide,
(*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-4-en-1-yl)acrylamide,
(*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)piperidin-1-yl)prop-2-en-1-one,
(*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)azetidin-1-yl)prop-2-en-1-one,
(*E*)-1-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)pyrrolidin-1-yl)prop-2-en-1-one,
(*E*)-*N*-methyl-*N*-(1-(2-(4-((3-methyl-4-((1-methyl-1*H*-benzo[*d*]imidazol-5-yl)oxy)phenyl)amino)pyrido[3,2-*d*]pyrimidin-6-yl)vinyl)cyclopropyl)acrylamide,
(*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)but-2-enamide,
(*E*)-4-(dimethylamino)-*N*-methyl-*N*-((*E*)-3-(4-((3-methyl-4-((1-methyl-1*H-*benzo[*d*]imidazol-5-yl)oxy)phenyl)amino)pyrido[3,2-*d*]pyrimidin-6-yl)allyl)but-2-enamide,
(*E*)-*N*-(3-(4-((3-methyl-4-((1-methyl-1*H*-benzo[*d*]imidazol-5-yl)oxy)phenyl)amino)pyrido[3,2-*d*]pyridin-6-yl)allyl)acrylamide,
(*E*)-*N*-(3-(4-((3-methyl-4-((3-methyl-3*H*-imidazo[4,5-*b*]pyridin-6-yl)oxy)phenyl)amino)pyrido[3,2-*d*]pyrimidin-6-yl)allyl)acrylamide,
(*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)acrylamide,
(*E*)-*N*-(3-(4-((2-chloro-3-methyl-4-((1-methyl-1*H*-benzo[*d*]imidazol-5-yl)oxy)phenyl)amino)pyrido[3,2-*d*]pyrimidin-6-yl)allyl)acrylamide,
(*E*)-*N*-(3-(4-((3-methyl-4-(pyrazolo[1,5-*a*]pyridin-5-yloxy)phenyl)amino)pyrido[3,2-*d*]pyrimidin-6-yl)allyl)acrylamide,
*E*)-*N*-(3-(4-((4-([1,2,4]triazolo[1,5-*a*]pyridin-7-yloxy)-3-methylphenyl)amino)pyrido[3,2-*d*]pyrimidin-6-yl)allyl)acrylamide,
(*E*)-*N*-(3-(4-((4-(imidazo[1,2-*a*]pyridin-7-yloxy)-3-methylphenyl)amino)pyrido[3,2-*d*]pyrimidin-6-yl)allyl)acrylamide,
(*E*)-4-(dimethylamino)-*N*-((*E*)-3-(4-((3-methyl-4-((1-methyl-1*H-*benzo[*d*]imidazol-5-yl)oxy)phenyl)amino)pyrido[3,2-*d*]pyrimidin-6-yl)allyl)but-2-enamide,
(*E*)-4-(dimethylamino)-*N*-((*E*)-3-(4-((2-fluoro-3-methyl-4-((1-methyl-1*H-*benzo[*d*]imidazol-5-yl)oxy)phenyl)amino)pyrido[3,2-*d*]pyrimidin-6-yl)allyl)but-2-enamide,
(*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(*E*)-*N*-methyl-*N*-(3-(4-((3-methyl-4-((3-methyl-3*H*-imidazo[4,5-*b*]pyridin-6-yl)oxy)phenyl)amino)pyrido[3,2-*d*]pyrimidin-6-yl)allyl)acrylamide,
(*E*)-*N*-methyl-N-(3-(4-((3-methyl-4-((3-methyl-3*H*-imidazo[4,5-*b*]pyridin-6-yl)oxy)phenyl)amino)pyrido[3,2-*d*]pyrimidin-6-yl)allyl)acrylamide,
(*E*)-*N*-(3-(4-((2-fluoro-5-methyl-4-((1-methyl-1*H*-benzo[*d*]imidazol-5-yl)oxy)phenyl)amino)pyrido[3,2-*d*]pyrimidine -6-yl)allyl)-*N*-methylacrylamide,
(*E*)-*N*-(3-(4-((2-fluoro-3-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(*E*)-*N*-(3-(4-((2-fluoro-3-methyl-4-((3-methyl-3*H*-imidazolo[4,5-*b*]pyridin-6-yl)oxy)phenyl)amino)pyrido[3,2-*d*]pyrimidin-6-yl)allyl)-*N*-methylacrylamide,
(*E*)-1-(3-(2-(4-((3-methyl-4-((2-methyl-2*H*-indazol-6-yl)oxy)phenyl)amino)pyrido[3,2-*d*]pyrimidin-6-yl)vinyl)azetidin-1-yl)prop-2-en-1-one,
(*E*)-*N*-ethyl-*N*-(3-(4-((3-methyl-4-((1-methyl-1*H*-benzo[*d*]imidazol-5-yl)oxy)phenyl)amino)pyrido[3,2-*d*]pyrimidin-6-yl)allyl)acrylamide,
(*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(*S*,*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(*R*,*E*)-*N*-methyl-*N*-(4-(4-((3-methyl-4-((1-methyl-1*H*-benzo[*d*]imidazol-5-yl)oxy)phenyl)amino)pyrido[3,2-*d*]pyrimidin-6-yl)but-3-en-2-yl)acrylamide,
(*E*)-*N*-(*tert*-butyl)-*N*-(3-(4-((3-methyl-4-((1-methyl-1*H*-benzo[*d*]imidazol-5-yl)oxy)phenyl)amino)pyrido[3,2-*d*]pyrimidin-6-yl)allyl)acrylamide,
(*E*)-*N*-(3-(4-((2-methoxy-5-methyl-4-((1-methyl-1*H*-benzo[*d*]imidazol-5-yl)oxy)phenyl)amino)pyrido[3,2-*d*]pyrimidin-6-yl)allyl)-*N*-methylacrylamide,
(*E*)-*N*-(3-(4-((3-fluoro-4-((1-methyl-1*H*-benzo[*d*]imidazol-5-yl)oxy)phenyl)amino)pyrido[3,2-*d*]pyrimidin-6-yl)allyl)-*N*-methylacrylamide,
(*E*)-*N*-(3-(4-((4-([1,2,4]triazolo[1,5-*a*]pyridin-7-ylmethyl)-3-methylphenyl)amino)pyrido[3,2-*d*]pyrimidin-6-yl)allyl)-*N*-methylacrylamide,
(*E*)-*N*-(3-(4-((4-(benzo[*d*]oxazol-5-yloxy)-3-methylphenyl)amino)pyrido[3,2-*d*]pyrimidin-6-yl)allyl)-*N*-methylacrylamide,
(*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-3-((*R*)-1-methylpyrrolidin-2-yl)acrylamide,
(*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-(piperidin-1-yl)but-2-enamide,
(*E*)-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-morpholinobut-2-enamide,
(*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-(piperidin-1-yl)but-2-enamide,
(*E*)-*N*-methyl-*N*-((*E*)-3-(4-((3-methyl-4-((1-methyl-1*H*-benzo[*d*]imidazol-5-yl)oxy)phenyl)amino)pyrido[3,2-*d*]pyrimidin-6-yl)allyl)-4-morpholinobut-2-enamide,
(*E*)-*N*-methyl-*N*-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)phenyl)acrylamide,
(*E*)-*N*-(3-(2-(4-((3-methyl-4-((1-methyl-1*H*-benzo[*d*]imidazol-5-yl)oxy)phenyl)amino)pyrido[3,2-*d*]pyrimidin-6-yl)vinyl)phenyl)acrylamide,
*N*-(3-(4-((4-([1,2,4]triazolo[1,5-*a*]pyridin-7-yloxy)-3-methylphenyl)amino)quinazolin-6-yl)prop-2 -yn-1-yl)-*N*-methylacrylamide,
*N*-(3-(4-((4-([1,2,4]triazolo[1,5-*a*]pyridin-7-yloxy)-3-methylphenyl)amino)pyrido[2,3-*d*]pyrimidin-6-yl)prop-2-yn-1-yl)-*N*-methylacrylamide,
*N*-(3-(4-((4-([1,2,4]triazolo[1,5-*a*]pyridin-7-yloxy)-3-methylphenyl)amino)pyrido[3,4-*d*]pyrimidin-6-yl)prop-2-yn-1-yl)-*N*-methylacrylamide,
*N*-(3-(4-((4-([1,2,4]triazolo[1,5-*a*]pyridin-7-yloxy)-3-methylphenyl)amino)pyrido[3,2-*d*]pyrimidin-6-yl)prop-2-yn-1-yl)-*N*-methylacrylamide,
*N*-(3-(8-((4-([1,2,4]triazolo[1,5-*a*]pyridin-7-yloxy)-3-methylphenyl)amino)pyrimido[5,4-*d*]pyrimidin-2-yl)prop-2-yn-1-yl)-*N*-methylacrylamide,
*N*-(3-(4-((4-([1,2,4]triazolo[1,5-*a*]pyridin-7-yloxy)-3-methylphenyl)amino)-3-cyanoquinolin-6-yl)prop-2-yn-1-yl)-*N*-methylacrylamide,
*N*-methyl-*N*-(3-(4-((3-methyl-4-((1-methyl-1*H*-benzo[*d*]imidazol-5-yl)oxy)phenyl)amino)pyrido[3,2-*d*]pyrimidin-6-yl)prop-2-yn-1-yl)acrylamide,
*N*-methyl-*N*-(3-(8-((3-methyl-4-((1-methyl-1*H*-benzo[*d*]imidazol-5-yl)oxy)phenyl)amino)pyrido[5,4-*d*]pyrimidin-2-yl)prop-2-yn-1-yl)acrylamide,
*N*-(3-(4-((4-([1,2,4]triazolo[1,5-*a*]pyridin-7-yloxy)-3-methylphenyl)amino)-7-methoxyquinazolin-6-yl) prop-2-yn-1-yl)-*N*-methylacrylamide,
(*R*)-1-(2-((4-((4-([1,2,4]triazolo[1,5-*a*]pyridin-7-yloxy)-3-methylphenyl)amino)quinazolin-6-yl)ethynyl)pyrrolidin-1-yl)prop-2-en-1-one,
(*S*)-1-(2-((4-((4-([1,2,4]triazolo[1,5-*a*]pyridin-7-yloxy)-3-methylphenyl)amino)quinazolin-6-yl)ethynyl)pyrrolidin-1-yl)prop-2-en-1-one,
(*R*)-1-(2-((4-((4-([1,2,4]triazolo[1,5-*a*]pyridin-7-yloxy)-3-methylphenyl)amino)quinazolin-6-yl)ethynyl)piperidin-1-yl)prop-2-en-1-one,
(*S*)-1-(2-((4-((4-([1,2,4]triazolo[1,5-*a*]pyridin-7-yloxy)-3-methylphenyl)amino)quinazolin-6-yl)ethynyl)piperidin-1-yl)prop-2-en-1-one,
(*R*)-1-(2-((4-((4-([1,2,4]triazolo[1,5-*a*]pyridin-7-yloxy)-3-methylphenyl)amino)quinazolin-6-yl)ethynyl)azetidin-1-yl)prop-2-en-1-one,
(*S*)-1-(2-((4-((4-([1,2,4]triazolo[1,5-*a*]pyridin-7-yloxy)-3-methylphenyl)amino)quinazolin-6-yl)ethynyl)azetidin-1-yl)prop-2-en-1-one,
(*R*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)pyrrolidin-1-yl)prop-2-en-1-one,
(*S*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)pyrrolidin-1-yl)prop-2-en-1-one,
(*R*)-1-(2-((4-((4-([1,2,4]triazolo[1,5-*a*]pyridin-7-yloxy)-3-methylphenyl)amino)pyrido[3,2-*d*]pyrimidin-6-yl)ethynyl)piperidin-1-yl)prop-2-en-1-one,
(*R*)-1-(2-((8-((4-([1,2,4]triazolo[1,5-*a*]pyridin-7-yloxy)-3-methylphenyl)amino)pyrimido[5,4-*d*]pyrimidin-2-yl)ethynyl)pyrrolidin-1-yl)prop-2-en-1-one,
(*S*)-1-(2-((8-((4-([1,2,4]triazolo[1,5-*a*]pyridin-7-yloxy)-3-methylphenyl)amino)pyrimido[5,4-*d*]pyrimidin-2-yl)ethynyl)pyrrolidin-1-yl)prop-2-en-1-one,
(*R*)-1-(2-((4-((3-methyl-4-((1-methyl-1*H*-benzo[*d*]imidazol-5-yl)oxy)phenyl)amino)quinazolin-6-yl)ethynyl)pyrrolidin-1-yl)prop-2-en-1-one,
1-((2*R*)-2-((4-((3-methyl-4-((1-methyl-3*a*,7*a*-dihydro-1*H*-benzo[*d*]imidazol-5-yl)oxy)phenyl)amino)pyrido[3,2-*d*]pyrimidin-6-yl)ethynyl)pyrrolidin-1-yl)prop-2-en-1-one,
(*S*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)pyrrolidin-1-yl)prop-2-en-1-one,
(*R*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)piperidin-1-yl)prop-2-en-1-one,
(*S*)-1-(2-((4-((3-methyl-4-((1-methyl-1*H*-benzo[*d*]imidazol-5-yl)oxy)phenyl)amino)pyrido[3,2-*d*]pyrimidin-6-yl)ethynyl)piperidin-1-yl)prop-2-en-1-one,
1-((2*R*)-2-((8-((3-methyl-4-((1-methyl-3*a*,7*a*-dihydro-1*H*-benzo[*d*]imidazol-5-yl)oxy)phenyl)amino)pyrimido[5,4-*d*]pyrimidin-2-yl)ethynyl)pyrrolidin-1-yl)prop-2-en-1-one,
(*R*)-1-(2-((8-((3-methyl-4-((1-methyl-1*H*-benzo[*d*]imidazol-5-yl)oxy)phenyl)amino)pyrimido[5,4-*d*]pyrimidin-2-yl)ethynyl)piperidin-1-yl)prop-2-en-1-one and
(*S*)-1-(2-((8-((3-methyl-4-((1-methyl-1*H*-benzo[*d*]imidazol-5-yl)oxy)phenyl)amino)pyrimido[5,4-*d*]pyrimidin-2-yl)ethynyl)piperidin-1-yl)prop-2-en-1-one.

16. A pharmaceutical composition for preventing or treating cancer, said composition comprising a therapeutically effective amount of a compound of claim 1 and a pharmaceutically acceptable excipient.
